(19) Europäisches Patentamt
European Patent Office
Office européen des brevets

(11) EP 4 537 812 A1

(12) **EUROPEAN PATENT APPLICATION**
published in accordance with Art. 153(4) EPC

(43) Date of publication:
**16.04.2025 Bulletin 2025/16**

(21) Application number: **23819727.1**

(22) Date of filing: **31.05.2023**

(51) International Patent Classification (IPC):
***A61K 8/891*** (2006.01) ***A61K 8/31*** (2006.01)
***A61K 8/41*** (2006.01) ***A61K 8/897*** (2006.01)
***A61K 8/898*** (2006.01) ***A61Q 5/00*** (2006.01)
***A61Q 5/06*** (2006.01) ***A61Q 5/12*** (2006.01)
***D06M 13/513*** (2006.01) ***D06M 15/21*** (2006.01)
***D06M 15/263*** (2006.01) ***D06M 15/643*** (2006.01)

(52) Cooperative Patent Classification (CPC):
**A61K 8/31; A61K 8/41; A61K 8/891; A61K 8/897; A61K 8/898; A61Q 5/00; A61Q 5/06; A61Q 5/12; D06M 13/513; D06M 15/21; D06M 15/263; D06M 15/643**

(86) International application number:
**PCT/JP2023/020256**

(87) International publication number:
**WO 2023/238747 (14.12.2023 Gazette 2023/50)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **10.06.2022 JP 2022094485**
**30.09.2022 JP 2022157591**

(71) Applicant: **Kao Corporation**
**Tokyo 103-8210 (JP)**

(72) Inventors:
- **HIRAGA Taito**
**Tokyo 131-8501 (JP)**
- **MAEKAWA Tomoka**
**Tokyo 131-8501 (JP)**
- **MARUYAMA Aguri**
**Tokyo 131-8501 (JP)**
- **FURUKAWA Junichi**
**Tokyo 131-8501 (JP)**

(74) Representative: **Hoffmann Eitle**
**Patent- und Rechtsanwälte PartmbB**
**Arabellastraße 30**
**81925 München (DE)**

# (54) HAIR TREATMENT AGENT, OR FIBER TREATMENT AGENT FOR HEADWEAR PRODUCTS

(57) Disclosed is a hair processing agent or a fiber processing agent for a headwear product, which contains (I) the following components (A) to (C) and has a content of water of 10% by mass or less, or (II) the following components (A), (B1), (C) and (D): (A) a film-forming polymer containing an M unit represented by $(R')_3SiO_{1/2}$ ($R^1$ represents a hydrocarbon group having 1 or more and 12 or less carbon atoms which may be substituted with fluorine or a hydroxy group, and a plurality of $R^1$'s may be the same or different from each other) and a Q unit represented by $SiO_{4/2}$; (B) a film-forming polymer other than the component (A); (B1) a film-forming polymer other than the component (A), excluding a cationic polymer containing a quaternary ammonium cation; (C) a high-molecular-weight organopolysiloxane having a degree of polymerization of 2,200 or more and 20,000 or less; (D) an organopolysiloxane other than the components (A), (B1), and (C), having a polyalkylene oxide moiety and a cationic group.

EP 4 537 812 A1

## Description

Field of the Invention

The present invention relates to a hair processing agent or a fiber processing agent for headwear products such as wigs and extensions.

Background of the Invention

In recent years, in the field of cosmetics, a technique for forming a hydrophobic film has been studied as a method for imparting various performances such as gloss and good touch feeling to keratin substances such as skin and hair.

As a hydrophobic film-forming polymer, a technique using a silicone-based film-forming polymer is known. For example, JP H09-501934 A (PTL 1) discloses a topical personal care composition for use in a hair styling composition, the topical personal care composition containing: a polysiloxane grafted pressure-sensitive adhesive polymer; a volatile water-insoluble solvent for the polysiloxane grafted polymer; a non-volatile drying aid for the polysiloxane grafted polymer, the non-volatile drying aid being selected from the group consisting of a silicone fluid having 1 to 100 siloxy units, a wax, a silane, a silicone resin, and a mixture thereof, the silicone fluid being soluble in the solvent at 45°C and being water-insoluble at 25°C; and a weight ratio of the polysiloxane grafted pressure-sensitive adhesive polymer to the drying aid being no greater than a predetermined value. It also discloses that the composition can reduce the drying time of the hair styling composition without any substantial loss of hair retention performance when completely dried.

In addition, since the performance imparted to the keratin substance by the cosmetic or the performance imparted to the fiber for a headwear product by the fiber processing agent is often lost by friction caused by daily activities or by one washing, a cosmetic or a fiber processing agent having excellent durability of the effect is required. Since the film formed by a silicone-based film-forming polymer is generally hard and brittle, studies have been made on improvements in the touch feeling and durability.

For example, JP 2018-2643 A and JP 2021-134166 A (PTLs 2 and 3) disclose that a cosmetic characterized by blending a silicone resin composed of constituent units ($R^1{}_3SiO_{1/2}$), ($R^2{}_2SiO_{2/2}$), ($R^3SiO_{3/2}$), and ($SiO_{4/2}$) (in which, $R^1$, $R^2$, and R3 are each independently a group selected from an alkyl group having 1 to 8 carbon atoms, an aryl group having 6 to 12 carbon atoms, and a fluorine-substituted alkyl group having 1 to 8 carbon atoms) and having a ratio of each constituent unit and a weight-average molecular weight within a predetermined range, does not become sticky when applied, spreads well, has an excellent use feeling, has good water resistance, and has good adhesion to the skin, and thus has excellent cosmetic durability and can prevent adhesion to clothes (secondary adhesion).

JP 2019-513712 A (PTL 4) discloses a cosmetic composition capable of forming a multilayer structure after application to a keratin substance, the cosmetic composition containing at least one silicone and/or hydrocarbon-containing film-forming agent having at least one glass transition temperature of less than 60°C, and one or more silicone compounds in an amount sufficient to realize a viscosity of about 1,000 cSt to 22,000,000 cSt in a predetermined weight ratio, and describes that the composition has improved cosmetic properties upon application, in particular, good durability, feel, shine, brightness, and/or mat feeling properties.

JP 2021-526180 A (PTL 5) discloses that a phenyl silicone resin represented by a predetermined composition formula and having a weight-average molecular weight and a refractive index of a coating film each in a predetermined range is soluble in an organic-based oily component such as a silicone oil or a hydrocarbon oil, and a uniform continuous coating film is obtained, and the coating film has no stickiness and brittleness and has good compatibility with an ultraviolet absorber. In addition, it is described that a cosmetic blending the phenyl silicone resin has good use feeling and can prevent adhesion to clothes (secondary adhesion).

Summary of the Invention

The present invention relates to the following.

[1] A hair processing agent or a fiber processing agent for a headwear product of the following (I) or (II):

(I) in which the hair processing agent or the fiber processing agent for a headwear product contains the following components (A) to (C):

(A) a film-forming polymer containing an M unit represented by $(R^1)_3SiO_{1/2}$ ($R^1$ represents a hydrocarbon group having 1 or more and 12 or less carbon atoms and optionally substituted with fluorine, or a hydroxy group, and a plurality of $R^1$'s may be the same or different from each other) and a Q unit represented by $SiO_{4/2}$;

(B) a film-forming polymer other than the component (A); and

(C) a high-molecular-weight organopolysiloxane having a degree of polymerization of 2,200 or more and 20,000 or less,

in which a content of water is 10% by mass or less;

(II) in which the hair processing agent or the fiber processing agent for a headwear product contains the following components (A), (B1), (C), and (D):

(A) a film-forming polymer containing an M unit represented by $(R^1)_3SiO_{1/2}$ ($R^1$ represents a hydrocarbon group having 1 or more and 12 or less carbon atoms and optionally substituted with fluorine, or a hydroxy group, and a plurality of $R^1$'s may be the same or different from each other) and a Q unit represented by $SiO_{4/2}$;

(B1) a film-forming polymer other than the component (A), excluding a cationic polymer containing a quaternary ammonium cation;

(C) a high-molecular-weight organopolysiloxane having a degree of polymerization of 2,200 or more and 20,000 or less; and

(D) an organopolysiloxane other than the components (A), (B 1), and (C), having a polyalkylene oxide moiety and a cationic group.

[2] A method for treating hair or a fiber for a headwear product, including a step of applying the hair processing agent or the fiber processing agent for a headwear product according to [1] to hair or a fiber for a headwear product and then drying the hair or the fiber for a headwear product.

Detailed Description of the Invention

It is desired that a hair processing agent or a fiber processing agent for a headwear product such as a wig can impart a good touch feeling not only to hair or fibers for a headwear product in a dry state but also to hair or fibers for a headwear product in a wet state.

Further, as described in PTL 1, among hair processing agents and fiber processing agents for headwear products, in styling agents, it is desired to improve the shape retention performance of hair or fibers for headwear products. Specific examples of the shape retention performance of the hair or the fiber for a headwear product include the ability to easily create a voluminous style during styling (ability to impart a feeling of volume), the ability to form a clear shape during styling with heating using a hair iron or the like (heat setting property), and the ability to hardly lose effects such as the ability to impart a feeling of volume and the heat setting property even if hair washing is performed after applying a hair processing agent or a fiber processing agent for a headwear product. However, PTLs 1 to 5 do not disclose a hair processing agent or a fiber processing agent for a headwear product that can exhibit all of these effects.

Furthermore, in the heat setting of hair or a product for headwear fibers, good slipperiness when a hair iron is used (slipperiness of the hair iron) is also important.

A first object of the present invention is to provide a hair processing agent or a fiber processing agent for a headwear product that can impart a good touch feeling to hair or a fiber for a headwear product in either a dry state or a wet state, exhibits excellent property of imparting a feeling of volume and heat setting property during styling, and can suppress a decrease in effects such as the property of imparting a feeling of volume and the heat setting property even after hair washing.

A second object of the present invention is to provide a hair processing agent or a fiber processing agent for a headwear product that can impart a good touch feeling to hair or a fiber for a headwear product in either a dry state or a wet state, exhibits excellent heat setting property and slipperiness of a hair iron during styling, and can suppress a decrease in the heat setting property even after hair washing.

The present inventors have found that a hair processing agent or a fiber processing agent for a headwear product, which contains a silicone-based film-forming polymer having a specific constituent unit and another film-forming polymer, and a high molecular weight organopolysiloxane having a degree of polymerization in a specific range, and in which the content of water is a predetermined amount or less, can solve the first problem, and thus completed the first invention.

The present inventors have found that a hair processing agent or a fiber processing agent for a headwear product, which contains a silicone-based film-forming polymer having a specific constituent unit and another film-forming polymer, a high molecular weight organopolysiloxane having a degree of polymerization in a specific range, and an organopolysiloxane having a polyalkylene oxide moiety and a cationic group can solve the second problem, and thus completed the second invention.

According to the hair processing agent or the fiber processing agent for a headwear product of the first invention, it

is possible to impart a good touch feeling to hair or a fiber for a headwear product in either a dry state or a wet state, exhibit excellent property of imparting a feeling of volume and heat setting property during styling, and suppress a decrease in effects such as the property of imparting a feeling of volume and the heat setting property even after hair washing.

According to the hair processing agent or the fiber processing agent for a headwear product of the second invention, it is possible to impart a good touch feeling to hair or a fiber for a headwear product in either a dry state or a wet state, exhibit excellent heat setting property and slipperiness of a hair iron during styling, and suppress a decrease in the heat setting property even after hair washing.

[Definitions]

"Polymer" as used herein means a compound corresponding to a repetition of one or plural units (these units are derived from a compound known as a monomer). This or these units are repeated at least two times preferably at least three times.

"Hair" as used herein means primarily head hair, and also includes hair used in headwear products.

"Headwear product" as used herein means, for example, a hair wig, a wig, a weaving, a hair extension, a blade hair, a hair accessory, and a doll hair.

"Fiber for a headwear product" as used herein means a fiber used in the headwear product.

"Hydrophobic" as used herein means that a solubility in water of a substance is less than 1% by mass at 25°C.

"Film formation" as used herein means that, when applied to a substrate, preferably a solid film is left thereon.

"Durability" as used herein mainly means that the effect imparted by a hair processing agent or a fiber processing agent for a headwear product persists even if hair washing is performed after the application of the hair processing agent or the fiber processing agent for a headwear product (resistance to hair washing). In addition, "hair washing" in the present description also includes washing the fibers for headwear products. In the following description, the hair processing agent and the fiber processing agent for a headwear product may be collectively referred to as a "processing agent".

"Good touch feeling to hair or the fibers for headwear products in a wet state" means that the touch feeling of hair or the fibers for headwear products is good when the hair or the fibers for headwear products are washed and rinsed after a processing agent is applied to the hair or the fibers for headwear products and dried.

"Volatile" as used herein means a substance having a boiling point of 260°C or lower under normal pressure.

[Hair Processing Agent or Fiber Processing Agent for Headwear Product]

The hair processing agent or the fiber processing agent for a headwear product of the present invention is a hair processing agent or a fiber processing agent for a headwear product of the following (I) or (II):

(I) in which the hair processing agent or the fiber processing agent for a headwear product contains the following components (A) to (C):

(A) a film-forming polymer containing an M unit represented by $(R^1)_3SiO_{1/2}$ ($R^1$ represents a hydrocarbon group having 1 or more and 12 or less carbon atoms and optionally substituted with fluorine, or a hydroxy group, and a plurality of $R^1$'s may be the same or different from each other) and a Q unit represented by $SiO_{4/2}$;
(B) a film-forming polymer other than the component (A); and
(C) a high-molecular-weight organopolysiloxane having a degree of polymerization of 2,200 or more and 20,000 or less, and

in which a content of water is 10% by mass or less;
(II) in which the hair processing agent or the fiber processing agent for a headwear product contains the following components (A), (B1), (C), and (D):

(A) a film-forming polymer containing an M unit represented by $(R^1)_3SiO_{1/2}$ ($R^1$ represents a hydrocarbon group having 1 or more and 12 or less carbon atoms and optionally substituted with fluorine, or a hydroxy group, and a plurality of $R^1$'s may be the same or different from each other) and a Q unit represented by $SiO_{4/2}$;
(B1) a film-forming polymer other than the component (A), excluding a cationic polymer containing a quaternary ammonium cation;
(C) a high-molecular-weight organopolysiloxane having a degree of polymerization of 2,200 or more and 20,000 or less; and
(D) an organopolysiloxane other than the components (A), (B 1), and (C), having a polyalkylene oxide moiety and a cationic group.

Hereinafter, the hair processing agent or the fiber processing agent for a headwear product of the above (I) is also referred to as "the processing agent of the first invention" or "the processing agent (I)", the hair processing agent or the fiber processing agent for a headwear product of the above (II) is also referred to as "the processing agent of the second invention" or "the processing agent (II)", and the hair processing agents or the fiber processing agents for a headwear product of the above (I) and (II) are also collectively referred to as "the processing agent of the present invention".

By having the above-described constitution, the hair processing agent or the fiber processing agent for a headwear product (I) of the first invention can impart a good touch feeling to hair or a fiber for a headwear product in either a dry state or a wet state, exhibit excellent property of imparting a feeling of volume and heat setting property during styling, and suppress a decrease in effects such as the property of imparting a feeling of volume and the heat setting property even after hair washing.

Further, by having the above-described constitution, the hair processing agent or the fiber processing agent for a headwear product (II) of the second invention can impart a good touch feeling to hair or a fiber for a headwear product in either a dry state or a wet state, exhibit excellent heat setting property and slipperiness of a hair iron during styling, and suppress a decrease in the heat setting property even after hair washing.

The reason why the hair processing agent or the fiber processing agent for a headwear product of the present invention exhibits the above-mentioned effects is not clear, but is presumed as follows.

The hair processing agent or the fiber processing agent for a headwear product (I) of the first invention contains the component (A) and the component (B), and thus when applied to hair or a fiber for a headwear product, a hydrophobic film can be formed on the surface of the hair or the fiber for a headwear product. It is considered that this film-forming effect also improves the property of imparting a feeling of volume and the heat setting property during styling.

Since the component (A) contains the Q unit, a hard film can be formed, but since the film is hard and brittle, it is easily peeled off by friction or washing, and further, the feeling of the hair or the fiber for a headwear product may be deteriorated. However, since the hair processing agent or the fiber processing agent for a headwear product of the present invention contains the component (B) together with the component (A), it is possible to form a film which is more flexible than a film formed of only the component (A), and therefore, it is considered that a decrease in the touch feeling of the hair or the fiber for a headwear product and a decrease in the property of imparting a feeling of volume after hair washing and the heat setting property can also be suppressed.

It is considered that a large amount of the component (C) is present on the film surface side (air side) in the hydrophobic film to be formed, and thus the component (C) serves as a protective function for the surface of hair or the surface of a fiber for a headwear product, and also contributes to the touch feeling, the property of imparting a feeling of volume during styling, and the improvement of durability. In particular, it is considered that since the component (C) has a predetermined degree of polymerization, it is possible to suppress the occurrence of stickiness and impart a good feeling to hair or fibers for headwear products in both a dry state and a wet state, and to improve the property of imparting a feeling of volume during styling.

In addition, it is considered that in a case where the content of water in the hair processing agent or the fiber processing agent for a headwear product (I) of the first invention is 10% by mass or less, the drying speed is improved, and when the hair processing agent or the fiber processing agent for a headwear product (I) is applied to hair or fibers for a headwear product, a film having a phase separation structure in which the component (C) is unevenly distributed on the surface side (air side) is efficiently formed, and thus the effects of the present invention are more easily exhibited.

The hair processing agent or the fiber processing agent for a headwear product (II) of the second invention contains the component (A) and the component (B1), and thus when applied to hair or a fiber for a headwear product, a highly hydrophobic film can be formed on the surface of the hair or the fiber for a headwear product. It is considered that the heat setting property during styling is improved by the film forming effect.

Since the component (A) contains the Q unit, a hard film can be formed, but since the film is hard and brittle, it is easily peeled off by friction or washing, and further, the feeling of the hair or the fiber for a headwear product may be deteriorated. However, since the hair processing agent or the fiber processing agent for a headwear product (II) of the second invention contains the component (B1) together with the component (A), it is possible to form a film which is more flexible than a film formed of only the component (A), and therefore, it is considered that a decrease in the touch feeling of the hair or the fiber for a headwear product and a decrease in the heat setting property after hair washing can also be suppressed.

It is considered that the component (C) is phase-separated in the hydrophobic film to be formed and is present in a large amount on the film surface side (air side). Thus, it is considered that the component (C) serves as a protective function for the surface of hair or the surface of a fiber for a headwear product, and also contributes to the improvement of touch feeling and the improvement of durability. In particular, it is considered that since the component (C) has a predetermined degree of polymerization, it is possible to suppress the occurrence of stickiness and impart a good feeling to hair or fibers for headwear products in both a dry state and a wet state.

It is considered that the component (D) has an effect of accelerating the phase separation in the hydrophobic film. Due to this effect, the component (C) having high hydrophobicity and low surface tension is likely to be unevenly distributed

on the film surface side, the frictional force of the film surface to be formed is reduced, and the effects of improving the touch feeling, durability, and slipperiness of the hair iron can be obtained.

It should be noted that the action mechanism of the present invention is not limited to the above.

<Hair or Fiber for Headwear Product>

The application target of the processing agent of the present invention is hair or a fiber for a headwear product. Among these, hair is more preferable.

The fiber for a headwear product to which the processing agent of the present invention is applied may be either a naturally derived fiber or a synthetic fiber, but the naturally derived fiber is preferred. The naturally derived fibers refer to fibers collected from natural animals and plants, or fibers artificially produced using, as a raw material, polymers or oligomers such as proteins or polysaccharides derived from collagen, casein, soybean, peanut, corn, silk waste, or silk protein (for example, silk fibroin). Among these, fibers artificially produced using, as a raw material, polymers or oligomers such as animal hair, human hair, or proteins or polysaccharides derived from keratin, collagen, casein, soybean, peanut, corn, silk waste, or silk protein (for example, silk fibroin) are preferable; regenerated protein fibers using, as a raw material, proteins derived from keratin, collagen, casein, soybean protein, peanut protein, corn protein, or silk protein (for example, silk fibroin) are more preferable; regenerated protein fibers such as regenerated collagen fibers using collagen as a raw material and regenerated silk fibers using silk fibroin as a raw material are more preferable; and regenerated collagen fibers are still more preferable.

The regenerated collagen fibers can be produced by a known technique. The composition of the regenerated collagen fibers need not be 100% collagen, and may contain natural polymers, synthetic polymers, and additives for quality improvement. Furthermore, the regenerated collagen fibers may be those subjected to post-processing or post-processing. The regenerated collagen fibers are preferably in the form of filaments. The filament is generally taken out from a bobbin-wound state or a packed state. It is also possible to directly utilize the filament coming out from the drying step in the production process of the regenerated collagen fiber.

Examples of the synthetic fiber include a fiber containing a synthetic resin as a main component. From the viewpoint of ease of production of the synthetic fiber and from the viewpoint of obtaining a texture close to that of hair, the synthetic resin is preferably a thermoplastic resin, and more preferably one or more selected from the group consisting of a polyester resin, a polyamide resin, a polyimide resin, a polyamide-imide resin, a vinyl chloride resin, a polycarbonate resin, a polyphenylene sulfide resin, and a modacrylic resin (a copolymer of acrylonitrile and vinyl chloride). The term "main component" as used herein means a component having a content in the synthetic fiber of preferably 50% by mass or more, more preferably 60% by mass or more, still more preferably 70% by mass or more, even more preferably 80% by mass or more, and even more preferably 90% by mass or more, and 100% by mass or less.

The synthetic fiber may further contain various additives such as a flame retardant, a flame retardant aid, a light or heat stabilizer, a fluorescent agent, an antioxidant, an anti-static agent, and an ultraviolet absorber in addition to the synthetic resin within a range not inhibiting the effect of the present invention.

Examples of the hair processing agent or the fiber processing agent for a headwear product include a detergent such as shampoo, a rinse, a conditioning agent, a treatment agent (including a non-washout type), a styling agent, a hair dye, and a hair growth agent.

It is preferable that the hair processing agent or the fiber processing agent for a headwear product of the present invention is used without being washed off after being applied to the hair or the fiber for a headwear product, from the viewpoint of the effects of the present invention that an effect of improving the touch feeling of the hair or the fiber for a headwear product in a dry state and a wet state, an effect of imparting a feeling of volume, a heat setting property, a slipperiness of a hair iron during styling, and a sustained effect of imparting a feeling of volume and a heat setting property after hair washing are obtained. Therefore, among the above, the processing agent of the present invention is preferably a conditioning agent, a treatment agent, or a styling agent, and more preferably a styling agent.

Hereinafter, each component contained in the hair processing agent or the fiber processing agent for a headwear product of the present invention will be described.

<Component (A): Film-Forming Polymer Containing M Unit and Q Unit>

The processing agent of the present invention contains, as a component (A), a film-forming polymer containing an M unit represented by $(R^1)_3SiO_{1/2}$ ($R^1$ represents a hydrocarbon group having 1 or more and 12 or less carbon atoms and optionally substituted with fluorine or a hydroxy group, and a plurality of $R^1$'s may be the same or different from each other) and a Q unit represented by $SiO_{4/2}$.

Since the component (A) contains the Q unit, the component (A) has a crosslinked structure in the molecule. It is considered that by having this structure, a hard film can be formed and the property of imparting a feeling of volume and the heat setting property during styling, and the sustained effect of the property of imparting a feeling of volume and the heat

setting property after hair washing are obtained. The component (A) does not contain a polyorganosiloxane cured product powder which is infusible and does not have a softening point and which is generally insoluble in an organic solvent.

In the formula, $R^1$ is a hydrocarbon group having 1 or more and 12 or less carbon atoms and optionally substituted with fluorine, or a hydroxy group. The number of carbon atoms of the hydrocarbon group is 1 or more, and is preferably 9 or less, more preferably 6 or less, and still more preferably 4 or less, from the viewpoint of the film formability, the property of imparting a feeling of volume and the heat setting property during styling, and improving the sustained effect of the property of imparting a feeling of volume and the heat setting property after hair washing.

The hydrocarbon group may be any of an aliphatic group or an aromatic group, and examples thereof include an alkyl group, an alkenyl group, an aryl group, and an aralkyl group. The alkyl group and the alkenyl group may be linear or branched.

Among the above, from the viewpoint of availability and stability, the hydrocarbon group is preferably an alkyl group, an aryl group, or an aralkyl group.

The alkyl group includes a methyl group, an ethyl group, a n-propyl group, an isopropyl group, various butyl groups, various pentyl groups, various hexyl groups, various heptyl groups, various octyl groups, various nonyl group, various decyl groups, various undecyl groups, and various dodecyl groups. The word "various" means a linear or branched hydrocarbon group, and for example, "various butyl groups" include "a n-butyl group, a sec-butyl group, an isobutyl group, and a tert-butyl group".

The aryl group includes a phenyl group, a toluyl group, a dimethylphenyl group, and a naphthyl group, and is preferably a phenyl group.

The aralkyl group includes a benzyl group, a phenylethyl group, a phenylpropyl group, and a phenylbutyl group, and is preferably a phenylpropyl group.

In the case where $R^1$ is substituted with fluorine, at least one hydrogen atom of the hydrocarbon group may be substituted with a fluorine atom.

$R^1$ is preferably an alkyl group having 1 or more and 12 or less carbon atoms, an aryl group having 6 or more and 12 or less carbon atoms, or an aralkyl group having 7 or more and 12 or less carbon atoms, each optionally substituted with fluorine, more preferably an alkyl group having 1 or more and 8 or less carbon atoms or a phenyl group, each optionally substituted with fluorine, and still more preferably an alkyl group having 1 or more and 6 or less carbon atoms or a phenyl group, each optionally substituted with fluorine, from the viewpoint of the film formability, the property of imparting a feeling of volume and the heat setting property during styling, and improving the sustained effect of the property of imparting a feeling of volume and the heat setting property after hair washing. The fluorine-substituted alkyl group is preferably a group represented by $CF_3$-R- in which R represents an alkylene group having 2 or more and 7 or less carbon atoms, preferably 2 or more and 5 or less carbon atoms.

$R^1$ is even more preferably a trifluoropropyl group, an alkyl group having 1 or more and 4 or less carbon atoms, or a phenyl group, even more preferably a trifluoropropyl group, a methyl group, an ethyl group, a n-propyl group, an isopropyl group or a n-butyl group, even more preferably a trifluoropropyl group, a methyl group or a n-propyl group, and even more preferably a methyl group.

The component (A) may further contain one or more units selected from the group consisting of a T unit represented by $R^1SiO_{3/2}$ and a D unit represented by $(R^1)_2SiO_{2/2}$, in which $R^1$ is the same as above.

However, from the viewpoint of the film formability, the property of imparting a feeling of volume and the heat setting property during styling, and improving the sustained effect of the property of imparting a feeling of volume and the heat setting property after hair washing, it is preferable that a ratio of the M unit and the Q unit in the entire unit constituting the component (A) is high. Specifically, the ratio of the total number of moles of the M unit and the Q unit to the total number of moles of the M unit, the Q unit, the T unit, and the D unit in the component (A) is preferably 50% or more, more preferably 70% or more, still more preferably 80% or more, and even more preferably 90% or more, and is 100% or less.

Examples of the component (A) include trimethylsiloxysilicate, phenylpropyldimethylsiloxysilicate, fluorine-modified alkyldimethylsiloxysilicates, or crosspolymers produced by crosslinking these siloxysilicates with dimethiconol or the like, and one or more of these can be used. Fluorine-modified alkyldimethylsiloxysilicates include trifluoroalkyldimethyltrimethylsiloxysilicate, such as trifluoropropyldimethyltrimethylsiloxysilicate of, as ICNI nomenclature, Trifluoropropyldimethyl/Trimethylsiloxysilicate. Crosspolymers produced by crosslinking siloxysilicates with dimethiconol or the like include, as ICNI nomenclature, (trimethylsiloxysilicate/dimethiconol) crosspolymer.

Above all, from the viewpoint of the film formability, the property of imparting a feeling of volume and the heat setting property during styling, and improving the sustained effect of the property of imparting a feeling of volume and the heat setting property after hair washing, the component (A) is preferably one or more selected from the group consisting of trimethylsiloxysilicate, trifluoropropyldimethyltrimethylsiloxysilicate and (trimethylsiloxysilicate/dimethiconol) crosspolymer, more preferably one or more selected from the group consisting of trimethylsiloxysilicate, and trifluoropropyldimethyltrimethylsiloxysilicate, and still more preferably trimethylsiloxysilicate.

Various substances are used as the trimethylsiloxysilicate, and the viscosity of a 50% by mass solution in which the trimethylsiloxysilicate as an active ingredient is dissolved in dimethicone KF-96L-2cs (manufactured by Shin-Etsu

Chemical Co., Ltd.) is preferably 10 $mm^2/s$ or more, more preferably 15 $mm^2/s$ or more, still more preferably 20 $mm^2/s$ or more, and even more preferably 50 $mm^2/s$ or more. These viscosities are values measured at 25°C by the same method as the viscosity of the component (C) described later.

Commercially available products of trimethylsiloxysilicate of the component (A) include KF-7312J (50% by mass decamethylcyclopentasiloxane solution), KF-9021 (50% by mass decamethylcyclopentasiloxane solution), X-21-5249(50% by mass decamethylcyclopentasiloxane solution), X-21-5595 (60% by mass isododecane solution), and X-21-5616 (60% by mass isododecane solution) (all manufactured by Shin-Etsu Chemical Co., Ltd.), SS4267 (35% by mass dimethylpolysiloxane solution), SR1000, SS4230 (45% by mass cyclopentasiloxane solution), SS4267 (35% by mass dimethylpolysiloxane solution), and Silsoft 74 (75% by mass isododecane solution) (all manufactured by Momentive Performance Materials, Inc.), BY11-018 (30% by mass cyclopentasiloxane solution), and MQ-1600 Solid Resin (all manufactured by Dow Toray Co., Ltd.), and BELSIL TMS 803 (manufactured by Wacker Asahikasei Silicone Co., Ltd.).

Commercially available products of phenylpropyldimethylsiloxysilicate include SilShine 151 (manufactured by Momentive Performance Materials, Inc.).

Commercially available products of fluorine-modified alkyldimethylsiloxysilicates include, XS66-B8226 (50% by mass cyclopentasiloxane solution), XS66-C1191, and XS66-B8636 (50% by mass dimethicone solution) (all manufactured by Momentive Performance Materials, Inc.), as INCI nomenclature, (Trifluoropropyldimethyl/Trimethylsiloxysilicate).

In addition, commercially available products of trimethylsiloxysilicate crosspolymers include DOWSIL FC-5002 IDD Resin Gum (a 40% by mass isododecane solution of a trimethylsiloxysilicate/dimethiconol crosspolymer) (manufactured by Dow Toray Co., Ltd.).

<Component (B): Film-Forming Polymer other than Component (A), Component (B1): Film-Forming Polymer other than Component (A) excluding Cationic Polymer Containing Quaternary Ammonium Cation>

The component (B) used in the processing agent (I) is a film-forming polymer other than the component (A). The component (B1) used in the processing agent (II) is a film-forming polymer other than the component (A), excluding a cationic polymer containing a quaternary ammonium cation.

As the component (B) and the component (B1), a film-forming polymer which is usually used in cosmetics can be used, but a film-forming polymer which is solid at 25°C is preferable.

As the component (B) and the component (B1), any of silicone-based film-forming polymers and non-silicone-based film-forming polymers can be used.

Examples of the silicone-based film-forming polymer used as the component (B) and the component (B1) include a silicone resin containing a T unit represented by $R^1SiO_{3/2}$ and substantially not containing a Q unit represented by $SiO_{4/2}$ ($R^1$ is the same as described above), and a silicone polymer containing a polysiloxane moiety and a moiety composed of a non-silicone organic chain. The silicone resin substantially not containing a Q unit means that the constitutional ratio of the Q unit in the silicone resin is less than 1 mol%.

Examples of the silicone resin containing a T unit represented by $R^1SiO_{3/2}$ and substantially not containing a Q unit represented by $SiO_{4/2}$ ($R^1$ is the same as described above) include polysilsesquioxanes such as polymethylsilsesquioxane, polypropylsilsesquioxane, polyphenylsilsesquioxane, polymethylphenylsilsesquioxane, and fluorine-modified alkyldimethylpolysilsesquioxanes, and one or more of these can be used. Fluorine-modified alkyldimethylpolysilsesquioxanes include, as INCI nomenclature, (Trifluoropropyldimethylsiloxy/Trimethylsiloxy)silsesquioxane.

Examples of the non-silicone-based film-forming polymer used as the component (B) and the component (B1) include non-silicone-based cationic polymers, non-silicone-based anionic polymers, non-silicone-based nonionic polymers, and non-silicone-based ampholytic polymers. These polymers are preferably hydrophobic polymers.

Examples of the cationic polymer used as the component (B) include vinylpyrrolidone/N,N-dimethylaminoethyl methacrylate copolymer diethyl sulfate (polyquaternium-11) (Gafquat 734 (manufactured by Ashland Specialty Ingredients), H. C. polymer (manufactured by Osaka Organic Chemical Industry Ltd.)), a vinylpyrrolidone/dimethylaminopropylmethacrylamide/lauryldimethylaminopropylmethacryl amide copolymer (Stylese W-20 (manufactured by Ashland Specialty Ingredients)), polychlorinated dimethylmethylenepiperidinium (Merquat 100 (manufactured by Lubrizol Corporation)), a dimethyldiallylammonium chloride/acrylamide copolymer (Merquat 550 (manufactured by Lubrizol Corporation)), a vinylpyrrolidone/dimethylaminoethyl methacrylate copolymer (Gafquat 440 (manufactured by Ashland Specialty Ingredients)), ammonium-modified hydroxyethyl cellulose (SoftCAT polymer SL-30 (manufactured by Dow Chemical Company)), and an acrylamide/acrylic acid DMAPA/methacrylic acid methoxy PEG copolymer.

Examples of the cationic polymer used as the component (B1) include a vinylpyrrolidone/alkylamino (meth)acrylate copolymer, a vinylpyrrolidone/alkylamino (meth)acrylate/vinylcaprolactam copolymer, an alkylacrylamide/(meth)acrylate/alkylaminoalkylacrylamide/polyethylene glycol (meth)acrylate copolymer, an adipic acid/dimethylaminohydroxypropylethylenetriamine copolymer, and an acrylamide/acrylic acid DMAPA/methacrylic acid methoxy PEG copolymer.

Examples of the anionic polymer include (acrylates/diacetoneacrylamide) copolymers (Plascize L-9540B,

Plascize L-9600U, and Plascize L-53 (manufactured by Goo Chemical Co., Ltd.)), (acrylates/alkyl (C1-18) acrylate/alkyl (C1-8) acrylamide) copolymers (Plascize L-9909B, Plascize L-9900 (manufactured by Goo Chemical Co., Ltd.)), an alkyl acrylate/octylacrylamide copolymer (Dermacryl 79 (manufactured by Akzo Nobel)), a vinyl acetate/crotonic acid/vinyl neodecanoate copolymer (RESYN 28-2930 (manufactured by Akzo Nobel)), acrylic acid/acrylamide/ethyl acrylate copolymers (Ultrahold 8, Ultrahold Strong (manufactured by BASF)), alkyl acrylate copolymers (Aniset NF-1000, Aniset HS-300, and the like (manufactured by Osaka Organic Chemical Industry Ltd.)), and an isophorone diisocyanate/dimethylolpropionic acid/(polyoxyethylene/polyoxypropylene) 4,4'-isopropylidenediphenol copolymer (DynamX (manufactured by Akzo Nobel)).

Examples of the nonionic polymer include an acrylates/methoxy methacrylate PEG-23 copolymer (Plascize L-188K (manufactured by Goo Chemical Co., Ltd.)), dimethylacrylamide/hydroxyethylacrylate/methoxyethylacrylate copolymers (Plascize L-2700 and Plascize L-2714 (manufactured by Goo Chemical Co., Ltd.)), a hydroxyethylacrylate/methoxyethylacrylate copolymer (Plascize L-2200 (manufactured by Goo Chemical Co., Ltd.)), polyvinylpyrrolidones (Luviskol K17, Luviskol K30, and Luviskol K90 (manufactured by BASF)), vinylpyrrolidone/vinyl acetate copolymers (Luviskol VA73E and Luviskol 37E (manufactured by BASF)), vinyl methyl ether/alkyl maleate copolymers (Gantrez A-425 and Gantrez ES-225 (manufactured by Ashland Specialty Ingredients)), a vinylpyrrolidone/methacrylamide/vinylimidazole copolymer (Luviset Clear (manufactured by BASF)), and a polyvinylcaprolactam (Luviskol Plus (manufactured by BASF)).

Examples of the ampholytic polymer include an acrylates/lauryl acrylate/stearyl acrylate/ethyl methacrylate amine oxide copolymer (Diaformer Z651 (manufactured by Mitsubishi Chemical Corporation)), methacryloyloxyethyl carboxybetaine/alkyl methacrylate copolymers (Yukaformer M75 and Yukaformer R205 (manufactured by Mitsubishi Chemical Corporation)), an octylacrylamide/hydroxypropyl acrylate/butyl aminoethyl methacrylate copolymer (AMPHOMER 28-4910 (manufactured by Akzo Nobel)), and an octylamide acrylate/hydroxypropyl acrylate/butyl aminoethyl methacrylate copolymer (AMPHOMER SH30 (manufactured by Akzo Nobel)).

From the viewpoint of the touch feeling of hair or a fiber for a headwear product in a dry state or a wet state, the property of imparting a feeling of volume and the heat setting property during styling, and improving the sustained effect of the property of imparting a feeling of volume and the heat setting property after hair washing, the component (B) and the component (B1) are preferably a silicone-based film-forming polymer, and more preferably a silicone polymer containing a polysiloxane moiety and a moiety composed of a non-silicone organic chain. The non-silicone organic monomer to constitute the moiety composed of a non-silicone organic chain is, from the viewpoint of availability on the market, preferably selectable from a radical-polymerizable ethylenically-unsaturated monomer, a polycondensation-polymerizable monomer (e.g., those to form polyamides, polyesters, or polyurethanes), and a ring-cleavable monomer (e.g., oxazoline or caprolactone-type ones).

As the silicone polymer used as the component (B) and the component (B1) and containing a polysiloxane moiety and a moiety composed of a non-silicone organic chain, a silicone polymer including one or more selected from the group consisting of the following components (B-1) to (B-5) is preferably exemplified.

(B-1) An acryl silicone polymer
(B-2) A silicone-modified alicyclic structure-containing polymer
(B-3) A silicone-modified pullulan
(B-4) A polyurea/urethane silicone
(B-5) An oxazoline-modified silicone

[Acryl Silicone Polymer (B-1)]

The acryl silicone polymer of the component (B-1) includes an acrylic polymer having a carbosiloxane dendrimer structure in the side chain, an acryl-silicone graft copolymer, and a graft-type copolymer or alternate block-type copolymer where a structural unit of a polysiloxane group and a structural unit of a polymer of an unsaturated monomer bond via a sulfide bond.

The acrylic polymer having a carbosiloxane dendrimer structure in the side chain includes a silicone dendrimer-acryl copolymer, and for example, can be produced according to the production method described in JP H11-1530 A and JP 2000-63225 A.

The acrylic polymer having a carbosiloxane dendrimer structure in the side chain is preferably, as INCI nomenclature, Acrylates/Polytrimethylsiloxymethacrylate Copolymer. Commercially available products thereof include DOWSIL FA 4001 CM Silicone Acrylate (30% by mass decamethylcyclopentasiloxane solution), DOWSIL FA 4002 ID Silicone Acrylate (40% by mass isododecane solution), DOWSIL FA 4003 DM Silicone Acrylate (40% by mass dimethicone solution), and DOWSIL FA 4004 ID Silicone Acrylate (40% by mass isododecane solution) (all manufactured by Dow Toray Co., Ltd.).

The acryl-silicone graft copolymer includes a radical polymer of an organopolysiloxane compound having a

radical polymerizable group at one terminal of the molecular chain and a radical polymerizable monomer mainly composed of an acrylate and/or a methacrylate.

Examples of the radical polymer of an organopolysiloxane compound having a radical polymerizable group at one terminal of the molecular chain and a radical polymerizable monomer mainly composed of an acrylate and/or a methacrylate usable here include those described in JP H2-25411 A and JP H2-132141 A, and acryl-silicone graft copolymers described in JP H3-162442 A and JP 2003-104825 A.

The acryl-silicone graft copolymer is preferably, as INCI nomenclature, (Acrylates/Dimethicone) Copolymer. Commercially available products thereof include KP-545 (30% by mass decamethylcyclopentasiloxane solution), KP-549 (40% by mass methyltrimethicone solution), and KP-550 (40% by mass isododecane solution) (all manufactured by Shin-Etsu Chemical Co., Ltd.).

The graft-type copolymer or alternate block-type copolymer where a structural unit of a polysiloxane group and a structural unit of a polymer of an unsaturated monomer bond via a sulfide bond include graft-type copolymers or alternate block-type copolymers described in JP H6-92825 A.

Above all, from the viewpoint of the film formability, the touch feeling of hair or a fiber for a headwear product in a dry state or a wet state, the property of imparting a feeling of volume and the heat setting property during styling, and improving the sustained effect of the property of imparting a feeling of volume and the heat setting property after hair washing, the component (B-1) is preferably one or more selected from the group consisting of an acrylic polymer having a carbosiloxane dendrimer structure in the side chain and an acryl-silicone graft copolymer, more preferably one or more selected from (acrylates/polytrimethylsiloxymethacrylate) copolymer and (acrylates/dimethicone) copolymer, and still more preferably (acrylates/dimethicone) copolymer.

[Silicone-Modified Alicyclic Structure-Containing Polymer (B-2)]

Examples of the silicone-modified alicyclic structure-containing polymer include silicone-modified cyclic polyolefins, and preferred examples thereof include silicone-modified polynorbornenes represented by the following general formula (B-2-1).

[Chem 1]

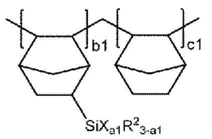

(B-2-1)

In the formula, each $R^2$ independently represents an alkyl group having 1 or more and 12 or less carbon atoms, X represents a group represented by the following formula (i), a1 is an integer of 1 or more and 3 or less, b1 and c1 each are a repeating unit number, and are each independently an integer of 1 or more.

[Chem 2]

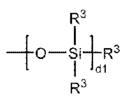

(i)

In the formula, each $R^3$ independently represents a hydrocarbon group having 1 or more and 12 or less carbon atoms, and d1 is an integer of 1 or more and 5 or less.

In the general formula (B-2-1), $R^2$ is preferably a methyl group, an ethyl group, a n-propyl group, a butyl group, or a pentyl group, and more preferably a methyl group.

X is a group represented by the formula (i), and in the formula (i), each $R^3$ is independently a hydrocarbon group having 1 or more and 12 or less carbon atoms. $R^3$ is preferably an alkyl group having 1 or more and 12 or less carbon atoms or a phenyl group, more preferably an alkyl group having 1 or more and 3 or less carbon atoms, and even more preferably a methyl group. d1 is an integer of 1 or more and 5 or less, and is, from the viewpoint of versatility, preferably d1 = 1. Specifically, X is preferably a trimethylsiloxy group.

a1 is an integer of 1 or more and 3 or less, and, for example, in the polymer, a repeating unit of a1 = 2 and a repeating unit of a1 = 3 may exist as mixed. From the viewpoint of versatility, a1 is preferably 3.

The proportion of b1 and c1 in the general formula (B-2-1) is preferably b1/c1 = 20/80 to 90/10 (mol/mol), more preferably 30/70 to 80/20 (mol/mol), and still more preferably 50/50 to 70/30 (mol/mol). The proportion of b1 and c1 can be determined by $^1$H-NMR measurement.

The silicone-modified polynorbornene is preferably a silicone-modified polynorbornene represented by the following formula (B-2-2).

[Chem 3]

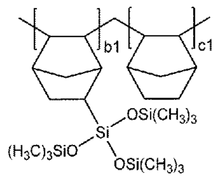

(B-2-2)

In the formula, b1 and c1 are the same as above.

Examples of the silicone-modified polynorbornene represented by the formula (B-2-2) include a compound of, as INCI nomenclature, (NORBORNENE/TRIS(TRIMETHYLSILOXY)SILYLNORBORNENE COPOLYMER).

Commercially available products of the silicone-modified polynorbornene include NBN-30-ID (isododecane solution of (norbornene/tris(trimethylsiloxy)silylnorbornene) copolymer) (by Shin-Etsu Chemical Co., Ltd.).

[Silicone-Modified Pullulan (B-3)]

The silicone-modified pullulan includes a pullulan having a silicone structure in the side chain, and specifically preferred is a silicone-modified pullulan in which at least a part of the hydrogen atoms of the OH groups in pullulan are substituted with a group represented by the following general formula (ii).

[Chem 4]

$$—R^4—SiX_{a1}R^2_{3-a1} \quad \text{(ii)}$$

In the formula, $R^4$ represents a single bond or a divalent organic group, and $R^2$, X and a1 are the same as above. From the viewpoint of versatility, X is preferably a trimethylsiloxyl group, and a1 is preferably 3.

In the general formula (ii), $R^4$ is preferably a divalent organic group, more preferably a divalent group represented by the following general formula (iii) or (iv), and even more preferably a divalent group represented by the general formula (iv).

[Chem 5]

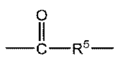

(iii)

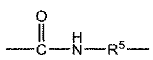

(iv)

In the formulae, $R^5$ represents an alkylene group having 1 or more and 10 or less carbon atoms, and examples thereof include a methylene group, an ethylene group, a trimethylene group, a propylene group, and a butylene group. Among these, preferred are an ethylene group, a trimethylene group and a propylene group; and more preferred are a trimethylene group and a propylene group.

Commercially available products of the silicone-modified pullulan include TSPL-30-ID (isododecane solution of tri(trimethylsiloxy)silylpropylcarbamate pullulan), and TSPL-30-D5 (cyclopentasiloxane solution of tri(trimethylsiloxy) silylpropylcarbamate pullulan) (all manufactured by Shin-Etsu Chemical Co., Ltd.).

[Polyurea/Urethane Silicone (B-4)]

Examples of the polyurea/urethane silicone of the component (B-4) include a polysiloxane/polyurea/polyurethane block terpolymer. For example, it is a dimethylpolysiloxane/urea copolymer of "polyurea-dimethicone" as INCI nomenclature.

The polymer can be produced by copolymerization of an α,ω-aminosilicone and a diisocyanate. Commercially available products of the polyurea/urethane silicone include Wacker-Belsil UD 60, Wacker-Belsil UD 80, Wacker-Belsil UD 140, and Wacker-Belsil UD 200 (all manufactured by Wacker Corporation).

[Oxazoline-Modified Silicone (B-5)]

Examples of the oxazoline-modified silicone as the component (B-5) include an organopolysiloxane in which a poly(N-acylalkyleneimine) segment composed of a repeating unit represented by the following general formula (B-5-1):

[Chem 6]

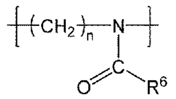

(B-5-1)

(in the formula, $R^6$ represents a hydrogen atom, an alkyl group having 1 or more and 22 or less carbon atoms, an aralkyl group having 7 or more and 22 or less carbon atoms, or an aryl group having 6 or more and 22 or less carbon atoms; and n represents 2 or 3) is bonded to at least two silicon atoms of an organopolysiloxane segment constituting a main chain via an alkylene group containing a heteroatom.

At least two poly(N-acylalkyleneimine) segments can be bonded to any silicon atom constituting the organopolysiloxane segment via an alkylene group containing a hetero atom, and the poly(N-acylalkyleneimine) segment is preferably bonded to one or more silicon atoms excluding both terminals via the alkylene group, and more preferably bonded to two or more silicon atoms excluding both terminals via the alkylene group.

The alkylene group containing a heteroatom functions as a linking group of the poly(N-acylalkyleneimine) segment. Examples of such an alkylene group include an alkylene group having 2 or more and 20 or less carbon atoms and containing 1 to 3 nitrogen atoms, oxygen atoms, or sulfur atoms.

In $R^6$ in the general formula (B-5-1), examples of the alkyl group having 1 or more and 22 or less carbon atoms include linear, branched, or cyclic alkyl groups having 1 or more and 22 or less carbon atoms. Specifically, examples thereof include a methyl group, an ethyl group, a n-propyl group, an isopropyl group, a n-butyl group, a tert-butyl group, a pentyl group, a hexyl group, a cyclohexyl group, a heptyl group, an octyl group, a nonyl group, a decyl group, an undecyl group, a dodecyl group, an octadecyl group, a nonadecyl group, an eicosyl group, and a docosyl group. Among these, an alkyl group having 1 or more and 10 or less carbon atoms is preferable, and an alkyl group having 1 or more and 6 or less carbon atoms is more preferable.

In $R^6$ in the general formula (B-5-1), specific examples of the aralkyl group having 7 or more and 22 or less carbon atoms include a benzyl group, a phenethyl group, a trityl group, a naphthylmethyl group, and an anthracenylmethyl group. Among these, an aralkyl group having 7 or more and 14 or less carbon atoms is preferable, and an aralkyl group having 7 or more and 10 or less carbon atoms is more preferable, from the viewpoint of imparting a feeling of volume to the hair or the fiber for a headwear product.

In $R^6$ in the general formula (B-5-1), specific examples of the aryl group having 6 or more and 22 or less carbon atoms include a phenyl group, a tolyl group, a xylyl group, a naphthyl group, a biphenyl group, an anthryl group, and a phenanthryl group. Among these, an aryl group having 6 or more and 14 or less carbon atoms is preferable, and an aryl group having 6 or more and 12 or less carbon atoms is more preferable, from the viewpoint of imparting a feeling of volume to the hair or the fiber for a headwear product.

Among the above, $R^6$ in the general formula (B-5-1) is preferably an alkyl group having 1 or more and 22 or less carbon atoms, more preferably an alkyl group having 1 or more and 10 or less carbon atoms, still more preferably an alkyl group having 1 or more and 6 or less carbon atoms, and even more preferably an alkyl group having 1 or more and 3 or less carbon atoms.

Examples of the oxazoline-modified silicone as the component (B-5) include poly(N-formylethyleneimine) organosiloxane, poly(N-acetylethyleneimine) organosiloxane, and poly(N-propionylethyleneimine) organosiloxane, and one or more of these can be used. Among these, a polymer represented by INCI nomenclature "Polysilicone-9" is preferably used.

As the component (B) and the component (B1), one or two or more can be used. Among the above-described components, from the viewpoint of the film formability, the touch feeling of the hair or the fiber for a headwear product in a dry state and a wet state, the property of imparting a feeling of volume and the heat setting property during styling, and improving the sustained effect of the property of imparting a feeling of volume and the heat setting property after hair washing, the component (B) and the component (B1) preferably include one or more selected from the group consisting of the components (B-1) to (B-5), more preferably include one or more selected from the group consisting of the acryl silicone polymer as the component (B-1), and the oxazoline-modified silicone as the component (B-5), and still more preferably include the acryl silicone polymer as the component (B-1).

More specifically, from the viewpoint of the film formability, the touch feeling of the hair or the fiber for a headwear product in a dry state and a wet state, the property of imparting a feeling of volume and the heat setting property during styling, and improving the sustained effect of the property of imparting a feeling of volume and the heat setting property

after hair washing, the component (B) and the component (B1) preferably include one or more selected from the group consisting of an (acrylates/polytrimethylsiloxymethacrylate) copolymer, an (acrylates/dimethicone) copolymer, and an oxazoline-modified silicone, more preferably include one or more selected from the group consisting of an (acrylates/dimethicone) copolymer and an oxazoline-modified silicone, and still more preferably include an (acrylates/dimethicone) copolymer.

The total content of the component (B-1) and the component (B-5) in the component (B) and the component (B1) is preferably 50% by mass or more, more preferably 70% by mass or more, still more preferably 80% by mass or more, and even more preferably 90% by mass or more, and is 100% by mass or less, from the viewpoint of the film formability, the touch feeling of the hair or the fiber for a headwear product in a dry state and a wet state, the property of imparting a feeling of volume and the heat setting property during styling, and improving the sustained effect of the property of imparting a feeling of volume and the heat setting property after hair washing.

<Component (C): High-Molecular Weight Organopolysiloxane>

The processing agent of the present invention contains, as the component (C), a high-molecular weight organopolysiloxane having a degree of polymerization of 2,200 or more and 20,000 or less.

The degree of polymerization of the component (C) is 2,200 or more, preferably 2,600 or more, more preferably 2,900 or more, and still more preferably 3,200 or more, from the viewpoint of the touch feeling of the hair or the fiber for a headwear product in a dry state and a wet state, the heat setting property and the slipperiness of a hair iron during styling, the property of imparting a feeling of volume during styling, and improving the sustained effect of the property of imparting a feeling of volume and the heat setting property after hair washing, and is 20,000 or less, preferably 10,000 or less, more preferably 4,500 or less, still more preferably 4,000 or less, and even more preferably 3,900 or less, from the viewpoint of availability. The degree of polymerization of the component (C) is 2,200 or more and 20,000 or less, preferably 2,600 or more and 10,000 or less, more preferably 2,600 or more and 4,500 or less, still more preferably 2,900 or more and 4,000 or less, and even more preferably 3,200 or more and 3,900 or less.

More specifically, the component (C) is preferably an organopolysiloxane represented by the following general formula (1).

[Chem 7]

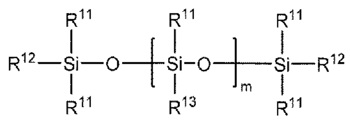
(1)

In the formula, each $R^{11}$ independently represents a hydrocarbon group having 1 or more and 6 or less carbon atoms, and each $R^{12}$ independently represents a hydroxy group, an alkoxy group having 1 or more and 6 or less carbon atoms, or a hydrocarbon group having 1 or more and 6 or less carbon atoms; $R^{13}$ represents a hydrocarbon group having 1 or more and 6 or less carbon atoms or a primary to tertiary amino group-containing group; m represents a degree of polymerization and is a number of 2,200 or more and 20,000 or less; and m'$sR^{13}$ groups may be the same as or different from each other.

In the general formula (1), the hydrocarbon group in $R^{11}$ may be either an aliphatic group or an aromatic group, and examples thereof include an alkyl group, an alkenyl group, and a phenyl group. The alkyl group and the alkenyl group may be linear or branched.

Among the above, $R^{11}$ is preferably an alkyl group having 1 or more and 6 or less carbon atoms or a phenyl group, more preferably an alkyl group having 1 or more and 3 or less carbon atoms or a phenyl group, still more preferably a methyl group or a phenyl group, and even more preferably a methyl group.

In the general formula (1), each $R^{12}$ independently represents a hydroxy group, an alkoxy group having 1 or more and 6 or less carbon atoms, or a hydrocarbon group having 1 or more and 6 or less carbon atoms.

Examples of the alkoxy group in $R^{12}$ include a methoxy group, an ethoxy group, a propoxy group, and a butoxy group.

The hydrocarbon group in $R^{12}$ is the same as in $R^{11}$, and is preferably an alkyl group having 1 or more and 6 or less carbon atoms or a phenyl group, more preferably an alkyl group having 1 or more and 3 or less carbon atoms or a phenyl group, still more preferably a methyl group or a phenyl group, and even more preferably a methyl group.

$R^{12}$ is preferably a hydroxy group, an alkyl group having 1 or more and 6 or less carbon atoms, or a phenyl group, more preferably a hydroxy group, an alkyl group having 1 or more and 3 or less carbon atoms, or a phenyl group, still more preferably a hydroxy group, a methyl group, or a phenyl group, and even more preferably a methyl group, from the viewpoint of the touch feeling of the hair or the fiber for a headwear product in a dry state and a wet state, the heat setting

property and the slipperiness of a hair iron during styling, the property of imparting a feeling of volume during styling, and improving the sustained effect of the property of imparting a feeling of volume and the heat setting property after hair washing.

In the general formula (1), $R^{13}$ represents a hydrocarbon group having 1 or more and 6 or less carbon atoms or a primary to tertiary amino group-containing group.

The hydrocarbon group in $R^{13}$ is the same as in $R^{11}$, and is preferably an alkyl group having 1 or more and 6 or less carbon atoms or a phenyl group, more preferably an alkyl group having 1 or more and 3 or less carbon atoms or a phenyl group, still more preferably a methyl group or a phenyl group, and even more preferably a methyl group.

The primary to tertiary amino group-containing group in $R^{13}$ (hereinafter, also simply referred to as "amino group-containing group") is preferably a group represented by $-N(R^{14})_2$, $-NR^{14}(CH_2)_pN(R^{14})_2$, or $-NR^{14}(CH_2)_pN(R^{15})CO-R^{16}$. Here, $R^{14}$ represents a hydrogen atom or a monovalent hydrocarbon group having 1 or more and 4 or less carbon atoms, and preferably a hydrogen atom, a methyl group, or an ethyl group. $R^{15}$ represents a monovalent hydrocarbon group having 1 or more and 4 or less carbon atoms, and is preferably a methyl group or an ethyl group. $R^{16}$ represents a monovalent hydrocarbon group having 1 or more and 4 or less carbon atoms. p is a number of 2 or more and 6 or less, and is preferably a number of 2 or more and 4 or less.

The amino group-containing group in $R^{13}$ is preferably $-(CH_2)_3-NH_2$, $-(CH_2)_3-N(CH_3)_2$, $-(CH_2)_3-NH-(CH_2)_2-NH_2$, or $-(CH_2)_2-NH-(CH_2)_2-N(CH_3)_2$, and more preferably $-(CH_2)_3-NH_2$.

$R^{13}$ is preferably a hydrocarbon group having 1 or more and 6 or less carbon atoms, more preferably an alkyl group having 1 or more and 6 or less carbon atoms, or a phenyl group, still more preferably an alkyl group having 1 or more and 3 or less carbon atoms, or a phenyl group, even more preferably a methyl group or a phenyl group, and even more preferably a methyl group, from the viewpoint of the touch feeling of the hair or the fiber for a headwear product in a dry state and a wet state, the heat setting property and the slipperiness of a hair iron during styling, the property of imparting a feeling of volume during styling, and improving the sustained effect of the property of imparting a feeling of volume and the heat setting property after hair washing.

In the general formula (1), m represents a degree of polymerization and is a number of 2,200 or more and 20,000 or less. m is preferably 2,600 or more, more preferably 2,900 or more, and still more preferably 3,200 or more, from the viewpoint of the touch feeling of the hair or the fiber for a headwear product in a dry state and a wet state, the heat setting property and the slipperiness of a hair iron during styling, the property of imparting a feeling of volume during styling, and improving the sustained effect of the property of imparting a feeling of volume and the heat setting property after hair washing, and is preferably 10,000 or less, more preferably 4,500 or less, still more preferably 4,000 or less, and even more preferably 3,900 or less, from the viewpoint of availability. Thus, m in the general formula (1) is 2,200 or more and 20,000 or less, preferably 2,600 or more and 10,000 or less, more preferably 2,600 or more and 4,500 or less, still more preferably 2,900 or more and 4,000 or less, and even more preferably 3,200 or more and 3,900 or less.

The viscosity of the component (C) is preferably 1,000,000 $mm^2/s$ or more, more preferably 3,000,000 $mm^2/s$ or more, still more preferably 5,000,000 $mm^2/s$ or more, and even more preferably 10,000,000 $mm^2/s$ or more from the viewpoint of the touch feeling of the hair or the fiber for a headwear product in a dry state and a wet state, the heat setting property and the slipperiness of a hair iron during styling, the property of imparting a feeling of volume during styling, and improving the sustained effect of the property of imparting a feeling of volume and the heat setting property after hair washing. On the other hand, from the viewpoint of availability, it is preferably 125,000,000 $mm^2/s$ or less, more preferably 50,000,000 $mm^2/s$ or less, and still more preferably 40,000,000 $mm^2/s$ or less.

Thus, the viscosity of the component (C) is preferably 1,000,000 $mm^2/s$ or more and 125,000,000 $mm^2/s$ or less, more preferably 3,000,000 $mm^2/s$ or more and 125,000,000 $mm^2/s$ or less, still more preferably 5,000,000 $mm^2/s$ or more and 50,000,000 $mm^2/s$ or less, and even more preferably 10,000,000 $mm^2/s$ or more and 40,000,000 $mm^2/s$ or less.

The viscosity is a value measured at 25°C based on JIS Z 8803:2011 "Methods for viscosity measurement of liquid". For example, the viscosity can be measured by selecting an appropriate viscometer from a capillary viscometer, a falling ball viscometer, a rotary viscometer, and a vibration viscometer. In addition, when the viscosity exceeds the common measurement range of a viscometer, it can be obtained from a diluted solution of the component (C) by the following method.

A toluene solution of the component (C) having a concentration of 1 g/100 mL is prepared, and the specific viscosity $\eta sp$ (25°C) is determined by the following equation (1). Next, the intrinsic viscosity $[\eta]$ is determined by substituting the relationship of Huggins shown in the following equation (2). Further, $[\eta]$ is substituted into the A. Kolorlov equation shown in the following equation (3) to determine the molecular weight M. Finally, M is substituted into the A. J. Barry equation shown in the following equation (4) to determine the viscosity $\eta$ of the component (C) (see, for example, Silicone Oil KF-96 Performance Test Result 4.2, published by Shin-Etsu Chemical Co., Ltd.).

$$\eta sp = (\eta/\eta 0) - 1 \quad (1)$$

($\eta$0: viscosity of toluene, $\eta$: viscosity of solution)

$$\eta sp = [\eta] + K'[\eta]^2 \quad (2)$$

(K': Huggins constant, and the one described in Nakamuta, Nihon Kagakukai-shi, 77588 [1956] is used.)

$$[\eta] = 0.215 \times 10^{-4} M^{0.65} \quad (3)$$

$$\log\eta = 1.00 + 0.0123M^{0.5} \quad (4)$$

Examples of a commercially available product of dimethylpolysiloxane used as the component (C) include X-25-9074 (viscosity of 30,000,000 $mm^2/s$) (manufactured by Shin-Etsu Chemical Co., Ltd.), and Silsoft B3020 (viscosity of 20,000,000 $mm^2/s$) (manufactured by Momentive Performance Materials, Inc.).

The component (C) is preferably one or more selected from the group consisting of dimethylpolysiloxane, methylphenylpolysiloxane, aminopropylmethylpolysiloxane, and dimethiconol, each having a degree of polymerization within the above range, and more preferably dimethylpolysiloxane having a degree of polymerization within the above range, from the viewpoint of the touch feeling of the hair or the fiber for a headwear product in a dry state and a wet state, the heat setting property and the slipperiness of a hair iron during styling, the property of imparting a feeling of volume during styling, and improving the sustained effect of the property of imparting a feeling of volume and the heat setting property after hair washing.

<Component (D): Organopolysiloxane Other Than Components (A), (B1), and (C), Having Polyalkylene Oxide Moiety and Cationic Group>

The processing agent (II) of the second invention contains, as the component (D), an organopolysiloxane other than the components (A), (B 1), and (C), which has a polyalkylene oxide moiety and a cationic group, from the viewpoint of the slipperiness of a hair iron during styling, and improving the sustained effect of the heat setting property after hair washing.

Examples of the component (D) include a silicone having a polyalkylene oxide moiety and a cationic group in the main chain or a side chain of dimethylpolysiloxane (dimethicone) or methylphenylpolysiloxane.

The cationic group contained in the component (D) refers to a cationic group or a group which can be ionized to become a cationic group. Specific examples thereof include a primary amino group, a secondary amino group, a tertiary amino group, and a quaternary ammonium group. The cationic group contained in the component (D) is preferably one or more selected from the group consisting of a primary amino group, a secondary amino group, and a tertiary amino group.

More specifically, the component (D) is more preferably one or more selected from the group consisting of a silicone (D1) having a repeating unit represented by the following general formula (2) and a silicone (D2) having a repeating unit represented by the following general formula (3).

In the following description, the silicone (D1) having a repeating unit represented by the general formula (2) is also referred to as "component (D1)", and the silicone (D2) having a repeating unit represented by the general formula (3) is also referred to as "component (D2)".

[Component (D1)]

The component (D1) is a silicone having a repeating unit represented by the following general formula (2).

[Chem 8]

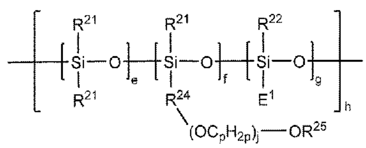

(2)

In the formula (2), $R^{21}$ represents a monovalent hydrocarbon group having 1 or more and 6 or less carbon atoms. $R^{22}$ represents any of $R^{21}$ or $E^1$. $E^1$ represents a monovalent group represented by $-R^{23}-Z^1$ (where $R^{23}$ represents a divalent hydrocarbon group having 1 or more and 6 or less carbon atoms, and $Z^1$ represents a primary to tertiary amino group-containing group). $R^{24}$ represents a divalent hydrocarbon group having 1 or more and 6 or less carbon atoms, $R^{25}$

represents a hydrogen atom or a monovalent hydrocarbon group having 1 or more and 4 or less carbon atoms.

e is a number of 1 or more and 50 or less, f is a number of 1 or more and 50 or less, g is a number of 1 or more and 50 or less, h is a number of 1 or more, j is a number of 2 or more and 100 or less. p is a number of 2 or more and 10 or less. The bonding order of the parenthesized structural units is not limited, and the bonding form thereof may be in a block form or a random form. j's $(OC_pH_{2p})$s can be the same or different. Plural $R^{21}$'s, $R^{22}$'s, $R^{24}$'s, $R^{25}$'s and $E^1$'s can be the same or different.

In the general formula (2), $R^{21}$ represents a monovalent hydrocarbon group having 1 or more and 6 or less carbon atoms, and is preferably an alkyl group having 1 or more and 6 or less carbon atoms, or a phenyl group, more preferably a methyl group or an ethyl group, and even more preferably a methyl group. $R^{22}$ is any of $R^{21}$ or $E^1$, and is preferably $R^{21}$.

In the general formula (2), $E^1$ represents a monovalent group represented by $-R^{23}-Z^1$ (where $R^{23}$ represents a divalent hydrocarbon group having 1 or more and 6 or less carbon atoms, and $Z^1$ represents a primary to tertiary amino group-containing group). $R^{23}$ is preferably a divalent hydrocarbon group having 2 or more and 4 or less carbon atoms, and more preferably an ethylene group, a trimethylene group, a propylene group or a tetramethylene group.

$Z^1$ is a primary to tertiary amino group-containing group, and is preferably a group represented by $-N(R^{26})_2$, $-NR^{26}(CH_2)_qN(R^{26})_2$, or $-NR^{26}(CH_2)_qN(R^{27})CO-R^{28}$. Here, $R^{26}$ represents a hydrogen atom or a monovalent hydrocarbon group having 1 or more and 4 or less carbon atoms, and preferably a hydrogen atom, a methyl group, or an ethyl group. $R^{27}$ represents a monovalent hydrocarbon group having 1 or more and 4 or less carbon atoms, and is preferably a methyl group or an ethyl group. $R^{28}$ represents a monovalent hydrocarbon group having 1 or more and 4 or less carbon atoms. q is a number of 2 or more and 6 or less, and is preferably a number of 2 or more and 4 or less.

In the general formula (2), the group $E^1$ is preferably $-(CH_2)_3-NH_2$, $-(CH_2)_3-N(CH_3)_2$, $-(CH_2)_3-NH-(CH_2)_2-NH_2$, or $-(CH_2)_2-NH-(CH_2)_2-N(CH_3)_2$, and more preferably $-(CH_2)_3-NH_2$, or $-(CH_2)_3-NH-(CH_2)_2-NH_2$.

In the general formula (2), $R^{24}$ is a divalent hydrocarbon group having 1 or more and 6 or less carbon atoms, preferably a divalent hydrocarbon group having 2 or more and 4 or less carbon atoms, and more preferably an ethylene group, a trimethylene group, a propylene group, or a tetramethylene group.

$R^{25}$ is a hydrogen atom or a monovalent hydrocarbon group having 1 or more and 4 or less carbon atoms, and is preferably a methyl group or an ethyl group.

In the general formula (2), e is a number of 1 or more and 50 or less, f is a number of 1 or more and 50 or less, g is a number of 1 or more and 50 or less, h is a number of 1 or more, and j is a number of 2 or more and 100 or less. p is a number of 2 or more and 10 or less, and is preferably 2 or more and 6 or less, and more preferably 2 or more and 4 or less.

In particular, the component (D1) is more preferably one represented by the following general formula (2-1).

[Chem 9]

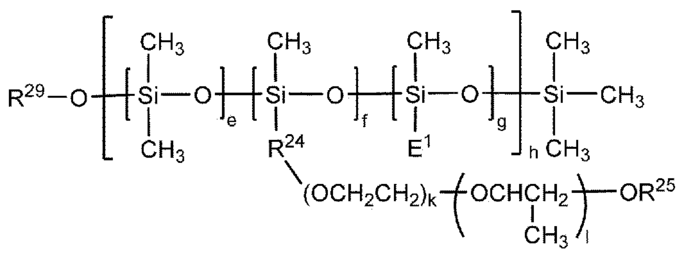
(2-1)

In the formula (2-1), $E^1$, $R^{24}$, $R^{25}$, e, f, g, and h are the same as above. $R^{29}$ represents a monovalent hydrocarbon group having 1 or more and 4 or less carbon atoms, or a trimethylsilyl group. k is a number of 1 or more and 50 or less, l is a number of 0 or more and 50 or less, and is preferably a number of 1 or more and 50 or less.

$R^{29}$ is preferably a methyl group, an ethyl group, or a trimethylsilyl group, and more preferably a trimethylsilyl group.

As the component (D1), one can be used, or two or more can be used in combination.

As the component (D1), commercially-available aminopolyether-modified silicones can be used. Examples thereof include ABIL Soft AF100 (aminopolyether-modified silicone represented by the general formula (2-1) (methoxy PEG/PPG-7/3 aminopropyl dimethicone)) (manufactured by Evonik Corporation).

[Component (D2)]

The component (D2) is a silicone having a repeating unit represented by the following general formula (3).

[Chem 10]

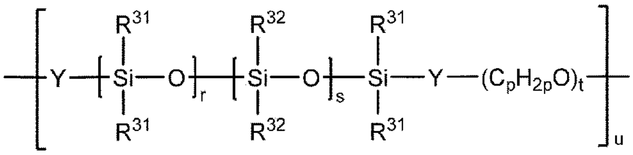 (3)

In the formula (3), $R^{31}$ represents a monovalent hydrocarbon group having 1 or more and 6 or less carbon atoms. $R^{32}$ represents any of $R^{31}$ or $E^2$. $E^2$ represents a monovalent group represented by $-R^{33}-Z^2$ (where $R^{33}$ represents a single bond, or a divalent hydrocarbon group having 1 or more and 20 or less carbon atoms, and $Z^2$ represents a primary to tertiary amino group-containing group). Y represents a single bond, or a divalent group having 1 or more and 12 or less carbon atoms. In the case where all $R^{32}$'s are $R^{31}$'s, at least one Y is an amino group-containing divalent group. In the case where all Y's are divalent groups not containing an amino group, at least one $R^{32}$ is $E^2$.

r is a number of 1 or more, s is a number of 1 or more, t is a number of 2 or more and 100 or less, and u is a number of 1 or more. p is the same as above, and is a number of 2 or more and 10 or less. The bonding order of the parenthesized structural units is not limited, and the bonding form thereof may be in a block form or a random form. t's $(C_pH_{2p}O)$s can be the same or different. Plural $R^{31}$'s, $R^{32}$'s, $E^2$'s and Y's can be the same or different.

In the general formula (3), $R^{31}$ represents a monovalent hydrocarbon group having 1 or more and 6 or less carbon atoms, and is independently preferably an alkyl group having 1 or more and 6 or less carbon atoms, or a phenyl group, more preferably a methyl group or an ethyl group, and still more preferably a methyl group.

In the general formula (3), $R^{32}$ is any of $R^{31}$ or $E^2$. $E^2$ represents a monovalent group represented by $-R^{33}-Z^2$ (where $R^{33}$ represents a single bond, or a divalent hydrocarbon group having 1 or more and 20 or less carbon atoms).

$R^{33}$ is preferably a divalent hydrocarbon group having 1 or more and 20 or less carbon atoms, more preferably an alkylene group having 1 or more and 20 or less carbon atoms, still more preferably a linear or branched alkylene group having 1 or more and 6 or less carbon atoms, even more preferably a methylene group, an ethylene group, a trimethylene group, a propylene group, a tetramethylene group, or a hexamethylene group, and even more preferably a trimethylene group or a propylene group.

$Z^2$ is a primary to tertiary amino group-containing group, and is preferably an amino group-containing group represented by $-N(R^{34})_2$, $-NR^{34}(CH_2)_qN(R^{34})_2$, or $-NR^{34}(CH_2)_qN(R^{35})CO-R^{36}$. Here, $R^{34}$ and $R^{35}$ each independently represent a hydrogen atom or an alkyl group having 1 or more and 3 or less carbon atoms, and is preferably a hydrogen atom or a methyl group. $R^{36}$ represents an alkyl group having 1 or more and 3 or less carbon atoms. q is a number of 1 or more and 6 or less, and is preferably a number of 2 or more and 4 or less.

In the general formula (3), the group $E^2$ is preferably $-(CH_2)_3-NH_2$, $-(CH_2)_3-N(CH_3)_2$, $-(CH_2)_3-NH-(CH_2)_2-NH_2$, or $-(CH_2)_2-NH-(CH_2)_2-N(CH_3)_2$, and more preferably $-(CH_2)_3-NH_2$, or $-(CH_2)_3-NH-(CH_2)_2-NH_2$.

In the general formula (3), Y is a single bond, or a divalent group having 1 or more and 12 or less carbon atoms. The divalent group having 1 or more and 12 or less carbon atoms is preferably an alkylene group having 1 or more and 6 or less carbon atoms, an alkyleneoxy group having 1 or more and 6 or less carbon atoms, or a divalent group represented by $-R^{37}-O-CH_2-CH(OH)-CH_2-N(R^{38})-R^{39}-O-$ of an amino group-containing divalent group. Here, $R^{37}$ and $R^{39}$ each independently represent an alkylene group having 1 or more and 6 or less carbon atoms, preferably an alkylene group having 2 or more and 4 or less carbon atoms, and more preferably a propylene group. $R^{38}$ represents a hydrogen atom or an alkyl group having 1 or more and 3 or less carbon atoms.

In the general formula (3), the alkylene group having 1 or more and 6 or less carbon atoms and the alkylene group in the alkyleneoxy group having 1 or more and 6 or less carbon atoms for Y are preferably an ethylene group, a propylene group, a trimethylene group, a n-butylene group (tetramethylene group) or an isobutylene group, and more preferably a n-butylene group or an isobutylene group. The isobutylene group as referred to herein includes $-CH(CH_3)CH_2CH_2-$, $-CH_2CH(CH_3)CH_2-$, and $-CH_2CH_2CH(CH_3)-$.

In the general formula (3), r is a number of 1 or more, s is a number of 1 or more, t is a number of 2 or more and 100 or less, and u is a number of 1 or more. r is preferably a number of 1 or more and 1000 or less, and more preferably a number of 2 or more and 200 or less. s is preferably a number of 1 or more and 100 or less, t is preferably a number of 4 or more and 80 or less, and more preferably 10 or more and 50 or less, u is preferably a number of 1 or more and 300 or less, and more preferably a number of 1 or more and 150 or less.

In the general formula (3), p is a number of 2 or more and 10 or less, preferably 2 or more and 6 or less, and more preferably 2 or more and 4 or less.

The component (D2) is more preferably one or more selected from the group consisting of a silicone having a structure represented by the following general formula (3-1) and a silicone having a structure represented by the following general formula (3-2).

[Chem 11]

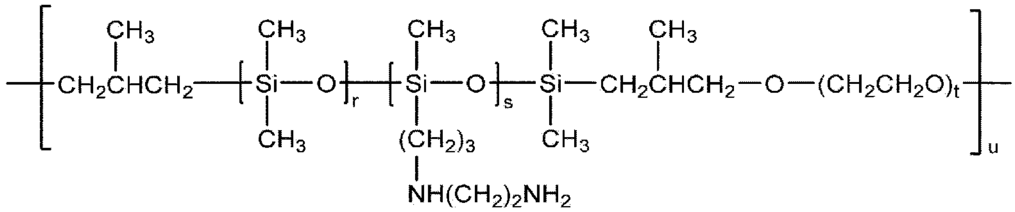

(3-1)

In the formula (3-1), r, s, t, and u are the same as above.

[Chem 12]

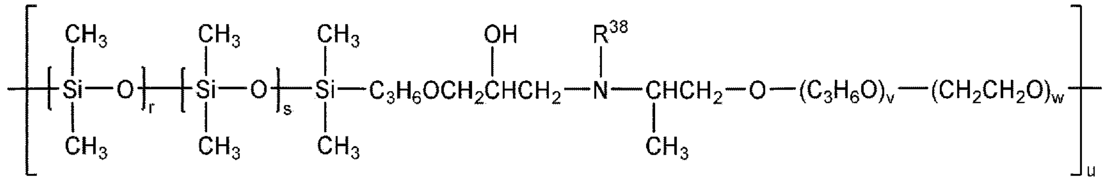

(3-2)

In the formula (3-2), $R^{38}$, r, s and u are the same as above. v is a number of 0 or more and 50 or less, and preferably a number of 2 or more and 50 or less. w is a number of 2 or more and 100 or less. v + w is a number of 2 or more and 100 or less, preferably a number of 4 or more and 80 or less, and more preferably a number of 10 or more and 50 or less.

As the component (D2), one can be used, or two or more can be used in combination.

As the component (D2), commercially-available aminopolyether-modified silicones can be used. Examples thereof include, as the aminopolyether-modified silicone having a structure represented by the general formula (3-1), DOWSIL SS-3588 Fluid (80% by mass ethanol solution of (bisisobutyl PEG-15/amodimethicone) copolymer), DOWSIL SILSTYLE 104 ((bisisobutyl PEG-14/amodimethicone) copolymer), DOWSIL SILSTYLE 201 ((bisisobutyl PEG-14/amodimethicone) copolymer), and DOWSIL SILSTYLE 401((bisisobutyl PEG/PPG-20/35/amodimethicone) copolymer) (all manufactured by Dow Toray Co., Ltd.). In addition, examples of the aminopolyether-modified silicone having a structure represented by the general formula (3-2) include Silsoft A+ (PEG-40/PPG-8 methylaminopropyl/hydroxypropyldimethicone copolymer) (manufactured by Momentive Performance Materials, Inc.).

As the component (D), one, or two or more of the above can be used. Among them, from the viewpoint of adsorption to a keratin substance or a fiber surface for a headwear product, the component (D) is preferably a silicone (D2) having a repeating unit represented by the general formula (3), more preferably one or more selected from the group consisting of a silicone having a structure represented by the general formula (3-1) and a silicone having a structure represented by the general formula (3-2), and still more preferably a silicone having a structure represented by the general formula (3-1).

<Content>

The content of the component (A) in the processing agent (I) and the processing agent (II) is preferably 0.5% by mass or more, more preferably 1.0% by mass or more, still more preferably 1.5% by mass or more, and even more preferably 2.0% by mass or more, from the viewpoint of the film formability, the property of imparting a feeling of volume and the heat setting property during styling, and improving the sustained effect of the property of imparting a feeling of volume and the heat setting property after hair washing, and is preferably 30% by mass or less, more preferably 20% by mass or less, still more preferably 10% by mass or less, and even more preferably 5.0% by mass or less, from the viewpoint of improving the touch feeling of the hair or the fiber for a headwear product in a dry state and a wet state. Thus, the content of the component (A) in the hair processing agent or the fiber processing agent for a headwear product is preferably 0.5% by mass or more and 30% by mass or less, more preferably 1.0% by mass or more and 20% by mass or less, still more preferably 1.5% by mass or more and 10% by mass or less, and even more preferably 2.0% by mass or more and 5.0% by mass or less.

The content of the component (B) in the processing agent (I) is preferably 0.1% by mass or more, more preferably 0.5% by mass or more, still more preferably 1.0% by mass or more, and even more preferably 1.5% by mass or more, and is preferably 30% by mass or less, more preferably 25% by mass or less, still more preferably 20% by mass or less, and even more preferably 15% by mass or less, from the viewpoint of the film formability, the touch feeling of the hair or the fiber for a headwear product in a dry state and a wet state, the property of imparting a feeling of volume and the heat setting property during styling, and improving the sustained effect of the property of imparting a feeling of volume and the heat setting

property after hair washing. Thus, the content of the component (B) in the processing agent (I) is preferably 0.1% by mass or more and 30% by mass or less, more preferably 0.5% by mass or more and 25% by mass or less, still more preferably 1.0% by mass or more and 20% by mass or less, and even more preferably 1.5% by mass or more and 15% by mass or less.

The total content of the component (A) and the component (B) in the processing agent (I) is preferably 1% by mass or more, more preferably 2% by mass or more, still more preferably 3% by mass or more, and even more preferably 4% by mass or more, from the viewpoint of the film formability, the property of imparting a feeling of volume and the heat setting property during styling, and improving the sustained effect of the property of imparting a feeling of volume and the heat setting property after hair washing. On the other hand, the total content is preferably 50% by mass or less, more preferably 40% by mass or less, still more preferably 30% by mass or less, even more preferably 20% by mass or less, and even more preferably 15% by mass or less, from the viewpoint of improving the touch feeling of the hair or the fiber for a headwear product in a dry state and a wet state, and improving the sustained effect of the property of imparting a feeling of volume and the heat setting property after hair washing. Thus, the total content of the component (A) and the component (B) in the processing agent (I) is preferably 1% by mass or more and 50% by mass or less, more preferably 2% by mass or more and 40% by mass or less, still more preferably 2% by mass or more and 30% by mass or less, even more preferably 2% by mass or more and 20% by mass or less, even more preferably 3% by mass or more and 15% by mass or less, and even more preferably 4% by mass or more and 15% by mass or less.

In addition, the mass ratio of the content of the component (A) to the total content of the component (A) and the component (B) in the processing agent (I), i.e., [(A)/{(A) + (B)}], is preferably 0.05 or more, more preferably 0.10 or more, still more preferably 0.15 or more, and even more preferably 0.20 or more, from the viewpoint of the film formability, the touch feeling of the hair or the fiber for a headwear product in a dry state and a wet state, the property of imparting a feeling of volume and the heat setting property during styling, and improving the sustained effect of the property of imparting a feeling of volume and the heat setting property after hair washing. On the other hand, it is preferably 0.98 or less, more preferably 0.90 or less, still more preferably 0.80 or less, and even more preferably 0.60 or less, from the viewpoint of the touch feeling of the hair or the fiber for a headwear product in a dry state and a wet state, and improving the sustained effect of the property of imparting a feeling of volume and the heat setting property after hair washing. Thus, the mass ratio of the content of the component (A) to the total content of the component (A) and the component (B) in the processing agent (I), i.e., [(A)/{(A) + (B)}], is preferably 0.05 or more and 0.98 or less, more preferably 0.10 or more and 0.90 or less, still more preferably 0.15 or more and 0.80 or less, and even more preferably 0.20 or more and 0.60 or less.

The content of the component (C) in the processing agent (I) and the processing agent (II) is preferably 0.01% by mass or more, more preferably 0.02% by mass or more, still more preferably 0.05% by mass or more, and even more preferably 0.10% by mass or more, from the viewpoint of the touch feeling of the hair or the fiber for a headwear product in a dry state and a wet state, the heat setting property and the slipperiness of a hair iron during styling, the property of imparting a feeling of volume during styling, and improving the sustained effect of the property of imparting a feeling of volume and the heat setting property after hair washing, and is preferably 20% by mass or less, more preferably 10% by mass or less, still more preferably 5.0% by mass or less, and even more preferably 3.0% by mass or less, from the viewpoint of suppressing stickiness and maintaining the touch feeling of the hair or the fiber for a headwear product in a dry state and a wet state. Thus, the content of the component (C) in the processing agent (I) and the processing agent (II) is preferably 0.01% by mass or more and 20% by mass or less, more preferably 0.02% by mass or more and 10% by mass or less, still more preferably 0.05% by mass or more and 5.0% by mass or less, and even more preferably 0.10% by mass or more and 3.0% by mass or less.

In addition, the mass ratio of the content of the component (C) to the total content of the component (A) and the component (B) in the processing agent (I), i.e., [(C)/{(A) + (B)}], is preferably 0.01 or more, more preferably 0.05 or more, still more preferably 0.1 or more, and even more preferably 0.2 or more, from the viewpoint of improving the touch feeling of the hair or the fiber for a headwear product in a dry state and a wet state. On the other hand, it is preferably 3 or less, more preferably 2 or less, still more preferably 0.5 or less, and even more preferably 0.3 or less, from the viewpoint of improving the sustained effect of the property of imparting a feeling of volume and the heat setting property after hair washing. Thus, the mass ratio of the content of the component (C) to the total content of the component (A) and the component (B) in the processing agent (I), i.e., [(C)/{(A) + (B)}], is preferably 0.01 or more and 3 or less, more preferably 0.05 or more and 2 or less, still more preferably 0.1 or more and 0.5 or less, and even more preferably 0.2 or more and 0.3 or less.

The content of the component (B1) in the processing agent (II) is preferably 0.1% by mass or more, more preferably 0.5% by mass or more, still more preferably 1.0% by mass or more, and even more preferably 1.5% by mass or more, and is preferably 30% by mass or less, more preferably 25% by mass or less, still more preferably 20% by mass or less, even more preferably 15% by mass or less, even more preferably 10% by mass or less, and even more preferably 5.0% by mass or less, from the viewpoint of the film formability, the touch feeling of the hair or the fiber for a headwear product in a dry state and a wet state, the heat setting property during styling, and improving the sustained effect of the heat setting property after hair washing. Thus, the content of the component (B1) in the processing agent (II) is preferably 0.1% by mass or more and 30% by mass or less, more preferably 0.5% by mass or more and 25% by mass or less, still more preferably 1.0% by mass or more and 20% by mass or less, even more preferably 1.5% by mass or more and 15% by mass

or less, even more preferably 1.5% by mass or more and 10% by mass or less, and even more preferably 1.5% by mass or more and 5.0% by mass or less.

The total content of the component (A) and the component (B1) in the processing agent (II) is preferably 0.6% by mass or more, more preferably 2.0% by mass or more, still more preferably 3.0% by mass or more, and even more preferably 3.5% by mass or more, from the viewpoint of the film formability, the touch feeling of the hair or the fiber for a headwear product in a dry state and a wet state, the heat setting property during styling, and improving the sustained effect of the heat setting property after hair washing. On the other hand, the total content is preferably 50% by mass or less, more preferably 40% by mass or less, still more preferably 30% by mass or less, even more preferably 20% by mass or less, even more preferably 15% by mass or less, and even more preferably 10% by mass or less, from the viewpoint of the touch feeling of the hair or the fiber for a headwear product in a dry state and a wet state, and improving the slipperiness of a hair iron. The total content of the component (A) and the component (B1) in the processing agent (II) is preferably 0.6% by mass or more and 50% by mass or less, more preferably 2.0% by mass or more and 40% by mass or less, still more preferably 2.0% by mass or more and 30% by mass or less, even more preferably 2.0% by mass or more and 20% by mass or less, even more preferably 3.0% by mass or more and 15% by mass or less, and even more preferably 3.5% by mass or more and 10% by mass or less.

The mass ratio of the content of the component (A) to the total content of the component (A) and the component (B1) in the processing agent (II), i.e., [(A)/{(A) + (B1)}], is preferably 0.05 or more, more preferably 0.10 or more, still more preferably 0.15 or more, even more preferably 0.20 or more, even more preferably 0.30 or more, and even more preferably 0.40 or more, and is preferably 0.98 or less, more preferably 0.90 or less, still more preferably 0.80 or less, even more preferably 0.70 or less, and even more preferably 0.60 or less, from the viewpoint of the film formability, the touch feeling of the hair or the fiber for a headwear product in a dry state and a wet state, the slipperiness of a hair iron, and improving the sustained effect of the heat setting property after hair washing. Thus, the mass ratio of the content of the component (A) to the total content of the component (A) and the component (B1) in the processing agent (II), i.e., [(A)/{(A) + (B1)}], is preferably 0.05 or more and 0.98 or less, more preferably 0.10 or more and 0.90 or less, still more preferably 0.15 or more and 0.80 or less, even more preferably 0.20 or more and 0.70 or less, even more preferably 0.30 or more and 0.60 or less, and even more preferably 0.40 or more and 0.60 or less.

The mass ratio of the content of the component (C) to the total content of the component (A) and the component (B1) in the processing agent (II), i.e., [(C)/{(A) + (B1)}], is preferably 0.01 or more, more preferably 0.05 or more, still more preferably 0.1 or more, and even more preferably 0.2 or more, from the viewpoint of improving the touch feeling of the hair or the fiber for a headwear product in a dry state and a wet state and the slipperiness of a hair iron. On the other hand, it is preferably 3 or less, more preferably 2 or less, still more preferably 1 or less, even more preferably 0.5 or less, and even more preferably 0.3 or less, from the viewpoint of improving the sustained effect of the heat setting property after hair washing. Thus, the mass ratio of the content of the component (C) to the total content of the component (A) and the component (B1) in the processing agent (II), i.e., [(C)/{(A) + (B1)}], is preferably 0.01 or more and 3 or less, more preferably 0.05 or more and 2 or less, still more preferably 0.1 or more and 1 or less, even more preferably 0.2 or more and 0.5 or less, and even more preferably 0.2 or more and 0.3 or less.

The content of the component (D) in the processing agent (II) is preferably 0.01% by mass or more, more preferably 0.02% by mass or more, still more preferably 0.05% by mass or more, even more preferably 0.1% by mass or more, even more preferably 0.2% by mass or more, and even more preferably 0.3% by mass or more, from the viewpoint of the slipperiness of a hair iron during styling and improving the sustained effect of the heat setting property after hair washing, and is preferably 20% by mass or less, more preferably 10% by mass or less, still more preferably 5.0% by mass or less, even more preferably 3.0% by mass or less, even more preferably 2.0% by mass or less, and even more preferably 1.0% by mass or less, from the viewpoint of suppressing stickiness and maintaining the touch feeling of the hair or the fiber for a headwear product in a dry state and a wet state. Thus, the content of the component (D) in the processing agent (II) is preferably 0.01% by mass or more and 20% by mass or less, more preferably 0.02% by mass or more and 10% by mass or less, still more preferably 0.05% by mass or more and 5.0% by mass or less, even more preferably 0.1% by mass or more and 3.0% by mass or less, even more preferably 0.2% by mass or more and 2.0% by mass or less, and even more preferably 0.3% by mass or more and 1.0% by mass or less.

The total content of the components (A), (B1), (C), and (D) in the processing agent (II) is preferably 1.5% by mass or more, more preferably 2.0% by mass or more, still more preferably 3.0% by mass or more, even more preferably 4.0% by mass or more, and even more preferably 5.0% by mass or more, and is preferably 50% by mass or less, more preferably 40% by mass or less, still more preferably 30% by mass or less, even more preferably 20% by mass or less, and even more preferably 15% by mass or less, from the viewpoint of the film formability, the touch feeling of the hair or the fiber for a headwear product in a dry state and a wet state, the heat setting property and the slipperiness of a hair iron during styling, and improving the sustained effect of the heat setting property after hair washing. The total content of the components (A), (B1), (C), and (D) in the processing agent (II) is preferably 1.5% by mass or more and 50% by mass or less, more preferably 2.0% by mass or more and 40% by mass or less, still more preferably 2.0% by mass or more and 30% by mass or less, even more preferably 3.0% by mass or more and 20% by mass or less, even more preferably 4.0% by mass or more and 20% by

mass or less, and even more preferably 5.0% by mass or more and 15% by mass or less.

The mass ratio of the content of the component (D) to the total content of the components (A), (B1), and (C) in the processing agent (II), i.e., [(D)/{(A) + (B1) + (C)}], is preferably 0.001 or more, more preferably 0.005 or more, still more preferably 0.01 or more, even more preferably 0.02 or more, and even more preferably 0.05 or more, from the viewpoint of the touch feeling of the hair or the fiber for a headwear product in a dry state and a wet state, the slipperiness of a hair iron, and improving the sustained effect of the heat setting property after hair washing. On the other hand, it is preferably 3 or less, more preferably 2 or less, still more preferably 1 or less, even more preferably 0.5 or less, even more preferably 0.3 or less, and even more preferably 0.1 or less, from the viewpoint of the touch feeling of the hair or the fiber for a headwear product in a dry state and a wet state, and improving the sustained effect of the heat setting property after hair washing. Thus, the mass ratio of the content of the component (D) to the total content of the components (A), (B1), and (C) in the processing agent (II), i.e., [(D)/{(A) + (B1) + (C)}], is preferably 0.001 or more and 3 or less, more preferably 0.005 or more and 2 or less, still more preferably 0.01 or more and 1 or less, even more preferably 0.02 or more and 0.5 or less, even more preferably 0.02 or more and 0.3 or less, and even more preferably 0.05 or more and 0.1 or less.

<Component (E): Volatile Solvent>

The hair processing agent or the fiber processing agent for a headwear product of the present invention preferably further contains a volatile solvent as a component (E) from the viewpoint of dissolving or dispersing the components (A) to (D), from the viewpoint of improving the drying speed when the hair processing agent or the fiber processing agent for a headwear product is applied to hair or a fiber for a headwear product to efficiently form a film having a phase separation structure in which the component (C) is unevenly distributed on the surface side (air side), from the viewpoint of suppressing stickiness and improving the touch feeling of the hair or the fiber for a headwear product in a dry state and a wet state, and from the viewpoint of adjusting the viscosity to be easily applied to hair or a fiber for a headwear product. The volatile solvent defined in the present invention does not include water.

Examples of the volatile solvent include alcohol-based solvents, ether-based solvents, ketone-based solvents, ester-based solvents, hydrocarbon-based solvents, and silicone-based solvents, and the solvent can be appropriately selected according to the formulation.

Examples of the alcohol-based solvent used as the component (E) include ethanol, 1-propanol, 2-propanol, 1-butanol, 2-butanol, and benzyl alcohol.

Examples of the ether-based solvent include diethyl ether and tetrahydrofuran.

Examples of the ketone-based solvent include acetone and methyl ethyl ketone.

Examples of the ester-based solvent include methyl acetate, ethyl acetate, butyl acetate, and isobutyl acetate.

Examples of the hydrocarbon-based solvent include pentane, isopentane, hexane, isohexane, heptane, isoheptane, decane, isodecane, dodecane, isododecane, tridecane, isotridecane, tetradecane, isotetradecane, and light liquid isoparaffin (containing isoparaffin having 8 to 16 carbon atoms as a main component).

Examples of the silicone-based solvent include cyclic silicones, dimethylpolysiloxanes having a viscosity at 25°C of 10 $mm^2/s$ or less, alkyl trimethicones such as methyl trimethicone, and methylphenylpolysiloxanes having a viscosity at 25°C of 20 $mm^2/s$ or less. As the component (E), one, or two or more of the above can be used.

From the viewpoint of the dissolution or dispersibility of the components (A) to (D), from the viewpoint of improving the drying speed, and from the viewpoint of improving the touch feeling of the hair or the fiber for a headwear product in a dry state and a wet state, the component (E) preferably includes one or more selected from the group consisting of an alcohol-based solvent, a hydrocarbon-based solvent, and a silicone-based solvent, preferably includes one or more selected from the group consisting of ethanol, pentane, isopentane, hexane, isohexane, heptane, isoheptane, decane, isodecane, dodecane, isododecane, tridecane, isotridecane, tetradecane, isotetradecane, and light liquid isoparaffin, dimethylpolysiloxane having a viscosity at 25°C of 10 $mm^2/s$ or less, methyltrimethicone, and methylphenylpolysiloxane having a viscosity at 25°C of 20 $mm^2/s$ or less, more preferably includes one or more selected from the group consisting of dimethylpolysiloxane having a viscosity at 25°C of 5 $mm^2/s$ or less, methyltrimethicone, isodecane, isododecane, isotetradecane, and light liquid isoparaffin, still more preferably includes one or more selected from the group consisting of ethanol, isodecane, isododecane, isotetradecane, and light liquid isoparaffin, and even more preferably includes isododecane. With regard to the processing agent (II), it is even more preferable to include ethanol and isododecane.

In a case where the processing agent (I) contains the component (E), the content of the component (E) in the processing agent (I) is preferably 40% by mass or more, more preferably 50% by mass or more, and still more preferably 60% by mass or more from the viewpoint of the dissolution or dispersibility of the components (A) to (C), from the viewpoint of improving the drying speed, and from the viewpoint of improving the touch feeling of the hair or the fiber for a headwear product in a dry state and a wet state, and is preferably 98% by mass or less and more preferably 97% by mass or less from the viewpoint of adjusting the viscosity of the hair processing agent or the fiber processing agent for a headwear product to a viscosity that is easy to apply to hair or a fiber for a headwear product. Thus, the content of the component (E) in the processing agent (I) is preferably 40% by mass or more and 98% by mass or less, more preferably 50% by mass or more

and 97% by mass or less, and still more preferably 60% by mass or more and 97% by mass or less.

In a case where the processing agent (II) contains the component (E), the content of the component (E) in the processing agent (II) is preferably 40% by mass or more, more preferably 50% by mass or more, still more preferably 60% by mass or more, and even more preferably 75% by mass or more from the viewpoint of the dissolution or dispersibility of the components (A), (B1), (C), and (D), from the viewpoint of improving the drying speed, and from the viewpoint of improving the touch feeling of the hair or the fiber for a headwear product in a dry state and a wet state, and is preferably 98% by mass or less, more preferably 95% by mass or less, and still more preferably 90% by mass or less from the viewpoint of adjusting the viscosity of the hair processing agent or the fiber processing agent for a headwear product to a viscosity that is easy to apply to hair or a fiber for a headwear product. Thus, the content of the component (E) in the processing agent (II) is preferably 40% by mass or more and 98% by mass or less, more preferably 50% by mass or more and 95% by mass or less, still more preferably 60% by mass or more and 95% by mass or less, and even more preferably 75% by mass or more and 90% by mass or less.

<Component (F): Cationic Compound, Component (F1): Cationic Surfactant>

The processing agent (I) may further contain a cationic surfactant as a component (F1) from the viewpoint of imparting a desired performance to the processing agent, in particular, from the viewpoint of further improving the touch feeling of the hair or the fiber for a headwear product in a wet state.

The cationic surfactant (F1) can be appropriately selected according to the aspect of the hair processing agent or the fiber processing agent for a headwear product, and desired performance. In particular, in a case where the processing agent (I) is a rinse, a conditioning agent, a treatment agent, or a styling agent, from the viewpoint of further improving the touch feeling of the hair or the fiber for a headwear product in a wet state, it is preferable that the processing agent (I) contains the component (F1).

In addition, from the viewpoint of imparting a desired performance to the processing agent, in particular, from the viewpoint of further improving the touch feeling of the hair or the fiber for a headwear product in a wet state, the processing agent (II) may further contain a cationic compound other than the components (A), (B1), (C), and (D) as a component (F). In particular, in a case where the processing agent (II) is a rinse, a conditioning agent, a treatment agent, or a styling agent, from the viewpoint of further improving the touch feeling of the hair or the fiber for a headwear product in a wet state, it is preferable that the processing agent (II) contains the component (F).

The term "cationic compound" as used herein means a compound having a cationic group and being positively charged as a whole. The definition of the cationic group is as described in the above-mentioned component (D).

The cationic compound other than the components (A), (B1), (C), and (D) as the component (F) is preferably a non-silicone-based cationic compound from the viewpoint of further improving the touch feeling of the hair or the fiber for a headwear product in a wet state. Specific examples thereof include one or more selected from the group consisting of (F1) a cationic surfactant and (F2) a cationic polymer containing a quaternary ammonium cation.

((F1) Cationic Surfactant)

Examples of the cationic surfactant used as the component (F1) include alkyltrimethylammonium salts such as cetyltrimethyl ammonium chloride [cetrimonium chloride], stearyltrimethyl ammonium chloride [steartrimonium chloride], and behenyltrimethyl ammonium chloride [behentrimonium chloride]; alkoxyalkyltrimethylammonium salts such as stearoxypropyltrimethyl ammonium chloride [stearoxypropyltrimonium chloride], stearoxyethyltrimethyl ammonium chloride, and stearoxyhydroxypropyltrimethyl ammonium chloride; dialkyldimethylammonium salts such as distearyldimethyl ammonium chloride; alkylamidoalkyltrimethylammonium salts such as palmitamidopropyltrimethyl ammonium chloride (palmitamidopropyltrimonium chloride); alkyldimethylamines such as N,N-dimethylbehenylamine and N,N-dimethylstearylamine and salts thereof; alkoxyalkyldimethylamines such as N,N-dimethyl-3-hexadecyloxypropylamine and N,N-dimethyl-3-octadecyloxypropylamine and salts thereof; and alkylamidoalkyldimethylamines such as N-[3-(dimethylamino)propyl] docosanamide and N-[3-(dimethylamino)propyl] stearamide and salts thereof. Among these, one can be used alone, or two or more can be used in combination.

Among the above, from the viewpoint of further improving the touch feeling of the hair or the fiber for a headwear product in a wet state, the cationic surfactant (F1) is preferably one or more selected from the group consisting of an alkyltrimethylammonium salt, an alkoxyalkyltrimethylammonium salt, an alkylamidoalkyltrimethylammonium salt, an alkyldimethylamine and a salt thereof, an alkoxyalkyldimethylamine and a salt thereof, and an alkylamidoalkyldimethylamine and a salt thereof, more preferably one or more selected from the group consisting of an alkyltrimethylammonium salt, an alkoxyalkyltrimethylammonium salt, and an alkoxyalkyldimethylamine and a salt thereof, still more preferably one or more selected from the group consisting of an alkyltrimethylammonium salt and an alkoxyalkyltrimethylammonium salt, and even more preferably one or more selected from the group consisting of stearyltrimethylammonium chloride [steartrimonium chloride], behenyltrimethylammonium chloride [behentrimonium chloride], and stearoxypropyltrimethylammonium chloride [stearoxypropyltrimonium chloride].

((F2) Cationic Polymer Containing Quaternary Ammonium Cation)

Examples of the cationic polymer containing a quaternary ammonium cation, which is used as the component (F2), include a methacryloyl ethyl trimethyl ammonium salt polymer, a quaternized dialkylaminoalkyl (meth)acrylate polymer, a diallyl quaternized ammonium salt polymer, a methacrylamidopropyl trimethyl ammonium salt polymer, and a vinyl imidazolium trichloride/vinyl pyrrolidone copolymer (polyquaternium-16). Among these, one can be used alone, or two or more can be used in combination. As used herein, the term "(meth)acrylic acid" means acrylic acid or methacrylic acid, and the term "(meth)acrylate" means acrylate or methacrylate.

Examples of the methacryloyl ethyl trimethyl ammonium salt polymer include a methacryloyl ethyl trimethyl ammonium chloride polymer (polyquaternium-37), a methacryloyl ethyl dimethyl betaine/methacryloyl ethyl trimethyl ammonium chloride/polyethylene glycol methoxy methacrylate copolymer (polyquaternium-49), and a methacryloyl ethyl dimethyl betaine/methacryloyl ethyl trimethyl ammonium chloride/2-hydroxyethyl methacrylate copolymer (polyquaternium-48).

Examples of the quaternized dialkylaminoalkyl (meth)acrylate polymer include a vinyl pyrrolidone/N,N-dimethylaminoethyl methacrylate diethyl sulfate copolymer (polyquaternium-11), and an N,N-dimethylaminoethyl methacrylate diethyl sulfate/N,N-dimethylacrylamide/polyethylene glycol dimethacrylate copolymer (polyquaternium-52).

Examples of the diallyl quaternized ammonium salt polymer include a dimethyl diallyl ammonium chloride polymer (polyquaternium-6), a dimethyl diallyl ammonium chloride/acrylic acid copolymer (polyquaternium-22), a dimethyl diallyl ammonium chloride/acrylamide copolymer (polyquaternium-7), and an acrylamide/acrylic acid/dimethyl diallyl ammonium chloride copolymer (polyquaternium-39).

Examples of the methacrylamidopropyl trimethyl ammonium salt polymer include a methacrylamidopropyl trimethyl ammonium chloride polymer, a vinylpyrrolidone/methacrylamidopropyl trimethyl ammonium chloride copolymer, an acrylic acid/methyl acrylate/methacrylamidopropyl trimethyl ammonium chloride copolymer (polyquaternium-47), and an acrylic acid/acrylamide/methacrylamidopropyl trimethyl ammonium chloride copolymer (polyquaternium-53).

Among the above, from the viewpoint of further improving the touch feeling of the hair or the fiber for a headwear product in a wet state, the cationic polymer containing a quaternary ammonium cation is preferably one or more selected from the group consisting of a quaternized dialkylaminoalkyl (meth)acrylate polymer and a diallyl quaternized ammonium salt polymer, more preferably a diallyl quaternized ammonium salt polymer, still more preferably one or more selected from the group consisting of a dimethyl diallyl ammonium chloride polymer (polyquaternium-6), a dimethyldiallylammonium chloride/acrylic acid copolymer (polyquaternium-22), a dimethyldiallylammonium chloride/acrylamide copolymer (polyquaternium-7), and an acrylamide/acrylic acid/dimethyldiallylammonium chloride copolymer (polyquaternium-39), and even more preferably a dimethyl diallyl ammonium chloride polymer (polyquaternium-6).

From the viewpoint of further improving the touch feeling of the hair or the fiber for a headwear product in a wet state, the component (F) is preferably one or more selected from the group consisting of an alkyltrimethylammonium salt, an alkoxyalkyltrimethylammonium salt, an alkylamidoalkyltrimethylammonium salt, an alkyldimethylamine and a salt thereof, an alkoxyalkyldimethylamine and a salt thereof, an alkylamidoalkyldimethylamine and a salt thereof, a quaternized dialkylaminoalkyl (meth)acrylate polymer, and a diallyl quaternized ammonium salt polymer, more preferably one or more selected from the group consisting of an alkyltrimethylammonium salt, an alkoxyalkyltrimethylammonium salt, an alkoxyalkyldimethylamine and a salt thereof, and a diallyl quaternized ammonium salt polymer, still more preferably one or more selected from the group consisting of an alkyltrimethylammonium salt, an alkoxyalkyltrimethylammonium salt, an alkoxyalkyldimethylamine and a salt thereof, and a diallyl quaternized ammonium salt polymer, still more preferably one or more selected from the group consisting of an alkyltrimethylammonium salt, an alkoxyalkyltrimethylammonium salt, a dimethyl diallyl ammonium chloride polymer (polyquaternium-6), a dimethyldiallyl ammonium chloride/acrylic acid copolymer (polyquaternium-22), a dimethyldiallyl ammonium chloride/acrylamide copolymer (polyquaternium-7), and an acrylamide/acrylic acid/dimethyldiallyl ammonium chloride copolymer (polyquaternium-39), and even more preferably one or more selected from the group consisting of stearyltrimethylammonium chloride [steartrimonium chloride], behenyltrimethylammonium chloride [behentrimonium chloride], stearoxypropyltrimethylammonium chloride [stearoxypropyltrimonium chloride], and a dimethyl diallyl ammonium chloride polymer (polyquaternium-6).

From the viewpoint of further improving the touch feeling of the hair or the fiber for a headwear product in a wet state, and from the viewpoint of the slipperiness of a hair iron during styling and improving the sustained effect of the heat setting property after hair washing, the component (F) is preferably (F 1) a cationic surfactant.

When the processing agent (I) contains the component (F1), the content of the component (F1) in the processing agent (I) is preferably 0.05% by mass or more, more preferably 0.10% by mass or more, and still more preferably 0.20% by mass or more, and is preferably 15% by mass or less, more preferably 10% by mass or less, still more preferably 5.0% by mass or less, and even more preferably 2.0% by mass or less, from the viewpoint of further improving the touch feeling of the hair or the fiber for a headwear product in a dry state and a wet state. Thus, the content of the component (F1) in the

processing agent (I) is preferably 0.05% by mass or more and 15% by mass or less, more preferably 0.10% by mass or more and 10% by mass or less, still more preferably 0.10% by mass or more and 5.0% by mass or less, and even more preferably 0.20% by mass or more and 2.0% by mass or less.

When the processing agent (II) contains the component (F), the content of the component (F) in the processing agent (II) is preferably 0.05% by mass or more, more preferably 0.1% by mass or more, and still more preferably 0.2% by mass or more, and is preferably 15% by mass or less, more preferably 10% by mass or less, still more preferably 5.0% by mass or less, even more preferably 2.0% by mass or less, even more preferably 1.0% by mass or less, and even more preferably 0.5% by mass or less, from the viewpoint of further improving the touch feeling of the hair or the fiber for a headwear product in a dry state and a wet state. Thus, the content of the component (F) in the processing agent (II) is preferably 0.05% by mass or more and 15% by mass or less, more preferably 0.10% by mass or more and 10% by mass or less, still more preferably 0.1% by mass or more and 5.0% by mass or less, even more preferably 0.1% by mass or more and 2.0% by mass or less, even more preferably 0.1% by mass or more and 1.0% by mass or less, even more preferably 0.1% by mass or more and 0.5% by mass or less, and even more preferably 0.2% by mass or more and 0.5% by mass or less.

When the processing agent (II) contains the component (F), the mass ratio of the content of the component (F) to the content of the component (C) in the processing agent (II), i.e., [(F)/(C)] is preferably 0.005 or more, more preferably 0.010 or more, still more preferably 0.020 or more, even more preferably 0.050 or more, and even more preferably 0.10 or more, from the viewpoint of further improving the touch feeling of the hair or the fiber for a headwear product in a dry state and a wet state. On the other hand, it is preferably 10 or less, more preferably 5.0 or less, still more preferably 2.0 or less, even more preferably 1.0 or less, and even more preferably 0.50 or less. Thus, the mass ratio of the content of the component (F) to the content of the component (C) in the processing agent (II), i.e., [(F)/(C)] is preferably 0.005 or more and 10 or less, more preferably 0.010 or more and 5.0 or less, still more preferably 0.020 or more and 2.0 or less, even more preferably 0.050 or more and 1.0 or less, and even more preferably 0.10 or more and 0.50 or less.

In addition to the above-described components, the processing agent of the present invention can contain components usually used in a hair processing agent or a fiber processing agent for a headwear product, for example, an antioxidant, an ultraviolet absorber, a perfume, an antiseptic, a thickener, a touch enhancer, a pH modifier, a blood circulation promoting agent, a cooling agent, an antiperspirant, a bactericide, a skin activating agent, a moisturizing agent, and a refreshing agent, other than the components (A) to (F).

In the processing agent (I), the content of water is 10% by mass or less, preferably 5% by mass or less, more preferably less than 5% by mass, and still more preferably less than 2% by mass, from the viewpoint of improving the drying speed after application to hair or fibers for a headwear product, efficiently forming a film having a phase separation structure in which the component (C) is unevenly distributed on the surface side (air side), and improving the resistance to hair washing.

In the processing agent (II), the content of water is preferably small from the viewpoint of improving the drying speed after application to hair or fibers for a headwear product, efficiently forming a film having a phase separation structure in which the component (C) is unevenly distributed on the surface side (air side), and improving the sustained effect of the heat setting property after hair washing. The content of water in the processing agent is preferably 10% by mass or less, more preferably 5% by mass or less, still more preferably less than 5% by mass, and even more preferably less than 2% by mass.

The hair processing agent or the fiber processing agent for a headwear product of the present invention preferably has a low solid oil content from the viewpoint of the touch feeling of the hair in a dry state and a wet state, the property of imparting a feeling of volume, the heat setting property and the slipperiness of a hair iron during styling, and improving the sustained effect of the property of imparting a feeling of volume and the heat setting property after hair washing. The solid oil is an oil agent that is solid at 25°C, and examples thereof include paraffin-based waxes such as solid paraffin, polyolefin-based waxes such as polyethylene wax, and beeswax. The content of the solid oil in the processing agent is preferably less than 50% by mass, more preferably less than 20% by mass, still more preferably less than 10% by mass, even more preferably less than 5% by mass, and even more preferably less than 1% by mass.

The hair processing agent or the fiber processing agent for a headwear product of the present invention preferably contains a small amount of a nonvolatile liquid oil agent other than the components (C) and (E), from the viewpoint of the touch feeling of the hair or the fiber for a headwear product in a dry state and a wet state, the property of imparting a feeling of volume, the heat setting property and the slipperiness of a hair iron during styling, and improving the sustained effect of the property of imparting a feeling of volume and the heat setting property after hair washing. The nonvolatile liquid oil agent is an oil agent that has a boiling point higher than 260°C at atmospheric pressure and is liquid at 25°C, and examples thereof include nonvolatile liquid silicones other than the components (C) and (E); triglycerides such as glyceryl trioctanoate, avocado oil, olive oil, sesame seed oil, rice bran oil, safflower oil, soybean oil, corn oil, rapeseed oil, castor oil, cotton seed oil, and mink oil; fatty acids such as oleic acid and isostearic acid; ester oils such as isopropyl myristate, butyl myristate, isopropyl palmitate, ethyl oleate, ethyl linoleate, isopropyl linoleate, cetyl caprylate, hexyl laurate, decyl myristate, decyl oleate, oleyl oleate, isostearyl laurate, isotridecyl myristate, isocetyl myristate, isostearyl

myristate, octyldodecyl myristate, octyl palmitate, isocetyl palmitate, isostearyl palmitate, isodecyl oleate, isopropyl isostearate, cetyl 2-ethylhexanoate, stearyl 2-ethylhexanoate, propylene glycol dicaprate, propylene glycol dioleate, glyceryl tri-2-ethylhexanoate, caprylic/capric triglyceride, isononyl isononanoate, diisopropyl sebacate, propylene glycol isostearate, 2-ethylhexyl paramethoxycinnamate, 2-ethoxyethyl paramethoxycinnamate, isopropyl paramethoxycinnamate/diisopropyl cinnamate ester mixture, methyl bis(trimethylsiloxy) silyl isopentyl trimethoxycinnamate, amyl paradimethylaminobenzoate, 2-ethylhexyl paradimethylaminobenzoate, ethylene glycol salicylate, 2-ethylhexyl salicylate, benzyl salicylate, homomenthyl salicylate, octocrylene, and dimethyl diethyl benzalmalonate; and branched or unsaturated higher alcohols such as 2-octyldodecanol, isostearyl alcohol, and oleyl alcohol. The content thereof in the processing agent is preferably less than 50% by mass, more preferably less than 40% by mass, still more preferably less than 30% by mass, even more preferably less than 20% by mass, and even more preferably less than 10% by mass.

The hair processing agent or the fiber processing agent for a headwear product of the present invention preferably has a low content of a volatile cyclic silicone such as decamethylcyclopentasiloxane from the viewpoint of improving the drying speed after application to hair or fibers for a headwear product. This is because the volatile cyclic silicone has a slower volatilization time than other volatile solvents and tends to have a longer drying time after application to the hair or the fiber for a headwear product. The content of the volatile cyclic silicone in the processing agent is preferably less than 5% by mass, more preferably less than 2% by mass, still more preferably less than 1% by mass, even more preferably less than 0.5% by mass, even more preferably less than 0.1% by mass, and even more preferably 0% by mass.

In the hair processing agent or the fiber processing agent for a headwear product of the present invention, it is preferable that the content of a polyhydric alcohol is small, from the viewpoint of the touch feeling of the hair or the fiber for a headwear product in a dry state and a wet state, the property of imparting a feeling of volume, the heat setting property and the slipperiness of a hair iron during styling, and improving the sustained effect of the property of imparting a feeling of volume and the heat setting property after hair washing. Examples of the polyhydric alcohol include polyhydric alcohols having a boiling point of more than 260°C at normal pressure, and propylene glycol, and glycerol can be exemplified. The content thereof in the processing agent is preferably less than 5% by mass, more preferably less than 2% by mass, still more preferably less than 1% by mass, even more preferably less than 0.5% by mass, and even more preferably less than 0.1% by mass.

In the hair processing agent or the fiber processing agent for a headwear product of the present invention, it is preferable that the content of an oil gelling agent other than the components (A) to (D) is small, from the viewpoint of the touch feeling of the hair or the fiber for a headwear product in a dry state and a wet state, the property of imparting a feeling of volume, the heat setting property and the slipperiness of a hair iron during styling, and improving the sustained effect of the property of imparting a feeling of volume and the heat setting property after hair washing. Examples of the oil gelling agent other than the components (A) to (D) include dextrin palmitate and 12-hydroxystearic acid. The content thereof in the processing agent is preferably less than 10% by mass, more preferably less than 5% by mass, still more preferably less than 2% by mass, even more preferably less than 1% by mass, and even more preferably less than 0.5% by mass.

<Formulation>

The formulation of the processing agent of the present invention is not particularly limited, and depending on the product form, the processing agent can be in the formulation of a liquid (oil or lotion), a paste, milk, cream, gel, wax, an aerosol foam, a non-aerosol foam, a mist, or a spray. Preferably, the processing agent is oil, milk, cream, gel, a mist, or a spray. More preferably, the processing agent is oil, a mist, or a spray. Further, in the processing agent of the present invention, the content of water is 10% by mass or less, and it is more preferable that the processing agent is a non-aqueous hair processing agent or a non-aqueous fiber processing agent for a headwear product. Here, the non-aqueous hair processing agent or the non-aqueous fiber processing agent for a headwear product means a processing agent having a water content of less than 1% by mass, preferably less than 0.5% by mass, and more preferably less than 0.1% by mass.

The processing agent of the present invention can be produced according to a conventional method.

The viscosity of a preferred formulation of the processing agent of the present invention is preferably 1.0 mPa·s or more, more preferably 1.5 mPa·s or more, and still more preferably 1.7 mPa·s or more, from the viewpoint of use feeling, and is preferably 1,000 mPa·s or less, more preferably 500 mPa·s or less, still more preferably 250 mPa·s or less, even more preferably 100 mPa·s or less, and even more preferably 20 mPa·s or less, from the viewpoint of coatability. Accordingly, the viscosity of the processing agent is preferably 1.0 mPa·s or more and 1,000 mPa·s or less, more preferably 1.5 mPa·s or more and 500 mPa·s or less, still more preferably 1.7 mPa·s or more and 250 mPa·s or less, even more preferably 1.7 mPa·s or more and 100 mPa·s or less, and even more preferably 1.7 mPa·s or more and 20 mPa·s or less.

The viscosity of the processing agent is a value measured at 30°C using a B-type viscometer, and can be specifically measured by the method described in Examples.

[Method for Treating Hair or Fiber for Headwear Product]

The present invention provides a method for treating hair or a fiber for a headwear product (hereinafter also referred to as "the processing method of the present invention"), which includes a step of applying the processing agent to hair or a fiber for a headwear product, and then drying the processing agent.

In a case where the processing agent of the present invention is not a homogeneous system, from the viewpoint of uniform coating properties and from the viewpoint of improving the uniformity of the structure of the film to be formed, it is preferable that the processing agent of the present invention is temporarily compatibilized or dispersed before being applied to the hair or the fiber for a headwear product and then applied to the hair or the fiber for a headwear product. As a means for temporarily compatibilization or dispersion, a thermodynamic means such as heating, a physical means such as mechanically applying shear stress, or a chemical means such as adding a compatible solvent can be arbitrarily used. From the viewpoint of user convenience, it is preferable to uniformly compatibilize or disperse the first agent by physical means such as stirring and shaking.

In the processing method of the present invention, the amount of the processing agent applied to the hair or the fiber for a headwear product is not particularly limited, and is usually in the range of 0.005 to 1 g per 1 g of the hair or the fiber for a headwear product.

The processing method of the present invention includes a step of applying the processing agent to hair or a fiber for a headwear product and then drying the processing agent. The processing agent can be applied to the hair or the fiber for a headwear product in either a dry state or a wet state, and is preferably applied to the hair or the fiber for a headwear product in a dry state, then dried, and used without washing away, from the viewpoint of the touch feeling of the hair or the fiber for a headwear product in a dry state and a wet state, the property of imparting a feeling of volume and the heat setting property during styling, and improving the sustained effect of the property of imparting a feeling of volume and the heat setting property after hair washing.

Examples of the application method of the processing agent include coating, spraying, and casting, and the application method may be appropriately selected according to the formulation of the processing agent. When the processing agent is applied to hair or a fiber for a headwear product, the processing agent may be applied by hand, or an applicator such as a brush may be used.

The hair or the fiber for a headwear product after the application of the processing agent may be air dried or dried using a device such as a hood or a hair drier. In a case where the device is used, from the viewpoint of suppressing heat damage to the hair or the fiber for a headwear product, it is preferable that drying is performed by applying a temperature of 40°C or higher and 220°C or lower. Drying is more preferably performed by a hood or a hair dryer, and the drying temperature is preferably 40°C or higher and 110°C or lower, and more preferably 50°C or higher and 90°C or lower. Here, the temperature refers to the temperature of the hair surface or the surface of the fiber for a headwear product, and can be measured by, for example, an infrared radiation thermometer.

The drying time is not particularly limited as long as the film is substantially formed on the hair surface or the surface of the fiber for a headwear product, is appropriately adjusted depending on the amount and quality of the hair or the fiber for a headwear product, and can be performed in the range of, for example, 10 seconds to 120 minutes.

After drying, brushing may be performed in order to loosen the hair or the fiber for a headwear product.

The hair or the fiber for a headwear product treated by the processing method of the present invention has a good touch feeling in a dry state. In addition, the touch feeling of the hair or the fiber for a headwear product when washed, and rinsed after the processing agent is applied to the hair or the fiber for a headwear product, and dried (the touch feeling of the hair or the fiber for a headwear product in a wet state) is also improved.

When the hair or the fiber for a headwear product treated by the processing method of the present invention is styled, a step of shaping the hair or the fiber for a headwear product while heating the hair or the fiber for a headwear product (heat setting step) can be performed from the viewpoint of improving the shaping effect of the hair or the fiber for a headwear product and the durability thereof.

Examples of the method for heating the hair or the fiber for a headwear product include methods using devices such as a hair dryer, a hair iron, and a hot curler. Among these, the method using a hair iron is preferable from the viewpoint of effectively exhibiting the effect of improving the slipperiness of the hair iron in the second invention.

The temperature of the hair surface or the surface of the fiber for a headwear product when the hair or the fiber for a headwear product is heated is preferably 40°C or higher, more preferably 50°C or higher, still more preferably 60°C or higher, even more preferably 70°C or higher, and even more preferably 75°C or higher, from the viewpoint of improving the shaping effect of the hair or the fiber for a headwear product and the durability thereof, and from the viewpoint of obtaining a natural finish. In addition, from the viewpoint of obtaining a natural finish and suppressing thermal damage to the hair or the fiber for a headwear product, the temperature of the hair surface or the surface of the fiber for a headwear product when the hair or the fiber for a headwear product is heated is preferably 220°C or lower, more preferably 200°C or lower, still more preferably 190°C or lower, and even more preferably 150°C or lower. The heating time of the hair or the fiber for a headwear product may be within a range in which the hair or the fiber for a headwear product can be shaped as desired. Here, the

surface temperature of the hair or the fiber for a headwear product can be measured by, for example, an infrared radiation thermometer.

Regarding the above-mentioned embodiments, the present invention discloses the following.

<1>
A hair processing agent or a fiber processing agent for a headwear product of the following (I) or (II):

(I) in which the hair processing agent or the fiber processing agent for a headwear product contains the following components (A) to (C):

(A) a film-forming polymer containing an M unit represented by $(R^1)_3SiO_{1/2}$ ($R^1$ represents a hydrocarbon group having 1 or more and 12 or less carbon atoms which may be substituted with fluorine or a hydroxy group, and a plurality of $R^1$'s may be the same or different from each other) and a Q unit represented by $SiO_{4/2}$;
(B) a film-forming polymer other than the component (A); and
(C) a high-molecular-weight organopolysiloxane having a degree of polymerization of 2,200 or more and 20,000 or less,

in which a content of water is 10% by mass or less;
(II) in which the hair processing agent or the fiber processing agent for a headwear product contains the following components (A), (B 1), (C), and (D):

(A) a film-forming polymer containing an M unit represented by $(R^1)_3SiO_{1/2}$ ($R^1$ represents a hydrocarbon group having 1 or more and 12 or less carbon atoms which may be substituted with fluorine or a hydroxy group, and a plurality of $R^1$'s may be the same or different from each other) and a Q unit represented by $SiO_{4/2}$;
(B1) a film-forming polymer other than the component (A), excluding a cationic polymer containing a quaternary ammonium cation;
(C) a high-molecular-weight organopolysiloxane having a degree of polymerization of 2,200 or more and 20,000 or less; and
(D) an organopolysiloxane other than the components (A), (B 1), and (C), having a polyalkylene oxide moiety and a cationic group.

<2>
The processing agent according to <1>, in which a ratio of a total number of moles of the M unit and the Q unit to a total number of moles of the M unit, the Q unit, a T unit represented by $R^1SiO_{3/2}$, and a D unit represented by $(R^1)_2SiO_{2/2}$ ($R^1$ is the same as described above) in the component (A) is preferably 50% or more, more preferably 70% or more, still more preferably 80% or more, even more preferably 90% or more, and 100% or less.
<3>
The processing agent according to <1> or <2>, in which the component (A) is one or more selected from the group consisting of trimethylsiloxysilicate, phenylpropyldimethylsiloxysilicate, fluorine-modified alkyldimethylsiloxysilicate, and a cross-polymer obtained by crosslinking these siloxysilicates with dimethiconol, preferably one or more selected from the group consisting of trimethylsiloxysilicate, trifluoropropyldimethyltrimethylsiloxysilicate, and a (trimethylsiloxysilicate/dimethiconol) cross-polymer, more preferably one or more selected from the group consisting of trimethylsiloxysilicate and trifluoropropyldimethyltrimethylsiloxysilicate, and still more preferably trimethylsiloxysilicate.
<4>
The processing agent according to any one of <1> to <3>, in which the trimethylsiloxysilicate has a viscosity at 25°C of a 50% by mass solution in which the trimethylsiloxysilicate as an active ingredient is dissolved in dimethicone KF-96L-2cs of preferably 10 $mm^2/s$ or more, more preferably 15 $mm^2/s$ or more, still more preferably 20 $mm^2/s$ or more, and even more preferably 50 $mm^2/s$ or more.
<5>
The processing agent according to any one of <1> to <4>, in which the component (B) and the component (B1) are preferably a silicone-based film-forming polymer, more preferably one or more selected from the group consisting of a silicone resin containing a T unit represented by $R^1SiO_{3/2}$ and substantially not containing a Q unit represented by $SiO_{4/2}$ ($R^1$ is the same as described above) and a silicone polymer containing a polysiloxane moiety and a moiety composed of a non-silicone organic chain, more preferably a silicone polymer containing a polysiloxane moiety and a moiety composed of a non-silicone organic chain, and still more preferably one or more selected from the group

consisting of the following components (B-1) to (B-5):

(B-1) An acryl silicone polymer
(B-2) A silicone-modified alicyclic structure-containing polymer
(B-3) A silicone-modified pullulan
(B-4) A polyurea/urethane silicone
(B-5) An oxazoline-modified silicone

<6>
The processing agent according to <5>, in which the acryl silicone polymer as the component (B-1) is one or more selected from the group consisting of an acrylic polymer having a carbosiloxane dendrimer structure in the side chain, an acryl-silicone graft copolymer, and a graft-type copolymer or alternate block-type copolymer where a structural unit of a polysiloxane group and a structural unit of a polymer of an unsaturated monomer bond via a sulfide bond, preferably one or more selected from the group consisting of an acrylic polymer having a carbosiloxane dendrimer structure in the side chain and an acryl-silicone graft copolymer, more preferably one or more selected from the group consisting of an (acrylates/polytrimethylsiloxymethacrylate) copolymer and an (acrylates/dimethicone) copolymer, and still more preferably an (acrylates/dimethicone) copolymer.
<7>
The processing agent according to <5>, in which the silicone-modified alicyclic structure-containing polymer as the component (B-2) is a silicone-modified cyclic polyolefin, preferably a silicone-modified polynorbornene represented by the following general formula (B-2-1), and more preferably a silicone-modified polynorbornene represented by the following formula (B-2-2):

[Chem 13]

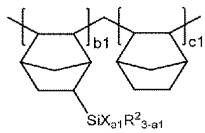
(B-2-1)

(in the formula, each $R^2$ is independently an alkyl group having 1 or more and 12 or less carbon atoms, X is a group represented by the following formula (I), a1 is an integer of 1 or more and 3 or less, b1 and c1 each are a repeating unit number, and are each independently an integer of 1 or more);

[Chem 14]

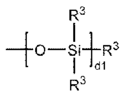
(i)

(in the formula, each $R^3$ is independently a hydrocarbon group having 1 or more and 12 or less carbon atoms, and d1 is an integer of 1 or more and 5 or less);

[Chem 15]

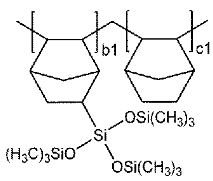
(B-2-2)

(in the formula, b1 and c1 are the same as described above).
<8>
The processing agent according to <5>, in which the silicone-modified pullulan as the component (B-3) is pullulan having a silicone structure in a side chain, and preferably a silicone-modified pullulan in which at least a part of hydrogen atoms of OH groups in pullulan is substituted with a group represented by the following general formula (ii):

[Chem 16]

$-R^4-SiX_{a1}R^2_{3\text{-}a1}$ (ii)

in the formula, $R^4$ is a single bond or a divalent organic group, $R^2$, X, and a1 are the same as described above, X is preferably a trimethylsiloxy group, and a1 is preferably 3.

<9>

The processing agent according to <5>, in which the polyurea/urethane silicone as the component (B-4) is preferably a polysiloxane/polyurea/polyurethane block terpolymer.

<10>

The processing agent according to <5>, in which the oxazoline-modified silicone as the component (B-5) is an organopolysiloxane in which a poly(N-acylalkyleneimine) segment consisting of a repeating unit represented by the following general formula (B-5-1) is bonded to at least two silicon atoms of an organopolysiloxane segment constituting a main chain via an alkylene group containing a heteroatom, and is preferably one or more selected from the group consisting of poly(N-formylethyleneimine) organosiloxane, poly(N-acetylethyleneimine) organosiloxane, and poly(N-propionylethyleneimine) organosiloxane, and more preferably a polymer represented by INCI nomenclature "Polysilicone-9":

[Chem 17]

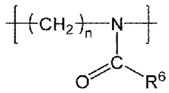 (B-5-1)

(in the formula, $R^6$ represents a hydrogen atom, an alkyl group having 1 or more and 22 or less carbon atoms, an aralkyl group having 7 or more and 22 or less carbon atoms, or an aryl group having 6 or more and 22 or less carbon atoms, and n is 2 or 3).

<11>

The processing agent according to any one of <5> to <10>, in which the component (B) and the component (B1) preferably include one or more selected from the group consisting of an acryl silicone polymer which is the component (B-1) and an oxazoline-modified silicone which is the component (B-5), and more preferably include an acryl silicone polymer which is the component (B-1).

<12>

The processing agent according to any one of <1> to <11>, in which the component (B) and the component (B1) preferably include one or more selected from the group consisting of an (acrylates/polytrimethylsiloxymethacrylate) copolymer, an (acrylates/dimethicone) copolymer, and an oxazoline-modified silicone, more preferably include one or more selected from the group consisting of an (acrylates/dimethicone) copolymer and an oxazoline-modified silicone, and still more preferably include an (acrylates/dimethicone) copolymer.

<13>

The processing agent according to any one of <5> to <12>, in which the total content of the component (B-1) and the component (B-5) in the component (B) and the component (B 1) is preferably 50% by mass or more, more preferably 70% by mass or more, still more preferably 80% by mass or more, even more preferably 90% by mass or more, and 100% by mass or less.

<14>

The processing agent according to any one of <1> to <13>, in which the degree of polymerization of the component (C) is preferably 2,600 or more and 10,000 or less, more preferably 2,600 or more and 4,500 or less, still more preferably 2,900 or more and 4,000 or less, and even more preferably 3,200 or more and 3,900 or less.

<15>

The processing agent according to any one of <1> to <14>, in which the component (C) is an organopolysiloxane represented by the following general formula (1):

[Chem 18]

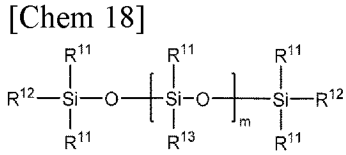 (1)

in which each $R^{11}$ independently represents a hydrocarbon group having 1 or more and 6 or less carbon atoms, each $R^{12}$ independently represents a hydroxy group, an alkoxy group having 1 or more and 6 or less carbon atoms, or a

hydrocarbon group having 1 or more and 6 or less carbon atoms, $R^{13}$ represents a hydrocarbon group having 1 or more and 6 or less carbon atoms or a primary to tertiary amino group-containing group, m represents a degree of polymerization and is a number of 2,200 or more and 20,000 or less, and m's $R^{13}$s may be the same as or different from each other.

<16>

The processing agent according to <15>, in which in the general formula (1), m is preferably 2,600 or more and 10,000 or less, more preferably 2,600 or more and 4,500 or less, still more preferably 2,900 or more and 4,000 or less, and even more preferably 3,200 or more and 3,900 or less.

<17>

The processing agent according to any one of <1> to <16>, in which the viscosity at 25°C of the component (C) is preferably 1,000,000 $mm^2/s$ or more and 125,000,000 $mm^2/s$ or less, more preferably 3,000,000 $mm^2/s$ or more and 125,000,000 $mm^2/s$ or less, still more preferably 5,000,000 $mm^2/s$ or more and 50,000,000 $mm^2/s$ or less, and even more preferably 10,000,000 $mm^2/s$ or more and 40,000,000 $mm^2/s$ or less.

<18>

The processing agent according to any one of <1> to <17>, in which the component (C) is preferably one or more selected from the group consisting of dimethylpolysiloxane, methylphenylpolysiloxane, aminopropylmethylpolysiloxane, and dimethiconol, and more preferably dimethylpolysiloxane.

<19>

The processing agent according to any one of <1> to <18>, in which the component (D) is at least one selected from the group consisting of a silicone (D1) having a repeating unit represented by the following general formula (2) and a silicone (D2) having a repeating unit represented by the following general formula (3):

[Chem 19]

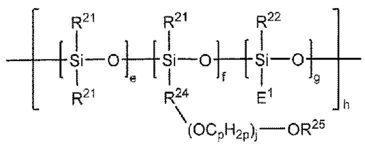

(2)

in the formula (2), $R^{21}$ represents a monovalent hydrocarbon group having 1 or more and 6 or less carbon atoms, $R^{22}$ represents any of $R^{21}$ or $E^1$, $E^1$ represents a monovalent group represented by $-R^{23}-Z^1$ (where $R^{23}$ represents a divalent hydrocarbon group having 1 or more and 6 or less carbon atoms, and $Z^1$ represents a primary to tertiary amino group-containing group), $R^{24}$ represents a divalent hydrocarbon group having 1 or more and 6 or less carbon atoms, $R^{25}$ represents a hydrogen atom or a monovalent hydrocarbon group having 1 or more and 4 or less carbon atoms, e is a number of 1 or more and 50 or less, f is a number of 1 or more and 50 or less, g is a number of 1 or more and 50 or less, h is a number of 1 or more, j is a number of 2 or more and 100 or less, p is a number of 2 or more and 10 or less, the bonding order of the parenthesized structural units is not limited, and the bonding form thereof may be in a block form or a random form,.j's $(OC_pH_{2p})$s can be the same or different, and plural $R^{21}$'s, $R^{22}$'s, $R^{24}$'s, $R^{25}$'s and $E^1$'s can be the same or different;

[Chem 20]

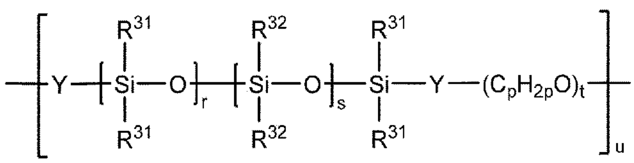

(3)

in the formula (3), $R^{31}$ represents a monovalent hydrocarbon group having 1 or more and 6 or less carbon atoms, $R^{32}$ represents any of $R^{31}$ or $E^2$, $E^2$ represents a monovalent group represented by $-R^{33}-Z^2$ (where $R^{33}$ represents a single bond, or a divalent hydrocarbon group having 1 or more and 20 or less carbon atoms, and $Z^2$ represents a primary to tertiary amino group-containing group), Y represents a single bond, or a divalent group having 1 or more and 12 or less carbon atoms, in the case where all $R^{32}$'s are $R^{31}$'s, at least one Y is an amino group-containing divalent group, in the case where all Y's are divalent groups not containing an amino group, at least one $R^{32}$ is $E^2$, r is a number of 1 or more, s is a number of 1 or more, t is a number of 2 or more and 100 or less, and u is a number of 1 or more, p is the same as above, and is a number of 2 or more and 10 or less, the bonding order of the parenthesized structural units is not limited, and the bonding form thereof may be in a block form or a random form, t's $(C_pH_{2p}O)$s can be the same or different, and plural $R^{31}$'s, $R^{32}$'s, $E^2$'s and Y's can be the same or different.

<20>

The processing agent according to <19>, in which the component (D1) is a compound represented by the following general formula (2-1):

[Chem 21]

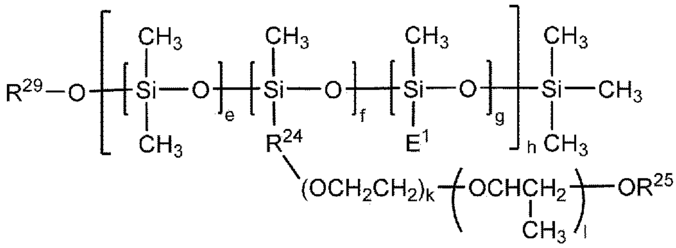

(2-1)

in the formula (2-1), $E^1$, $R^{24}$, $R^{25}$, e, f, g and h are the same as above, $R^{29}$ represents a monovalent hydrocarbon group having 1 or more and 4 or less carbon atoms, or a trimethylsilyl group, k is a number of 1 or more and 50 or less, l is a number of 0 or more and 50 or less, and is preferably a number of 1 or more and 50 or less.

<21>

The processing agent according to <19> or <20>, in which the component (D2) is one or more selected from the group consisting of a silicone having a structure represented by the following general formula (3-1) and a silicone having a structure represented by the following general formula (3-2):

[Chem 22]

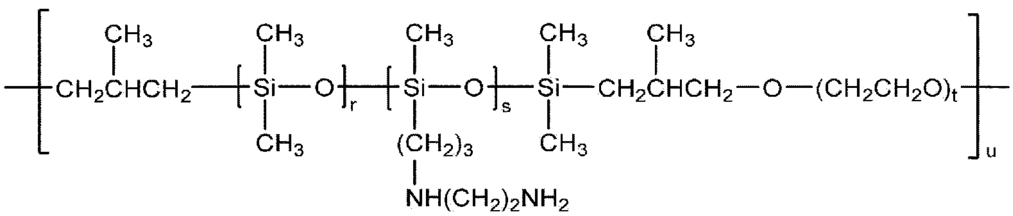

(3-1)

in the formula (3-1), r, s, t, and u are the same as described above;

[Chem 23]

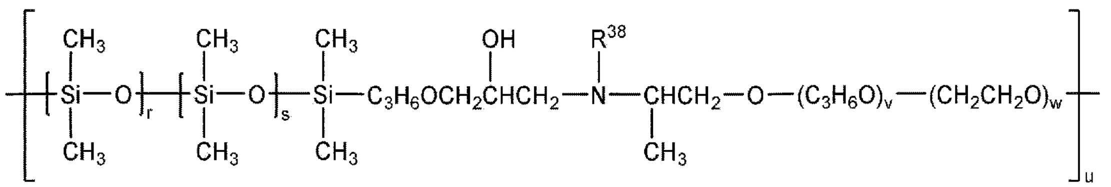

(3-2)

in the formula (3-2), $R^{38}$ represents a hydrogen atom or an alkyl group having 1 or more and 3 or less carbon atoms, r, s, and u are the same as described above, v is a number of 0 or more and 50 or less, and preferably a number of 2 or more and 50 or less, w is a number of 2 or more and 100 or less, v + w is a number of 2 or more and 100 or less, preferably a number of 4 or more and 80 or less, and more preferably a number of 10 or more and 50 or less.

<22>

The processing agent according to any one of <19> to <21>, in which the component (D) is a silicone (D2) having a repeating unit represented by the general formula (3), more preferably one or more selected from the group consisting of a silicone having a structure represented by the general formula (3-1) and a silicone having a structure represented by the general formula (3-2), and still more preferably a silicone having a structure represented by the general formula (3-1).

<23>

The processing agent according to any one of <1> to <22>, in which the content of the component (A) in the processing agent (I) and the processing agent (II) is preferably 0.5% by mass or more and 30% by mass or less, more preferably 1.0% by mass or more and 20% by mass or less, still more preferably 1.5% by mass or more and 10% by mass or less, and even more preferably 2.0% by mass or more and 5.0% by mass or less.

<24>
The processing agent according to any one of <1> to <23>, in which the content of the component (B) in the processing agent (I) is preferably 0.1% by mass or more and 30% by mass or less, more preferably 0.5% by mass or more and 25% by mass or less, still more preferably 1.0% by mass or more and 20% by mass or less, and even more preferably 1.5% by mass or more and 15% by mass or less.
<25>
The processing agent according to any one of <1> to <24>, in which the total content of the component (A) and the component (B) in the processing agent (I) is preferably 1% by mass or more and 50% by mass or less, more preferably 2% by mass or more and 40% by mass or less, still more preferably 2% by mass or more and 30% by mass or less, even more preferably 2% by mass or more and 20% by mass or less, even more preferably 3% by mass or more and 15% by mass or less, and even more preferably 4% by mass or more and 15% by mass or less.
<26>
The processing agent according to any one of <1> to <25>, in which a mass ratio [(A)/{(A) + (B)}] of the content of the component (A) to the total content of the component (A) and the component (B) in the processing agent (I) is preferably 0.05 or more and 0.98 or less, more preferably 0.10 or more and 0.90 or less, still more preferably 0.15 or more and 0.80 or less, and even more preferably 0.20 or more and 0.60 or less.
<27>
The processing agent according to any one of <1> to <26>, in which the content of the component (C) in the processing agent (I) and the processing agent (II) is preferably 0.01% by mass or more and 20% by mass or less, more preferably 0.02% by mass or more and 10% by mass or less, still more preferably 0.05% by mass or more and 5.0% by mass or less, and even more preferably 0.10% by mass or more and 3.0% by mass or less.
<28>
The processing agent according to any one of <1> to <27>, in which a mass ratio [(C)/{(A) + (B)}] of the content of the component (C) to the total content of the component (A) and the component (B) in the processing agent (I) is preferably 0.01 or more and 3 or less, more preferably 0.05 or more and 2 or less, still more preferably 0.1 or more and 0.5 or less, and even more preferably 0.2 or more and 0.3 or less.
<29>
The processing agent according to any one of <1> to <28>, in which the content of the component (B1) in the processing agent (II) is preferably 0.1% by mass or more and 30% by mass or less, more preferably 0.5% by mass or more and 25% by mass or less, still more preferably 1.0% by mass or more and 20% by mass or less, even more preferably 1.5% by mass or more and 15% by mass or less, even more preferably 1.5% by mass or more and 10% by mass or less, and even more preferably 1.5% by mass or more and 5.0% by mass or less.
<30>
The processing agent according to any one of <1> to <29>, in which the total content of the component (A) and the component (B1) in the processing agent (II) is preferably 0.6% by mass or more and 50% by mass or less, more preferably 2.0% by mass or more and 40% by mass or less, still more preferably 2.0% by mass or more and 30% by mass or less, even more preferably 2.0% by mass or more and 20% by mass or less, even more preferably 3.0% by mass or more and 15% by mass or less, and even more preferably 3.5% by mass or more and 10% by mass or less.
<31>
The processing agent according to any one of <1> to <30>, in which a mass ratio [(A)/{(A) + (B1)}] of the content of the component (A) to the total content of the component (A) and the component (B1) in the processing agent (II) is preferably 0.05 or more and 0.98 or less, more preferably 0.10 or more and 0.90 or less, still more preferably 0.15 or more and 0.80 or less, even more preferably 0.20 or more and 0.70 or less, even more preferably 0.30 or more and 0.60 or less, and even more preferably 0.40 or more and 0.60 or less.
<32>
The processing agent according to any one of <1> to <31>, in which a mass ratio [(C)/{(A) + (B1)}] of the content of the component (C) to the total content of the component (A) and the component (B1) in the processing agent (II) is preferably 0.01 or more and 3 or less, more preferably 0.05 or more and 2 or less, still more preferably 0.1 or more and 1 or less, even more preferably 0.2 or more and 0.5 or less, and even more preferably 0.2 or more and 0.3 or less.
<33>
The processing agent according to any one of <1> to <32>, in which the content of the component (D) in the processing agent (II) is preferably 0.01% by mass or more and 20% by mass or less, more preferably 0.02% by mass or more and 10% by mass or less, still more preferably 0.05% by mass or more and 5.0% by mass or less, even more preferably 0.1% by mass or more and 3.0% by mass or less, even more preferably 0.2% by mass or more and 2.0% by mass or less, and even more preferably 0.3% by mass or more and 1.0% by mass or less.
<34>
The processing agent according to any one of <1> to <33>, in which the total content of the components (A), (B1), (C), and (D) in the processing agent (II) is preferably 1.5% by mass or more and 50% by mass or less, more preferably 2.0%

by mass or more and 40% by mass or less, still more preferably 2.0% by mass or more and 30% by mass or less, even more preferably 3.0% by mass or more and 20% by mass or less, even more preferably 4.0% by mass or more and 20% by mass or less, and even more preferably 5.0% by mass or more and 15% by mass or less.

<35>

The processing agent according to any one of <1> to <34>, in which a mass ratio [(D)/{(A) + (B1) + (C)}] of the content of the component (D) to the total content of the components (A), (B1), and (C) in the processing agent (II) is preferably 0.001 or more and 3 or less, more preferably 0.005 or more and 2 or less, still more preferably 0.01 or more and 1 or less, even more preferably 0.02 or more and 0.5 or less, even more preferably 0.02 or more and 0.3 or less, and even more preferably 0.05 or more and 0.1 or less.

<36>

The processing agent according to any one of <1> to <35>, in which the processing agent further contains a volatile solvent as a component (E).

<37>

The processing agent according to <36>, in which the component (E) is one or more selected from the group consisting of an alcohol-based solvent, an ether-based solvent, a ketone-based solvent, an ester-based solvent, a hydrocarbon-based solvent, and a silicone-based solvent.

<38>

The processing agent according to <37>, in which the component (E) preferably includes one or more selected from the group consisting of an alcohol-based solvent, a hydrocarbon-based solvent, and a silicone-based solvent, more preferably includes one or more selected from the group consisting of ethanol, pentane, isopentane, hexane, isohexane, heptane, isoheptane, decane, isodecane, dodecane, isododecane, tridecane, isotridecane, tetradecane, isotetradecane, and light liquid isoparaffin, dimethylpolysiloxane having a viscosity at 25°C of 10 $mm^2/s$ or less, methyltrimethicone, and methylphenylpolysiloxane having a viscosity at 25°C of 20 $mm^2/s$ or less, still more preferably includes one or more selected from the group consisting of dimethylpolysiloxane having a viscosity at 25°C of 5 $mm^2/s$ or less, methyltrimethicone, isodecane, isododecane, isotetradecane, and light liquid isoparaffin, even more preferably includes one or more selected from the group consisting of ethanol, isodecane, isododecane, isotetradecane, and light liquid isoparaffin, and even more preferably includes isododecane.

<39>

The processing agent according to any one of <36> to <38>, in which the content of the component (E) in the processing agent (I) is preferably 40% by mass or more and 98% by mass or less, more preferably 50% by mass or more and 97% by mass or less, and still more preferably 60% by mass or more and 97% by mass or less.

<40>

The processing agent according to any one of <36> to <38>, in which the content of the component (E) in the processing agent (II) is preferably 40% by mass or more and 98% by mass or less, more preferably 50% by mass or more and 95% by mass or less, still more preferably 60% by mass or more and 95% by mass or less, and even more preferably 75% by mass or more and 90% by mass or less.

<41>

The processing agent according to any one of <1> to <40>, in which the processing agent (I) further contains a cationic surfactant as a component (F1).

<42>

The processing agent according to any one of <1> to <40>, in which the processing agent (II) further contains a cationic compound other than the components (A), (B1), (C), and (D) as a component (F).

<43>

The processing agent according to <42>, in which the component (F) is preferably one or more selected from the group consisting of (F1) a cationic surfactant and (F2) a cationic polymer containing a quaternary ammonium cation.

<44>

The processing agent according to <41> or <43>, in which the component (F1) is preferably one or more selected from the group consisting of an alkyltrimethylammonium salt, an alkoxyalkyltrimethylammonium salt, an alkylamidoalkyltrimethylammonium salt, an alkyldimethylamine and a salt thereof, an alkoxyalkyldimethylamine and a salt thereof, and an alkylamidoalkyldimethylamine and a salt thereof, more preferably one or more selected from the group consisting of an alkyltrimethylammonium salt, an alkoxyalkyltrimethylammonium salt, and an alkoxyalkyldimethylamine and a salt thereof, still more preferably one or more selected from the group consisting of an alkyltrimethylammonium salt and an alkoxyalkyltrimethylammonium salt, even more preferably one or more selected from the group consisting of stearyltrimethylammonium chloride [steartrimonium chloride], behenyltrimethylammonium chloride [behentrimonium chloride], and stearoxypropyltrimethylammonium chloride [stearoxypropyltrimonium chloride].

<45>

The processing agent according to <43>, in which the component (F2) is one or more selected from the group consisting of a methacryloyl ethyl trimethyl ammonium salt polymer, a quaternized dialkylaminoalkyl (meth)acrylate

polymer, a diallyl quaternized ammonium salt polymer, a methacrylamidopropyl trimethyl ammonium salt polymer, and a vinyl imidazolium trichloride/vinyl pyrrolidone copolymer (polyquaternium-16), preferably one or more selected from the group consisting of a quaternized dialkylaminoalkyl (meth)acrylate polymer and a diallyl quaternized ammonium salt polymer, more preferably a diallyl quaternized ammonium salt polymer, still more preferably one or more selected from the group consisting of a dimethyl diallyl ammonium chloride polymer (polyquaternium-6), a dimethyldiallyl ammonium chloride/acrylic acid copolymer (polyquaternium-22), a dimethyldiallyl ammonium chloride/acrylamide copolymer (polyquaternium-7), and an acrylamide/acrylic acid/dimethyldiallyl ammonium chloride copolymer (polyquaternium-39), and even more preferably a dimethyl diallyl ammonium chloride polymer (polyquaternium-6).

<46>

The processing agent according to <42> or <43>, in which the component (F) is preferably one or more selected from the group consisting of an alkyltrimethylammonium salt, an alkoxyalkyltrimethylammonium salt, an alkylamidoalkyltrimethylammonium salt, an alkyldimethylamine and a salt thereof, an alkoxyalkyldimethylamine and a salt thereof, an alkylamidoalkyldimethylamine and a salt thereof, a quaternized dialkylaminoalkyl (meth)acrylate polymer, and a diallyl quaternized ammonium salt polymer, more preferably one or more selected from the group consisting of an alkyltrimethylammonium salt, an alkoxyalkyltrimethylammonium salt, an alkoxyalkyldimethylamine and a salt thereof, and a diallyl quaternized ammonium salt polymer, still more preferably one or more selected from the group consisting of an alkyltrimethylammonium salt, an alkoxyalkyltrimethylammonium salt, a dimethyl diallyl ammonium chloride polymer (polyquaternium-6), a dimethyldiallyl ammonium chloride/acrylic acid copolymer (polyquaternium-22), a dimethyldiallyl ammonium chloride/acrylamide copolymer (polyquaternium-7), and an acrylamide/acrylic acid/dimethyldiallyl ammonium chloride copolymer (polyquaternium-39), and even more preferably one or more selected from the group consisting of stearyltrimethylammonium chloride [steartrimonium chloride], behenyltrimethylammonium chloride [behentrimonium chloride], stearoxypropyltrimethylammonium chloride [stearoxypropyltrimonium chloride], and a dimethyl diallyl ammonium chloride polymer (polyquaternium-6).

<47>

The processing agent according to <41>, in which the content of the component (F1) in the processing agent (I) is preferably 0.05% by mass or more and 15% by mass or less, more preferably 0.10% by mass or more and 10% by mass or less, still more preferably 0.10% by mass or more and 5.0% by mass or less, and even more preferably 0.20% by mass or more and 2.0% by mass or less.

<48>

The processing agent according to any one of <42> to <46>, in which the content of the component (F) in the processing agent (II) is preferably 0.05% by mass or more and 15% by mass or less, more preferably 0.10% by mass or more and 10% by mass or less, still more preferably 0.1% by mass or more and 5.0% by mass or less, even more preferably 0.1% by mass or more and 2.0% by mass or less, even more preferably 0.1% by mass or more and 1.0% by mass or less, even more preferably 0.1% by mass or more and 0.5% by mass or less, and even more preferably 0.2% by mass or more and 0.5% by mass or less.

<49>

The processing agent according to any one of <42> to <46> and <48>, in which a mass ratio [(F)/(C)] of the content of the component (F) to the content of the component (C) in the processing agent (II) is preferably 0.005 or more and 10 or less, more preferably 0.010 or more and 5.0 or less, still more preferably 0.020 or more and 2.0 or less, even more preferably 0.050 or more and 1.0 or less, and even more preferably 0.10 or more and 0.50 or less.

<50>

The processing agent according to any one of <1> to <49>, in which the content of water in the processing agent (I) is preferably 5% by mass or less, more preferably less than 5% by mass, and still more preferably less than 2% by mass.

<51>

The processing agent according to any one of <1> to <50>, in which the content of water in the processing agent (II) is preferably 10% by mass or less, more preferably 5% by mass or less, still more preferably less than 5% by mass, and even more preferably less than 2% by mass.

<52>

The processing agent according to any one of <1> to <51>, in which the viscosity at 30°C of the processing agent is preferably 1.0 mPa·s or more and 1,000 mPa·s or less, more preferably 1.5 mPa·s or more and 500 mPa·s or less, still more preferably 1.7 mPa·s or more and 250 mPa·s or less, even more preferably 1.7 mPa·s or more and 100 mPa·s or less, even more preferably 1.7 mPa·s or more and 20 mPa·s or less.

<53>

The processing agent according to any one of <1> to <52>, in which the processing agent is used without being washed off after being applied to hair or a fiber for a headwear product.

<54>

The processing agent according to any one of <1> to <53>, in which the processing agent is a detergent, a rinse, a

conditioning agent, a treatment agent, a styling agent, a hair dye, or a hair growth agent, preferably a conditioning agent, a treatment agent, or a styling agent, and more preferably a styling agent.

<55>

A method for treating hair or a fiber for a headwear product, including a step of applying the processing agent according to any one of <1> to <54> to hair or a fiber for a headwear product and then drying the hair or the fiber for a headwear product.

<56>

The processing method according to <55>, in which the processing agent is applied to the hair or the fiber for a headwear product, preferably in a dry state, and then dried to be used without being washed off.

<57>

A method for styling hair or a fiber for a headwear product, including a step of shaping the hair or the fiber for a headwear product treated by the processing method according to <55> or <56>, while heating.

Examples

Hereinafter, the present invention will be described with reference to Examples, but the present invention is not limited to the scope of Examples. In these Examples, various measurements and evaluations were performed by the following methods.

<Method for Measuring Viscosity of Hair Processing Agent>

Using a B-type viscometer (TVB-10, manufactured by Toki Sangyo Co., Ltd., rotor L/Adp), the steady shear viscosity of the hair processing agent was measured under the conditions of 30°C and a rotor rotation speed of 60 r/m (rotation speed of 1.5 r/m in Example 6 only).

<Touch Feeling in Dry State>

A tress of 30 cm in length and 5 g in mass was prepared using straight hair of Japanese women who had not been subjected to chemical treatment. The tress was washed with a plain shampoo having the following composition and then completely dried with a dryer (Nobby Professional Protective Ion Hair Dryer NIB3000 [SPEED MODE] manufactured by Tescom Denki Co., Ltd.).

The hair processing agent of each example was uniformly mixed and applied to the tress at a bath ratio of 1:0.2 (hair mass:hair processing agent mass). Next, using the above-mentioned dryer, while combing, warm air was blown from a position away from the tress by 15 cm for 2 minutes, and then cold air was blown for 1 minute for drying. The hair surface temperature was 55 to 60°C. The hair surface temperature was measured using an infrared radiation thermometer ("SK-8940" manufactured by Sato Keiryoki Mfg. Co., Ltd.) from a position 10 cm from the hair tress so that the hair was irradiated with infrared rays perpendicular to the hair. Regarding the touch feeling of the tress after drying, three expert panelists performed a sensory evaluation based on the following criteria, and the total score of the three panelists was calculated.

(Evaluation criteria)

5: No stickiness is felt at all.
4: No stickiness is felt almost at all.
3: No stickiness is felt much at all.
2: Stickiness is felt slightly.
1: Stickiness is felt.

(Composition of Plain Shampoo)

| Component | (% by mass) |
|---|---|
| Sodium polyoxyethylene(2) lauryl ether sulfate (*1) | 15.5 |
| Lauric acid diethanolamide (*2) | 1.5 |

(continued)

| Component | (% by mass) |
|---|---|
| Edetate tetrasodium salt | 0.3 |
| Sodium benzoate | 1.43 |
| Purified water | balance |
| Total | 100.0 |
| *1: 57.4% by mass as EMAL 227 (manufactured by Kao Corporation, active ingredient 27% by mass) | |
| *2: AMINON L-02 (manufactured by Kao Corporation) | |

<Touch Feeling in Wet State>

A tress was prepared in the same manner as in the evaluation of "Touch Feeling in Dry State", and the hair processing agent of each example was applied to the tress and then dried. The dried tress was washed with the plain shampoo having the above-described composition, and then rinsed with warm water at 40°C. Three expert panelists performed a sensory evaluation based on the following criteria, and the total score of the three panelists was calculated. The untreated tress was washed with a plain shampoo having the above-described composition and then rinsed with warm water at 40°C.

(Evaluation criteria)

5: Smooth and good finger combing.
4: Slightly smooth and almost no feeling of resistance to finger combing.
3: Finger combing equivalent to that of untreated tress.
2: Slightly resistance to finger combing.
1: Poor finger combing.

<Property of Imparting Feeling of Volume during Styling>

Using the straight hair of a Caucasian woman who had not been subjected to chemical treatment, a tress was prepared by transplanting hair of 30 cm in length at a density of 200 hairs/$cm^2$ and a hair-growth angle of 60° in an area of 6 cm × 6 cm square. The tress was washed with a plain shampoo having the above-described composition and then completely dried with a dryer (Nobby Professional Protective Ion Hair Dryer NIB3000 [SPEED MODE] manufactured by Tescom Denki Co., Ltd.).

Each of the hair processing agents shown in Tables 1 to 3 was uniformly mixed and applied to the tress at a bath ratio of 1:0.2 (hair mass:hair processing agent mass). Next, using the above-mentioned dryer, while combing, warm air was blown from a position away from the tress by 15 cm for 2 minutes. The tress was washed again with a plain shampoo of the above-described composition, then dried with a towel, and completely dried with the dryer in a state in which the hair tips were directed downward. Regarding the feeling of volume when the tress after drying was placed upside down on a table, three expert panelists visually evaluated the feeling of volume based on the following criteria, and the total score of the three expert panelists was calculated. The untreated tress was washed with a plain shampoo having the above-described composition, dried with a towel, completely dried with the dryer in a state in which the hair tips were directed downward, and then placed upside down on a table to be used for comparison. The hair surface temperature during drying was 55 to 60°C. The hair surface temperature was measured using an infrared radiation thermometer ("SK-8940" manufactured by Sato Keiryoki Mfg. Co., Ltd.) from a position 10 cm from the hair tress so that the hair was irradiated with infrared rays perpendicular to the hair.

(Evaluation criteria)

5: Feeling of volume is firm.
4: Feeling of volume is present.
3: Feeling of volume is somewhat present.
2: Feeling of volume is weak.

1: Feeling of volume is absent (equivalent to untreated tress).

<Heat Setting Property>

A tress of 30 cm in length and 1 g in mass was prepared using the curly hair of a Caucasian woman who had not been subjected to chemical treatment. The tress was washed with a plain shampoo having the above-described composition and then air dried.

The hair processing agent of each example was uniformly mixed and applied to the tress at a bath ratio of 1:0.2 (hair mass:hair processing agent mass). Next, using the above-mentioned dryer, while combing, warm air was blown from a position away from the tress by 15 cm for 2 minutes, and then cold air was blown for 1 minute for drying.

In the tress after drying, the hair was sandwiched using a flat hair iron ("One am Digital Hair Iron for Straightening 38 mm AHI-938" manufactured by Miki Denki Sangyo Co., Ltd., set temperature: 180°C), and heat styling was performed by sliding the hair at a speed of 10 cm/sec. The tresses subjected to the heat styling were visually evaluated by three expert panelists based on the following criteria, and the total score of the three expert panelists was calculated. The hair surface temperature at the time of ironing was 130 to 140°C. The hair surface temperature was measured using an infrared radiation thermometer ("SK-8940" manufactured by Sato Keiryoki Mfg. Co., Ltd.) from a position 10 cm from the hair tress so that the hair was irradiated with infrared rays perpendicular to the hair.

(Evaluation criteria)

5: The curl of the hair is straightened and the whole tress is gathered.
4: The curl of the hair is elongated and the whole tress is almost gathered.
3: The curl of the hair is elongated, but the gathering of the tress is weak.
2: The curl of the hair is almost elongated, but the tress is not gathered.
1: The curl of the hair is not elongated, and the tress is not gathered.

<Resistance to Hair Washing (Property of Imparting Feeling of Volume during Styling after Washing Hair 7 Times)>

The tress treated and dried in the same manner as in the above-described evaluation of "Property of Imparting Feeling of Volume during Styling" was washed with a plain shampoo having the above-described composition, rinsed with warm water at 40°C, and dried six times. After washing and drying the tress six times, the tress was washed again with a plain shampoo having the above-described composition, then dried with a towel, and completely dried with the above-described dryer in a state in which the hair tips were directed downward. The feeling of volume when the tress after drying was placed upside down on a table was evaluated in the same manner as described above. The untreated tress was washed with a plain shampoo having the above-described composition, dried with a towel, and then completely dried with the dryer in a state in which the hair tips were directed downward, and used for comparison.

<Resistance to Hair Washing (Heat Setting Property after Washing Hair 7 Times)>

The tress treated and dried in the same manner as in the above-described evaluation of "Heat Setting Property" was washed with a plain shampoo having the above-described composition, rinsed with warm water at 40°C, and dried seven times. After washing and drying seven times, the tress was subjected to heat styling in the same manner as described above, and the heat setting property was evaluated.

<Slipperiness of Hair Iron>

A tress of 30 cm in length and 1 g in mass was prepared using the curly hair of a Caucasian woman who had not been subjected to chemical treatment. The tress was washed with a plain shampoo having the above-described composition and then air dried.

Each of the hair processing agents shown in Table 4 was uniformly mixed and applied to the tress at a bath ratio of 1:0.2 (hair mass:hair processing agent mass). Next, using the above-mentioned dryer, while combing, warm air was blown from a position away from the tress by 15 cm for 2 minutes, and then cold air was blown for 1 minute for drying. The tress was fixed to a spring scale, and then the hair was sandwiched using a flat hair iron ("One am Digital Hair Iron for Straightening 38 mm AHI-938" manufactured by Miki Denki Sangyo Co., Ltd., set temperature: 180°C), and heat styling was performed by sliding the hair at a speed of 10 cm/sec. At this time, the maximum value of the load (g) displayed on the spring scale was recorded. The above measurement was repeated 10 times, and the average of the maximum values of

the load was obtained and shown in Table 4. The smaller the value, the better the slipperiness of the hair iron.

Examples 1 to 12 and Comparative Examples 1 to 6 (Preparation and Evaluation of Hair Processing Agent (I) (Hair Styling Agent))

The components shown in Tables 1 to 3 were blended at the ratios shown in the tables and mixed until uniform to prepare a hair processing agent (I). Various evaluations were performed by the above-described methods using the obtained hair processing agent (I). The results are shown in Tables 1 to 3.

The blending amounts (% by mass) shown in Tables 1 to 3 are all active ingredient amounts.

Table 1

| Blending composition (% by mass) | | Product name | Example | | | |
|---|---|---|---|---|---|---|
| | | | 1 | 2 | 3 | 4 |
| (A) | Trimethylsiloxysilicate (MQ resin, Hard) | X-21-5595 *1 (Shin-Etsu Chemical Co., Ltd.) | 2.5 | 10.0 | 1.0 | 2.5 |
| | Trimethylsiloxysilicate (MQ resin, Soft) | X-21-5616 *2 (Shin-Etsu Chemical Co., Ltd.) | | | | |
| (B) | Polypropylsilsesquioxane (MT resin) | DOWSIL 680 ID Fluid *3 (Dow Toray Co., Ltd.) | | | | |
| | (Acrylates/dimethicone) copolymer | KP-550 *4 (Shin-Etsu Chemical Co., Ltd.) | 2.0 | 2.0 | 2.0 | 10.0 |
| | Oxazoline-modified silicone | Polysilicone-9 (Kao Corporation) *5 | | | | |
| (C) | High-molecular weight dimethylpolysiloxane (20,000,000 $mm^2/s$) | SILSOFT B3020 *6 (Momentive Performance Materials, Inc.) | 1.0 | 1.0 | 1.0 | 1.0 |
| (C') | High-molecular weight dimethylpolysiloxane (100,000 $mm^2/s$) | KF-96H-100000cs *7 (Shin-Etsu Chemical Co., Ltd.) | | | | |
| (E) | Isododecane | Marukasol R *8 (Maruzen Petrochemical Co., Ltd.) | balance | balance | balance | balance |
| | Ethanol | - | | | | |
| (F1) | Stearoxypropyltrimonium chloride | QUARTAMIN E-80K *9 (Kao Corporation) | | | | |
| | Water | - | | | | |
| Total | | | 100.0 | 100.0 | 100.0 | 100.0 |
| Component (A) content (% by mass) | | | 2.5 | 10.0 | 1.0 | 2.5 |
| Component (B) content (% by mass) | | | 2.0 | 2.0 | 2.0 | 10.0 |
| Component (C) content (% by mass) | | | 1.0 | 1.0 | 1.0 | 1.0 |
| Water content (% by mass) | | | 0.0 | 0.0 | 0.0 | 0.0 |
| Total content of component (A) and component (B) (% by mass) | | | 4.5 | 12.0 | 3.0 | 12.5 |
| Mass ratio [(A)/{(A) + (B)}] | | | 0.56 | 0.83 | 0.33 | 0.20 |
| Mass ratio [(C)/{(A) + (B)}] | | | 0.22 | 0.08 | 0.33 | 0.08 |
| Viscosity (mPa·s) | | | 3.27 | 3.85 | 3.33 | 4.63 |

(continued)

| Blending composition (% by mass) | | Product name | Example | | | |
|---|---|---|---|---|---|---|
| | | | 1 | 2 | 3 | 4 |
| Evaluation result | Touch feeling in dry state | | 15 | 10 | 13 | 12 |
| | Touch feeling in wet state | | 12 | 9 | 10 | 10 |
| | Imparting volume during styling | | 15 | 14 | 10 | 15 |
| | Heat setting property | | 13 | 15 | 12 | 15 |
| | Resistance to hair washing (feeling of volume during styling after washing hair 7 times) | | 12 | 11 | 9 | 11 |
| | Resistance to hair washing (heat setting property after washing hair 7 times) | | 12 | 13 | 9 | 13 |

Table 1 (continued)

| Blending composition (% by mass) | | Product name | Example | | |
|---|---|---|---|---|---|
| | | | 5 | 6 | 7 |
| (A) | Trimethylsiloxysilicate (MQ resin, Hard) | X-21-5595 *1 (Shin-Etsu Chemical Co., Ltd.) | 2.5 | 2.5 | 2.5 |
| | Trimethylsiloxysilicate (MQ resin, Soft) | X-21-5616 *2 (Shin-Etsu Chemical Co., Ltd.) | | | |
| (B) | Polypropylsilsesquioxane (MT resin) | DOWSIL 680 ID Fluid *3 (Dow Toray Co., Ltd.) | | | |
| | (Acrylates/dimethicone) copolymer | KP-550 *4 (Shin-Etsu Chemical Co., Ltd.) | 0.1 | 2.0 | 2.0 |
| | Oxazoline-modified silicone | Polysilicone-9 (Kao Corporation) *5 | | | |
| (C) | High-molecular weight dimethylpolysiloxane (20,000,000 $mm^2/s$) | SILSOFT B3020 *6 (Momentive Performance Materials, Inc.) | 1.0 | 10.0 | 0.1 |
| (C') | High-molecular weight dimethylpolysiloxane (100,000 $mm^2/s$) | KF-96H-100000cs *7 (Shin-Etsu Chemical Co., Ltd.) | | | |
| (E) | Isododecane | Marukasol R *8 (Maruzen Petrochemical Co., Ltd.) | balance | balance | balance |
| | Ethanol | - | | | |
| (F1) | Stearoxypropyltrimonium chloride | QUARTAMIN E-80K *9 (Kao Corporation) | | | |
| | Water | - | | | |
| Total | | | 100.0 | 100.0 | 100.0 |
| Component (A) content (% by mass) | | | 2.5 | 2.5 | 2.5 |
| Component (B) content (% by mass) | | | 0.1 | 2.0 | 2.0 |
| Component (C) content (% by mass) | | | 1.0 | 10.0 | 0.1 |
| Water content (% by mass) | | | 0.0 | 0.0 | 0.0 |
| Total content of component (A) and component (B) (% by mass) | | | 2.6 | 4.5 | 4.5 |
| Mass ratio [(A)/{(A) + (B)}] | | | 0.96 | 0.56 | 0.56 |
| Mass ratio [(C)/{(A) + (B)}] | | | 0.38 | 2.22 | 0.02 |

(continued)

| Blending composition (% by mass) | | Product name | Example | | |
|---|---|---|---|---|---|
| | | | 5 | 6 | 7 |
| Viscosity (mPa·s) | | | 3.29 | 242.8 | 1.72 |
| Evaluation result | Touch feeling in dry state | | 14 | 9 | 12 |
| | Touch feeling in wet state | | 11 | 9 | 11 |
| | Imparting volume during styling | | 10 | 15 | 12 |
| | Heat setting property | | 11 | 15 | 12 |
| | Resistance to hair washing (feeling of volume during styling after washing hair 7 times) | | 9 | 12 | 10 |
| | Resistance to hair washing (heat setting property after washing hair 7 times) | | 9 | 12 | 10 |

Table 2

| Blending composition (% by mass) | | Product name | Example | | |
|---|---|---|---|---|---|
| | | | 8 | 9 | 10 |
| (A) | Trimethylsiloxysilicate (MQ resin, Hard) | X-21-5595 *1 (Shin-Etsu Chemical Co., Ltd.) | 2.5 | 1.2 | |
| | Trimethylsiloxysilicate (MQ resin, Soft) | X-21-5616 *2 (Shin-Etsu Chemical Co., Ltd.) | | | 2.5 |
| (B) | Polypropylsilsesquioxane (MT resin) | DOWSIL 680 ID Fluid *3 (Dow Toray Co., Ltd.) | | | |
| | (Acrylates/dimethicone) copolymer | KP-550 *4 (Shin-Etsu Chemical Co., Ltd.) | 2.0 | 0.9 | 2.0 |
| | Oxazoline-modified silicone | Polysilicone-9 (Kao Corporation) *5 | | | |
| (C) | High-molecular weight dimethylpolysiloxane (20,000,000 $mm^2/s$) | SILSOFT B3020 *6 (Momentive Performance Materials, Inc.) | 1.0 | 1.0 | 1.0 |
| (C') | High-molecular weight dimethylpolysiloxane (100,000 $mm^2/s$) | KF-96H-100000cs *7 (Shin-Etsu Chemical Co., Ltd.) | | | |
| (E) | Isododecane | Marukasol R *8 (Maruzen Petrochemical Co., Ltd.) | balance | balance | balance |
| | Ethanol | - | | | |
| (F1) | Stearoxypropyltrimonium chloride | QUARTAMIN E-80K *9 (Kao Corporation) | | | |
| | Water | - | 10.0 | | |
| Total | | | 100.0 | 100.0 | 100.0 |
| Component (A) content (% by mass) | | | 2.5 | 1.2 | 2.5 |
| Component (B) content (% by mass) | | | 2.0 | 0.9 | 2.0 |
| Component (C) content (% by mass) | | | 1.0 | 1.0 | 1.0 |
| Water content (% by mass) | | | 10.0 | 0.0 | 0.0 |
| Total content of component (A) and component (B) (% by mass) | | | 4.5 | 2.1 | 4.5 |
| Mass ratio [(A)/{(A) + (B)}] | | | 0.56 | 0.57 | 0.56 |
| Mass ratio [(C)/{(A) + (B)}] | | | 0.22 | 0.48 | 0.22 |

(continued)

| Blending composition (% by mass) | | Product name | Example | | |
|---|---|---|---|---|---|
| | | | 8 | 9 | 10 |
| Viscosity (mPa·s) | | | 3.64 | 3.23 | 3.46 |
| Evaluation result | Touch feeling in dry state | | 11 | 12 | 9 |
| | Touch feeling in wet state | | 12 | 12 | 11 |
| | Imparting volume during styling | | 13 | 11 | 12 |
| | Heat setting property | | 12 | 10 | 12 |
| | Resistance to hair washing (feeling of volume during styling after washing hair 7 times) | | 10 | 9 | 9 |
| | Resistance to hair washing (heat setting property after washing hair 7 times) | | 10 | 9 | 9 |

Table 2 (continued)

| Blending composition (% by mass) | | Product name | Example | |
|---|---|---|---|---|
| | | | 11 | 12 |
| (A) | Trimethylsiloxysilicate (MQ resin, Hard) | X-21-5595 *1 (Shin-Etsu Chemical Co., Ltd.) | 2.5 | 2.5 |
| | Trimethylsiloxysilicate (MQ resin, Soft) | X-21-5616 *2 (Shin-Etsu Chemical Co., Ltd.) | | |
| (B) | Polypropylsilsesquioxane (MT resin) | DOWSIL 680 ID Fluid *3 (Dow Toray Co., Ltd.) | | |
| | (Acrylates/dimethicone) copolymer | KP-550 *4 (Shin-Etsu Chemical Co., Ltd.) | | 2.0 |
| | Oxazoline-modified silicone | Polysilicone-9 (Kao Corporation) *5 | 2.0 | |
| (C) | High-molecular weight dimethylpolysiloxane (20,000,000 $mm^2/s$) | SILSOFT B3020 *6 (Momentive Performance Materials, Inc.) | 1.0 | 1.0 |
| (C') | High-molecular weight dimethylpolysiloxane (100,000 $mm^2/s$) | KF-96H-100000cs *7 (Shin-Etsu Chemical Co., Ltd.) | | |
| (E) | Isododecane | Marukasol R *8 (Maruzen Petrochemical Co., Ltd.) | balance | balance |
| | Ethanol | - | | 20.0 |
| (F1) | Stearoxypropyltrimonium chloride | QUARTAMIN E-80K *9 (Kao Corporation) | | 0.48 |
| | Water | - | | |
| Total | | | 100.0 | 100.0 |
| Component (A) content (% by mass) | | | 2.5 | 2.5 |
| Component (B) content (% by mass) | | | 2.0 | 2.0 |
| Component (C) content (% by mass) | | | 1.0 | 1.0 |
| Water content (% by mass) | | | 0.0 | 0.0 |
| Total content of component (A) and component (B) (% by mass) | | | 4.5 | 4.5 |
| Mass ratio [(A)/{(A) + (B)}] | | | 0.56 | 0.56 |
| Mass ratio [(C)/{(A) + (B)}] | | | 0.22 | 0.22 |
| Viscosity (mPa·s) | | | 3.41 | 3.07 |

(continued)

| Blending composition (% by mass) | | Product name | Example | |
|---|---|---|---|---|
| | | | 11 | 12 |
| Evaluation result | Touch feeling in dry state | | 10 | 12 |
| | Touch feeling in wet state | | 11 | 15 |
| | Imparting volume during styling | | 10 | 14 |
| | Heat setting property | | 13 | 13 |
| | Resistance to hair washing (feeling of volume during styling after washing hair 7 times) | | 9 | 12 |
| | Resistance to hair washing (heat setting property after washing hair 7 times) | | 9 | 10 |

Table 3

| Blending composition (% by mass) | | Product name | Comparative Example | | |
|---|---|---|---|---|---|
| | | | 1 | 2 | 3 |
| (A) | Trimethylsiloxysilicate (MQ resin, Hard) | X-21-5595 *1 (Shin-Etsu Chemical Co., Ltd.) | | 2.5 | 2.5 |
| | Trimethylsiloxysilicate (MQ resin, Soft) | X-21-5616 *2 (Shin-Etsu Chemical Co., Ltd.) | | | |
| (B) | Polypropylsilsesquioxane (MT resin) | DOWSIL 680 ID Fluid *3 (Dow Toray Co., Ltd.) | | | |
| | (Acrylates/dimethicone) copolymer | KP-550 *4 (Shin-Etsu Chemical Co., Ltd.) | 2.0 | | 2.0 |
| | Oxazoline-modified silicone | Polysilicone-9 (Kao Corporation) *5 | | | |
| (C) | High-molecular weight dimethylpolysiloxane (20,000,000 $mm^2/s$) | SILSOFT B3020 *6 (Momentive Performance Materials, Inc.) | 1.0 | 1.0 | |
| (C') | High-molecular weight dimethylpolysiloxane (100,000 $mm^2/s$) | KF-96H-100000cs *7 (Shin-Etsu Chemical Co., Ltd.) | | | |
| (E) | Isododecane | Marukasol R *8 (Maruzen Petrochemical Co., Ltd.) | balance | balance | balance |
| | Ethanol | - | | | |
| (F1) | Stearoxypropyltrimonium chloride | QUARTAMIN E-80K *9 (Kao Corporation) | | | |
| | Water | - | | | |
| Total | | | 100.0 | 100.0 | 100.0 |
| Component (A) content (% by mass) | | | 0.0 | 2.5 | 2.5 |
| Component (B) content (% by mass) | | | 2.0 | 0.0 | 2.0 |
| Component (C) content (% by mass) | | | 1.0 | 1.0 | 0.0 |
| Water content (% by mass) | | | 0.0 | 0.0 | 0.0 |
| Total content of component (A) and component (B) (% by mass) | | | 2.0 | 2.5 | 4.5 |
| Mass ratio [(A)/{(A) + (B)}] | | | 0.00 | 1.00 | 0.56 |
| Mass ratio [(C)/{(A) + (B)}] | | | 0.50 | 0.40 | 0.00 |
| Viscosity (mPa·s) | | | 3.21 | 3.30 | 1.70 |

(continued)

| Blending composition (% by mass) | | Product name | Comparative Example | | |
|---|---|---|---|---|---|
| | | | 1 | 2 | 3 |
| Evaluation result | Touch feeling in dry state | | 12 | 8 | 6 |
| | Touch feeling in wet state | | 12 | 12 | 7 |
| | Imparting volume during styling | | 7 | 6 | 9 |
| | Heat setting property | | 9 | 9 | 12 |
| | Resistance to hair washing (feeling of volume during styling after washing hair 7 times) | | 5 | 4 | 6 |
| | Resistance to hair washing (heat setting property after washing hair 7 times) | | 6 | 5 | 7 |

Table 3(continued)

| Blending composition (% by mass) | | Product name | Comparative Example | | |
|---|---|---|---|---|---|
| | | | 4 | 5 | 6 |
| (A) | Trimethylsiloxysilicate (MQ resin, Hard) | X-21-5595 *1 (Shin-Etsu Chemical Co., Ltd.) | 2.5 | | 2.5 |
| | Trimethylsiloxysilicate (MQ resin, Soft) | X-21-5616 *2 (Shin-Etsu Chemical Co., Ltd.) | | | |
| (B) | Polypropylsilsesquioxane (MT resin) | DOWSIL 680 ID Fluid *3 (Dow Toray Co., Ltd.) | | 2.5 | |
| | (Acrylates/dimethicone) copolymer | KP-550 *4 (Shin-Etsu Chemical Co., Ltd.) | 2.0 | 2.0 | 2.0 |
| | Oxazoline-modified silicone | Polysilicone-9 (Kao Corporation) *5 | | | |
| (C) | High-molecular weight dimethylpolysiloxane (20,000,000 $mm^2/s$) | SILSOFT B3020 *6 (Momentive Performance Materials, Inc.) | 1.0 | 1.0 | |
| (C') | High-molecular weight dimethylpolysiloxane (100,000 $mm^2/s$) | KF-96H-100000cs *7 (Shin-Etsu Chemical Co., Ltd.) | | | 1.0 |
| (E) | Isododecane | Marukasol R *8 (Maruzen Petrochemical Co., Ltd.) | balance | balance | balance |
| | Ethanol | - | | | |
| (F1) | Stearoxypropyltrimonium chloride | QUARTAMIN E-80K *9 (Kao Corporation) | | | |
| | Water | - | 15.0 | | |
| Total | | | 100.0 | 100.0 | 100.0 |
| Component (A) content (% by mass) | | | 2.5 | 0.0 | 2.5 |
| Component (B) content (% by mass) | | | 2.0 | 4.5 | 2.0 |
| Component (C) content (% by mass) | | | 1.0 | 1.0 | 0.0 |
| Water content (% by mass) | | | 15.0 | 0.0 | 0.0 |
| Total content of component (A) and component (B) (% by mass) | | | 4.5 | 4.5 | 4.5 |
| Mass ratio [(A)/{(A) + (B)}] | | | 0.56 | 0.00 | 0.56 |
| Mass ratio [(C)/{(A) + (B)}] | | | 0.22 | 0.22 | 0.00 |
| Viscosity (mPa·s) | | | 4.33 | 3.70 | 2.41 |

(continued)

| Blending composition (% by mass) | | Product name | Comparative Example | | |
|---|---|---|---|---|---|
| | | | 4 | 5 | 6 |
| Evaluation result | Touch feeling in dry state | | 8 | 7 | 5 |
| | Touch feeling in wet state | | 10 | 12 | 11 |
| | Imparting volume during styling | | 10 | 9 | 9 |
| | Heat setting property | | 13 | 12 | 12 |
| | Resistance to hair washing (feeling of volume during styling after washing hair 7 times) | | 7 | 7 | 7 |
| | Resistance to hair washing (heat setting property after washing hair 7 times) | | 3 | 10 | 8 |

The components shown in Tables 1 to 3 are as follows.

*1: X-21-5595, manufactured by Shin-Etsu Chemical Co., Ltd., trimethylsiloxysilicate (active ingredient amount: 60% by mass, solvent: isododecane) (viscosity at 25°C of a 50% by mass solution in which the active ingredient is dissolved in dimethicone KF-96L-2cs: 120 $mm^2/s$)
*2: X-21-5616, manufactured by Shin-Etsu Chemical Co., Ltd., trimethylsiloxysilicate (active ingredient amount: 60% by mass, solvent: isododecane) (viscosity at 25°C of a 50% by mass solution in which the active ingredient is dissolved in dimethicone KF-96L-2cs: 10 $mm^2/s$)
*3:DOWSIL 680 **ID** Fluid, manufactured by Dow Toray Co., Ltd., polypropylsilsesquioxane (active ingredient amount: 75% by mass, solvent: isododecane)
*4: KP-550, manufactured by Shin-Etsu Chemical Co., Ltd., (acrylates/dimethicone) copolymer (active ingredient amount: 40% by mass, solvent: isododecane)
*5: Polysilicone-9, manufactured by Kao Corporation
*6: Silsoft B3020, manufactured by Momentive Performance Materials, Inc., high-molecular weight dimethylpolysiloxane (active ingredient amount: 20% by mass, solvent: isododecane), viscosity at 25°C: 20,000,000 $mm^2/s$ (degree of polymerization: 3,546)
*7: KF-96H-100000cs, manufactured by Shin-Etsu Chemical Co., Ltd., high-molecular weight dimethylpolysiloxane (active ingredient amount: 100% by mass), viscosity at 25°C: 100,000 $mm^2/s$ (degree of polymerization: 1,429)
*8: Marukasol R, manufactured by Maruzen Petrochemical Co., Ltd., isododecane (active ingredient amount: 100% by mass)
*9: QUARTAMIN E-80K, manufactured by Kao Corporation, stearoxypropyltrimonium chloride (active ingredient amount: 45% by mass)

Regarding the degree of polymerization (P) of the above-mentioned components *6 and *7, the molecular weight (M) is obtained from the above-mentioned formula (4) from the viscosity ($\eta$), and the degree of polymerization (P) can be obtained from the following formula (5) since the molecular weight of the basic unit of dimethylpolysiloxane is 74.

$$P = M/74 \quad (5)$$

From Tables 1 to 3, it is found that when the hair processing agent (I) of the present invention is used, excellent effects are exhibited in all of the touch feeling in a dry state and a wet state, the property of imparting a feeling of volume during styling, the heat setting property, and the property of imparting a feeling of volume and the heat setting property after 7 times of hair washing. On the other hand, in the hair processing agents of Comparative Example 1 in which the component (A) was not contained, and Comparative Example 2 in which the component (B) was not contained, the property of imparting a feeling of volume during styling, and the property of imparting a feeling of volume and the heat setting property after 7 times of hair washing were deteriorated. In the hair processing agent of Comparative Example 3 in which the component (C) was not contained, the touch feeling, the property of imparting a feeling of volume and the heat setting property after 7 times of hair washing were deteriorated, and in the hair processing agent of Comparative Example 4 in which the water content was more than 10% by mass, the property of imparting a feeling of volume and the heat setting property after 7 times of hair washing were remarkably deteriorated.
In Comparative Example 5, in which a film-forming polymer (T resin) not corresponding to the component (A) was used instead of the component (A), and in Comparative Example 6, in which an organopolysiloxane (C') not corresponding

to the component (C) was used instead of the component (C), the touch feeling in a dry state and the property of imparting a feeling of volume after 7 times of hair washing were both poor.

Examples 13 to 19 and Comparative Examples 7 to 9 (Preparation and Evaluation of Hair Processing Agent (II) (Hair Styling Agent))

The components shown in Table 4 were blended at the ratios shown in the tables and mixed until uniform to prepare a hair processing agent (II). Various evaluations were performed by the above-described methods using the obtained hair processing agent (II). The results are shown in Table 4.

The blending amounts (% by mass) described in the tables are all active ingredient amounts.

Table 4

| Blending composition (% by mass) | | Product name | Example | | | |
|---|---|---|---|---|---|---|
| | | | 13 | 14 | 15 | 16 |
| (A) | Trimethylsiloxysilicate (MQ resin, Hard) | X-21-5595 *1 (Shin-Etsu Chemical Co., Ltd.) | 2.5 | 2.5 | 2.5 | 2.5 |
| (B1) | (Acrylates/dimethicone) copolymer | KP-550 *4 (Shin-Etsu Chemical Co., Ltd.) | 2.0 | 2.0 | 2.0 | 2.0 |
| (C) | High-molecular weight dimethylpolysiloxane (20,000,000 $mm^2/s$) | SILSOFT B3020 *6 (Momentive Performance Materials, Inc.) | 1.0 | 1.0 | 1.0 | 1.0 |
| (D) | (Bisisobutyl PEG-15/amodimethicone) copolymer | DOWSIL SS-3588 *10 (Dow Toray Co., Ltd.) | 0.5 | 0.5 | 5.0 | 0.1 |
| | (Bisisobutyl PEG-14/amodimethicone) copolymer | DOWSIL Silstyle 201 *11 (Dow Toray Co., Ltd.) | | | | |
| (D') | PEG-11methyl ether dimethicone | KF-6011 *12 (Shin-Etsu Chemical Co., Ltd.) | | | | |
| (E) | Isododecane | Marukasol R *8 (Maruzen Petrochemical Co., Ltd.) | balance | balance | balance | balance |
| | Ethanol | Traceable 99 First Grade *13 (Japan Alcohol Corporation) | 20.1 | 0.1 | 20.1 | 20.1 |
| (F1) | Behentrimonium chloride | QUARTAMIN 2285E-E *14 (Kao Corporation) | | | | |
| | Steartrimonium chloride | QUARTAMIN 86P CONC *15 (Kao Corporation) | | | | |
| (F2) | Polyquaternium-6 | Merquat 100 *16 (Lubrizol Japan Limited) | | | | |
| | Amodimethicone | DOWSIL CF 1046 *17 (Dow Toray Co., Ltd.) | | | | |
| Total | | | 100.0 | 100.0 | 100.0 | 100.0 |
| Component (A) content (% by mass) | | | 2.5 | 2.5 | 2.5 | 2.5 |
| Component (B1) content (% by mass) | | | 2.0 | 2.0 | 2.0 | 2.0 |
| Component (C) content (% by mass) | | | 1.0 | 1.0 | 1.0 | 1.0 |
| Component (D) content (% by mass) | | | 0.5 | 0.5 | 5.0 | 0.1 |
| Total content of component (A) and component (B1) (% by mass) | | | 4.5 | 4.5 | 4.5 | 4.5 |
| Total content of components (A), (B1), (C), and (D) (% by mass) | | | 6.0 | 6.0 | 10.5 | 5.6 |

(continued)

| Blending composition (% by mass) | | Product name | Example | | | |
|---|---|---|---|---|---|---|
| | | | 13 | 14 | 15 | 16 |
| Mass ratio [(A)/{(A) + (B1)}] | | | 0.56 | 0.56 | 0.56 | 0.56 |
| Mass ratio [(C)/{(A) + (B1)}] | | | 0.22 | 0.22 | 0.22 | 0.22 |
| Mass ratio [(D)/{(A) + (B1) + (C)}] | | | 0.09 | 0.09 | 0.91 | 0.02 |
| Component (F) content (% by mass) | | | 0.00 | 0.00 | 0.00 | 0.00 |
| Mass ratio [(F)/(C)] | | | 0.00 | 0.00 | 0.00 | 0.00 |
| Viscosity (mPa·s) | | | 3.47 | 3.40 | 3.93 | 3.16 |
| Evaluation result | Touch feeling in dry state | | 14 | 13 | 9 | 13 |
| | Touch feeling in wet state | | 12 | 11 | 9 | 11 |
| | Heat setting property | | 15 | 15 | 15 | 13 |
| | Resistance to hair washing (heat setting property after washing hair 7 times) | | 14 | 13 | 13 | 10 |
| | Slipperiness of hair iron (g) | | 172 | 201 | 298 | 230 |

Table 4 (continued)

| Blending composition (% by mass) | | Product name | Example | | |
|---|---|---|---|---|---|
| | | | 17 | 18 | 19 |
| (A) | Trimethylsiloxysilicate (MQ resin, Hard) | X-21-5595 *1 (Shin-Etsu Chemical Co., Ltd.) | 2.5 | 2.5 | 2.5 |
| (B1) | (Acrylates/dimethicone) copolymer | KP-550 *4 (Shin-Etsu Chemical Co., Ltd.) | 2.0 | 2.0 | 2.0 |
| (C) | High-molecular weight dimethylpolysiloxane (20,000,000 $mm^2/s$) | SILSOFT B3020 *6 (Momentive Performance Materials, Inc.) | 1.0 | 1.0 | 1.0 |
| (D) | (Bisisobutyl PEG-15/amodimethicone) copolymer | DOWSIL SS-3588 *10 (Dow Toray Co., Ltd.) | 0.5 | 0.5 | |
| | (Bisisobutyl PEG-14/amodimethicone) copolymer | DOWSIL Silstyle 201 *11 (Dow Toray Co., Ltd.) | | | 0.5 |
| (D') | PEG-11 methyl ether dimethicone | KF-6011 *12 (Shin-Etsu Chemical Co., Ltd.) | | | |
| (E) | Isododecane | Marukasol R *8 (Maruzen Petrochemical Co., Ltd.) | balance | balance | balance |
| | Ethanol | Traceable 99 First Grade *13 (Japan Alcohol Corporation) | 20.2 | 20.1 | 20.1 |
| (F1) | Behentrimonium chloride | QUARTAMIN 2285E-E *14 (Kao Corporation) | 0.12 | | |
| | Steartrimonium chloride | QUARTAMIN 86P CONC *15 (Kao Corporation) | 0.12 | | |
| (F2) | Polyquaternium-6 | Merquat 100 *16 (Lubrizol Japan Limited) | | 0.24 | |
| | Amodimethicone | DOWSIL CF 1046 *17 (Dow Toray Co., Ltd.) | | | |
| Total | | | 100.0 | 100.0 | 100.0 |

(continued)

| Blending composition (% by mass) | | Product name | Example | | |
|---|---|---|---|---|---|
| | | | 17 | 18 | 19 |
| Component (A) content (% by mass) | | | 2.5 | 2.5 | 2.5 |
| Component (B1) content (% by mass) | | | 2.0 | 2.0 | 2.0 |
| Component (C) content (% by mass) | | | 1.0 | 1.0 | 1.0 |
| Component (D) content (% by mass) | | | 0.5 | 0.5 | 0.5 |
| Total content of component (A) and component (B1) (% by mass) | | | 4.5 | 4.5 | 4.5 |
| Total content of components (A), (B1), (C), and (D) (% by mass) | | | 6.0 | 6.0 | 6.0 |
| Mass ratio [(A)/{(A) + (B1)}] | | | 0.56 | 0.56 | 0.56 |
| Mass ratio [(C)/{(A) + (B1)}] | | | 0.22 | 0.22 | 0.22 |
| Mass ratio [(D)/{(A) + (B1) + (C)}] | | | 0.09 | 0.09 | 0.09 |
| Component (F) content (% by mass) | | | 0.24 | 0.24 | 0.00 |
| Mass ratio [(F)/(C)] | | | 0.24 | 0.24 | 0.00 |
| Viscosity (mPa·s) | | | 3.54 | 3.66 | 3.10 |
| Evaluat ion result | Touch feeling in dry state | | 15 | 10 | 14 |
| | Touch feeling in wet state | | 15 | 13 | 11 |
| | Heat setting property | | 15 | 15 | 14 |
| | Resistance to hair washing (heat setting property after washing hair 7 times) | | 14 | 11 | 13 |
| | Slipperiness of hair iron (g) | | 169 | 220 | 174 |

Table 4 (continued)

| Blending composition (% by mass) | | Product name | Comparative Example | | |
|---|---|---|---|---|---|
| | | | 7 | 8 | 9 |
| (A) | Trimethylsiloxysilicate (MQ resin, Hard) | X-21-5595 *1 (Shin-Etsu Chemical Co., Ltd.) | 2.5 | 2.5 | 2.5 |
| (B1) | (Acrylates/dimethicone) copolymer | KP-550 *4 (Shin-Etsu Chemical Co., Ltd.) | 2.0 | 2.0 | 2.0 |
| (C) | High-molecular weight dimethylpolysiloxane (20,000,000 $mm^2/s$) | SILSOFT B3020 *6 (Momentive Performance Materials, Inc.) | 1.0 | 1.0 | 1.0 |
| (D) | (Bisisobutyl PEG-15/amodimethicone) copolymer | DOWSIL SS-3588 *10 (Dow Toray Co., Ltd.) | | | |
| | (Bisisobutyl PEG-14/amodimethicone) copolymer | DOWSIL Silstyle 201 *11 (Dow Toray Co., Ltd.) | | | |
| (D') | PEG-11 methyl ether dimethicone | KF-6011 *12 (Shin-Etsu Chemical Co., Ltd.) | | | 0.5 |
| (E) | Isododecane | Marukasol R *8 (Maruzen Petrochemical Co., Ltd.) | balance | balance | balance |
| | Ethanol | Traceable 99 First Grade *13 (Japan Alcohol Corporation) | 20.0 | 20.0 | 20.0 |

(continued)

| Blending composition (% by mass) | | Product name | Comparative Example | | |
|---|---|---|---|---|---|
| | | | 7 | 8 | 9 |
| (F1) | Behentrimonium chloride | QUARTAMIN 2285E-E *14 (Kao Corporation) | | | |
| | Steartrimonium chloride | QUARTAMIN 86P CONC *15 (Kao Corporation) | | | |
| (F2) | Polyquaternium-6 | Merquat 100 *16 (Lubrizol Japan Limited) | | | |
| | Amodimethicone | DOWSIL CF 1046 *17 (Dow Toray Co., Ltd.) | | 0.5 | |
| Total | | | 100.0 | 100.0 | 100.0 |
| Component (A) content (% by mass) | | | 2.5 | 2.5 | 2.5 |
| Component (B1) content (% by mass) | | | 2.0 | 2.0 | 2.0 |
| Component (C) content (% by mass) | | | 1.0 | 1.0 | 1.0 |
| Component (D) content (% by mass) | | | 0.0 | 0.0 | 0.0 |
| Total content of component (A) and component (B1) (% by mass) | | | 4.5 | 4.5 | 4.5 |
| Total content of components (A), (B1), (C), and (D) (% by mass) | | | 5.5 | 5.5 | 5.5 |
| Mass ratio [(A)/{(A) + (B1)}] | | | 0.56 | 0.56 | 0.56 |
| Mass ratio [(C)/{(A) + (B1)}] | | | 0.22 | 0.22 | 0.22 |
| Mass ratio [(D)/{(A) + (B1) + (C)}] | | | 0.00 | 0.00 | 0.00 |
| Component (F) content (% by mass) | | | 0.00 | 0.50 | 0.00 |
| Mass ratio [(F)/(C)] | | | 0.00 | 0.50 | 0.00 |
| Viscosity (mPa·s) | | | 3.01 | 4.08 | 3.32 |
| Evaluation result | Touch feeling in dry state | | 14 | 8 | 8 |
| | Touch feeling in wet state | | 10 | 5 | 11 |
| | Heat setting property | | 13 | 11 | 12 |
| | Resistance to hair washing (heat setting property after washing hair 7 times) | | 10 | 3 | 4 |
| | Slipperiness of hair iron (g) | | 343 | 253 | 165 |

The components shown in Table 4 are as follows.

*1: X-21-5595, manufactured by Shin-Etsu Chemical Co., Ltd., trimethylsiloxysilicate (active ingredient amount: 60% by mass, solvent: isododecane) (viscosity at 25°C of a 50% by mass solution in which the active ingredient is dissolved in dimethicone KF-96L-2cs: 120 $mm^2/s$)
*4: KP-550, manufactured by Shin-Etsu Chemical Co., Ltd., (acrylates/dimethicone) copolymer (active ingredient amount: 40% by mass, solvent: isododecane)
*6: Silsoft B3020, manufactured by Momentive Performance Materials, Inc., high-molecular weight dimethylpolysiloxane (active ingredient amount: 20% by mass, solvent: isododecane), viscosity at 25°C: 20,000,000 $mm^2/s$ (degree of polymerization: 3,546)
*10: DOWSIL SS-3588 Fluid, manufactured by Dow Toray Co., Ltd., (bisisobutyl PEG-15/amodimethicone) copolymer (active ingredient amount: 80% by mass, solvent: ethanol)
*11: DOWSIL SILSTYLE 201, manufactured by Dow Toray Co., Ltd., (bisisobutyl PEG-14/amodimethicone) copolymer (active ingredient amount: 99.95% by mass)
*12: KF-6011, manufactured by Shin-Etsu Chemical Co., Ltd., PEG-11 methyl ether dimethicone (active ingredient amount: 100% by mass)
*8: Marukasol R, manufactured by Maruzen Petrochemical Co., Ltd., isododecane (active ingredient amount: 100%

by mass)
*13: Traceable 99 First Grade, manufactured by Japan Alcohol Corporation, Ethanol
*14: QUARTAMIN 2285E-E, manufactured by Kao Corporation, behentrimonium chloride (active ingredient amount: 58% by mass, solvent: ethanol, water)
*15: QUARTAMIN 86P CONC, manufactured by Kao Corporation, steartrimonium chloride (active ingredient amount: 63% by mass, solvent: isopropanol, water)
*16: Merquat 100, manufactured by Lubrizol Japan Limited, polyquaternium-6 (active ingredient amount: 41.5% by mass, solvent: water)
*17: DOWSIL CF 1046, manufactured by Dow Toray Co., Ltd., amodimethicone (active ingredient amount: 17.6% by mass, solvent: dimethicone)

From Table 4, it is found that when the hair processing agent (II) of the present invention is used, excellent effects are exhibited in all of the touch feeling in a dry state and a wet state, the heat setting property and the slipperiness of a hair iron during styling, and the heat setting property after 7 times of hair washing. On the other hand, in Comparative Example 7 in which the component (D) was not contained, the touch feeling in a wet state, the heat setting property after 7 times of hair washing, and the slipperiness of a hair iron were deteriorated. In the hair processing agents of Comparative Example 8 in which amodimethicone was used instead of the component (D) and Comparative Example 9 in which polyether-modified silicone having no cationic group was used instead of the component (D), the touch feeling in a dry state and the slipperiness of a hair iron were deteriorated, and the heat setting property after 7 times of hair washing was remarkably deteriorated.
Formulation examples of the hair processing agent (I) (hair oil) of the present invention are shown below.

Formulation Example 1-1 (hair oil)

| | (% by mass) |
|---|---|
| (A) Trimethylsiloxysilicate (MQ resin, Hard) (*1) | 2.5 |
| (B) (Acrylates/polytrimethylsiloxymethacrylate) copolymer (*2) | 2.0 |
| (C) High-molecular weight dimethylpolysiloxane (20,000,000 $mm^2/s$) (*3) | 1.0 |
| (D) Isododecane (*4) | balance |
| Total | 100.0 |

(*1) X-21-5595, manufactured by Shin-Etsu Chemical Co., Ltd.
(*2) DOWSIL FA 4004 ID Silicone Acrylate, manufactured by Dow Toray Co., Ltd.
(*3) SILSOFT B3020, manufactured by Momentive Performance Materials, Inc.
(*4) Marukasol R, manufactured by Maruzen Petrochemical Co., Ltd.

Formulation Example 1-2 (hair oil)

| | (% by mass) |
|---|---|
| (A) Trimethylsiloxysilicate (MQ resin, Hard) (*1) | 2.5 |
| (B) (Acrylates/polytrimethylsiloxymethacrylate) copolymer (*2) | 2.0 |
| (C) High-molecular weight dimethylpolysiloxane (20,000,000 $mm^2/s$) (*3) | 1.0 |
| (D) Isododecane (*4) | balance |
| (D) Ethanol | 20.0 |
| (E) Stearoxypropyltrimonium chloride (*5) | 0.48 |

(continued)

| | (% by mass) |
|---|---|
| Total | 100.0 |

(*1) X-21-5595, manufactured by Shin-Etsu Chemical Co., Ltd.
(*2) DOWSIL FA 4004 ID Silicone Acrylate, manufactured by Dow Toray Co., Ltd.
(*3) SILSOFT B3020, manufactured by Momentive Performance Materials, Inc.
(*4) Marukasol R, manufactured by Maruzen Petrochemical Co., Ltd.
(*5) QUARTAMIN E-80K, manufactured by Kao Corporation

Formulation examples of the hair processing agent (II) (hair oil) of the present invention are shown below.

Formulation Example 2-1 (hair oil)

| | (% by mass) |
|---|---|
| (A) Trimethylsiloxysilicate (MQ resin, Hard) (*1) | 2.5 |
| (B) (Acrylates/polytrimethylsiloxymethacrylate) copolymer (*2) | 2.0 |
| (C) High-molecular weight dimethylpolysiloxane (20,000,000 $mm^2/s$) (*3) | 1.0 |
| (D) (Bisisobutyl PEG-15/amodimethicone) copolymer (*4) | 0.5 |
| (E) Isododecane (*5) | balance |
| (E) Ethanol (*6) | 20.1 |
| Total | 100.0 |

(*1) X-21-5595, manufactured by Shin-Etsu Chemical Co., Ltd.
(*2) DOWSIL FA 4004 ID Silicone Acrylate, manufactured by Dow Toray Co., Ltd.
(*3) SILSOFT B3020, manufactured by Momentive Performance Materials, Inc.
(*4) DOWSIL SS-3588, manufactured by Dow Toray Co., Ltd.
(*5) Marukasol R, manufactured by Maruzen Petrochemical Co., Ltd.
(*6) Traceable 99 First Grade, manufactured by Japan Alcohol Corporation

Formulation Example 2-2 (hair oil)

| | (% by mass) |
|---|---|
| (A) Trimethylsiloxysilicate (MQ resin, Hard) (*1) | 2.5 |
| (B) (Acrylates/polytrimethylsiloxymethacrylate) copolymer (*2) | 2.0 |
| (C) High-molecular weight dimethylpolysiloxane (20,000,000 $mm^2/s$) (*3) | 1.0 |
| (D) (Bisisobutyl PEG-15/amodimethicone) copolymer (*4) | 0.5 |
| (E) Isododecane (*5) | balance |
| (E) Ethanol (*6) | 20.2 |
| (F) Behentrimonium chloride (*7) | 0.12 |
| (F) Steartrimonium chloride (*8) | 0.12 |

(continued)

| | (% by mass) |
|---|---|
| Total | 100.0 |

(*1) X-21-5595, manufactured by Shin-Etsu Chemical Co., Ltd.
(*2) DOWSIL FA 4004 ID Silicone Acrylate, manufactured by Dow Toray Co., Ltd.
(*3) SILSOFT B3020, manufactured by Momentive Performance Materials, Inc.
(*4) DOWSIL SS-3588, manufactured by Dow Toray Co., Ltd.
(*5) Marukasol R, manufactured by Maruzen Petrochemical Co., Ltd.
(*6) Traceable 99 First Grade, manufactured by Japan Alcohol Corporation
(*7) QUARTAMIN 2285E-E, manufactured by Kao Corporation
(*8) QUARTAMIN 86P CONC, manufactured by Kao Corporation

Industrial Applicability

According to the hair processing agent or the fiber processing agent for a headwear product of the first invention, it is possible to impart a good touch feeling to hair or a fiber for a headwear product in either a dry state or a wet state, exhibit excellent property of imparting a feeling of volume and heat setting property during styling, and suppress a decrease in the property of imparting a feeling of volume and the heat setting property even after hair washing.
According to the hair processing agent or the fiber processing agent for a headwear product of the second invention, it is possible to impart a good touch feeling to hair or a fiber for a headwear product in either a dry state or a wet state, exhibit excellent heat setting property and slipperiness of a hair iron during styling, and suppress a decrease in the heat setting property even after hair washing.

**Claims**

1. A hair processing agent or a fiber processing agent for a headwear product of the following (I) or (II):

(I) wherein the hair processing agent or the fiber processing agent for a headwear product comprises the following components (A) to (C):

(A) a film-forming polymer containing an M unit represented by $(R^1)_3SiO_{1/2}$ ($R^1$ represents a hydrocarbon group having 1 or more and 12 or less carbon atoms which may be substituted with fluorine or a hydroxy group, and a plurality of $R^1$'s may be the same or different from each other) and a Q unit represented by $SiO_{4/2}$;
(B) a film-forming polymer other than the component (A); and
(C) a high-molecular-weight organopolysiloxane having a degree of polymerization of 2,200 or more and 20,000 or less,

wherein a content of water is 10% by mass or less;
(II) wherein the hair processing agent or the fiber processing agent for a headwear product comprises the following components (A), (B 1), (C), and (D):

(A) a film-forming polymer containing an M unit represented by $(R^1)_3SiO_{1/2}$ ($R^1$ represents a hydrocarbon group having 1 or more and 12 or less carbon atoms which may be substituted with fluorine or a hydroxy group, and a plurality of $R^1$'s may be the same or different from each other) and a Q unit represented by $SiO_{4/2}$;
(B1) a film-forming polymer other than the component (A), excluding a cationic polymer containing a quaternary ammonium cation;
(C) a high-molecular-weight organopolysiloxane having a degree of polymerization of 2,200 or more and 20,000 or less; and
(D) an organopolysiloxane other than the components (A), (B 1), and (C), having a polyalkylene oxide moiety and a cationic group.

2. The hair processing agent or the fiber processing agent for a headwear product according to claim 1, wherein a total content of the component (A) and the component (B) in the processing agent (I) and a total content of the component

(A) and the component (B 1) in the processing agent (II) each are 2% by mass or more.

3. The hair processing agent or the fiber processing agent for a headwear product according to claim 1 or 2, wherein the degree of polymerization of the component (C) is 2,600 or more and 4,500 or less.

4. The hair processing agent or the fiber processing agent for a headwear product according to any one of claims 1 to 3, wherein the component (B) and the component (B1) contains an acrylic silicone polymer as a component (B-1).

5. The hair processing agent or the fiber processing agent for a headwear product according to any one of claims 1 to 4, wherein the processing agent further contains a volatile solvent as a component (E).

6. The hair processing agent or the fiber processing agent for a headwear product according to any one of claims 1 to 5, wherein the processing agent (I) further contains a cationic surfactant as a component (F1).

7. The hair processing agent or the fiber processing agent for a headwear product according to any one of claims 1 to 5, wherein the processing agent (II) further contains a cationic compound other than the components (A), (B1), (C), and (D) as a component (F).

8. The hair processing agent or the fiber processing agent for a headwear product according to claim 7, wherein the component (F) is one or more selected from the group consisting of (F1) a cationic surfactant and (F2) a cationic polymer containing a quaternary ammonium cation.

9. The hair processing agent or the fiber processing agent for a headwear product according to any one of claims 1 to 8, wherein the processing agent is used without being washed off after being applied to hair or a fiber for a headwear product.

10. The hair processing agent or the fiber processing agent for a headwear product according to any one of claims 1 to 9, wherein the processing agent is a styling agent.

11. A method for treating hair or a fiber for a headwear product, comprising a step of applying the processing agent according to any one of claims 1 to 10 to hair or a fiber for a headwear product and then drying the hair or the fiber for a headwear product.

**INTERNATIONAL SEARCH REPORT**

International application No.
**PCT/JP2023/020256**

**A. CLASSIFICATION OF SUBJECT MATTER**

***A61K 8/891***(2006.01)i; ***A61K 8/31***(2006.01)i; ***A61K 8/41***(2006.01)i; ***A61K 8/897***(2006.01)i; ***A61K 8/898***(2006.01)i; ***A61Q 5/00***(2006.01)i; ***A61Q 5/06***(2006.01)i; ***A61Q 5/12***(2006.01)i; ***D06M 13/513***(2006.01)i; ***D06M 15/21***(2006.01)i; ***D06M 15/263***(2006.01)i; ***D06M 15/643***(2006.01)i

FI: A61K8/891; A61K8/31; A61K8/41; A61K8/897; A61K8/898; A61Q5/00; A61Q5/06; A61Q5/12; D06M13/513; D06M15/21; D06M15/263; D06M15/643

According to International Patent Classification (IPC) or to both national classification and IPC

**B. FIELDS SEARCHED**

Minimum documentation searched (classification system followed by classification symbols)

A61K8/891; A61K8/31; A61K8/41; A61K8/897; A61K8/898; A61Q5/00; A61Q5/06; A61Q5/12; D06M13/513; D06M15/21; D06M15/263; D06M15/643

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

Mintel GNPD

**C. DOCUMENTS CONSIDERED TO BE RELEVANT**

| Category* | Citation of document, with indication, where appropriate, of the relevant passages | Relevant to claim No. |
|---|---|---|
| X | JP 2021-187851 A (KAO CORP) 13 December 2021 (2021-12-13) claims, paragraphs [0005], [0007], [0010], [0013], [0024], [0026], [0041], [0051], [0091], [0095], [0100], [0127]-[0145] | 1-5, 9-11 |
| Y | | 6-11 |
| X | JP 2021-187850 A (KAO CORP) 13 December 2021 (2021-12-13) claims, paragraphs [0008], [0011], [0015], [0019], [0027], [0030], [0032], [0046]-[0047], [0058], [0093], [0102], [0147]-[0177] | 1-5, 9-11 |
| Y | | 6-11 |
| Y | WO 2019/039548 A1 (SHISEIDO COMPANY, LTD.) 28 February 2019 (2019-02-28) claims, paragraphs [0010], [0037], [0061]-[0077] | 6-11 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **14 July 2023** | **08 August 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**
**Information on patent family members**

International application No.
**PCT/JP2023/020256**

| Patent document cited in search report | Publication date (day/month/year) | Patent family member(s) | Publication date (day/month/year) |
|---|---|---|---|
| JP 2021-187851 A | 13 December 2021 | EP 4159281 A1<br>claims, paragraphs [0013], [0019]-[0022], [0050], [0056], [0079], [0102], [0162], [0170], [0178], [0212]-[0234]<br>TW 202210059 A<br>CN 115666504 A<br>WO 2021/246273 A1 | |
| JP 2021-187850 A | 13 December 2021 | EP 4159190 A1<br>claims, paragraphs [0021], [0026]-[0037], [0041],[0059], [0065], [0071], [0094]-[0095], [0119], [0171], [0188], [0234]-[0275]<br>TW 202207899 A<br>CN 115666503 A<br>WO 2021/246272 A1 | |
| WO 2019/039548 A1 | 28 February 2019 | US 2020/0246234 A1<br>claims, paragraphs [0014], [0042], [0067]-[0089]<br>EP 3673894 A1<br>CN 110996892 A<br>TW 201919567 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H09501934 A
- JP 2018002643 A
- JP 2021134166 A
- JP 2019513712 A
- JP 2021526180 A
- JP H111530 A
- JP 2000063225 A
- JP H225411 A
- JP H2132141 A
- JP H3162442 A
- JP 2003104825 A
- JP H692825 A